# EUROPEAN PATENT SPECIFICATION

(11) **EP 4 409 579 B1**
(45) Date of publication and mention of the grant of the patent: **02.09.2026**
(21) Application number: 22793980.8
(22) Date of filing: 29.09.2022
(51) Int. Cl.: G16C 20/50, G16B 15/30

(54) **CHARACTERIZATION OF INTERACTIONS BETWEEN COMPOUNDS AND POLYMERS USING NEGATIVE POSE DATA AND MODEL CONDITIONING**
CHARAKTERISIERUNG VON WECHSELWIRKUNGEN ZWISCHEN VERBINDUNGEN UND POLYMEREN UNTER VERWENDUNG VON NEGATIVEN HALTUNGSDATEN UND MODELLKONDITIONIERUNG
CARACTÉRISATION D'INTERACTIONS ENTRE DES COMPOSÉS ET DES POLYMÈRES À L'AIDE DE DONNÉES DE POSE NÉGATIVE ET DE CONDITIONNEMENT DE MODÈLE

(30) Priority: 01.10.2021 US 202163251142 P
(43) Date of publication of application: 07.08.2024
(73) Proprietor: Numerion Labs, Inc., San Francisco, CA 94103 (US)
(72) Inventor: GNIEWEK, Pawel, San Francisco, CA 94103 (US); WORLEY, Brad, San Francisco, CA 94103 (US); ANDERSON, Brandon, San Francisco, CA 94103 (US); STAFFORD, Kate, San Francisco, CA 94103 (US); MYSINGER, Michael, San Francisco, CA 94103 (US)
(74) Representative: Grund, Martin
(86) International application number: PCT/US2022/045250
(87) International publication number: WO 2023/055949

(56) References cited:
- EP-B1- 3 356 999
- WO-A2-2018/183263
- MATTHEW RAGOZA ET AL: "Protein-Ligand Scoring with Convolutional Neural Networks", ARXIV.ORG, CORNELL UNIVERSITY LIBRARY, 201 OLIN LIBRARY CORNELL UNIVERSITY ITHACA, NY 14853, 8 December 2016 (2016-12-08), XP080737867, DOI: 10.1021/ACS.JCIM.6B00740
- PAWEL GNIEWEK ET AL: "Learning physics confers pose-sensitivity in structure-based virtual screening", ARXIV.ORG, CORNELL UNIVERSITY LIBRARY, 201 OLIN LIBRARY CORNELL UNIVERSITY ITHACA, NY 14853, 2 December 2021 (2021-12-02), XP091110774

## Description

### CROSS REFERENCE TO RELATED APPLICATION

This application claims priority to United States Provisional Patent Application No. 63/251,142, entitled "CHARACTERIZATION OF INTERACTIONS BETWEEN COMPOUNDS AND POLYMERS USING NEGATIVE POSE DATA AND MODEL CONDITIONING," filed October 1, 2021.

### TECHNICAL FIELD

This application is directed to using models to characterize interactions between test compounds and target polymers.

### BACKGROUND

Fundamentally, biological systems operate through the physical interaction of molecules, such as a compound with a target polymer. Structure-based, virtual high throughput screening (vHTS) machine learning methods have been used to characterize interactions between candidate (test) compounds and a target polymer through machine learning approaches. Such characterization, for instance, can report a continuous or categorical activity label, a PKa, or any other suitable metric to characterize the interaction between a candidate compound and a target polymer. EP3356999 B1 discloses computer system for predicting binding affinity of a chemical compound to a target polymer.

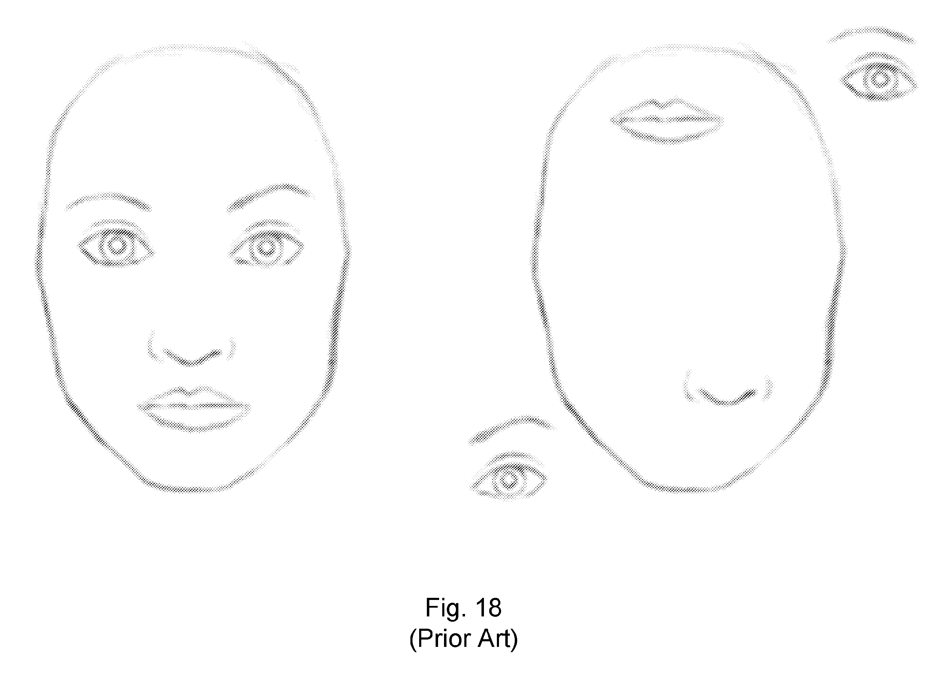

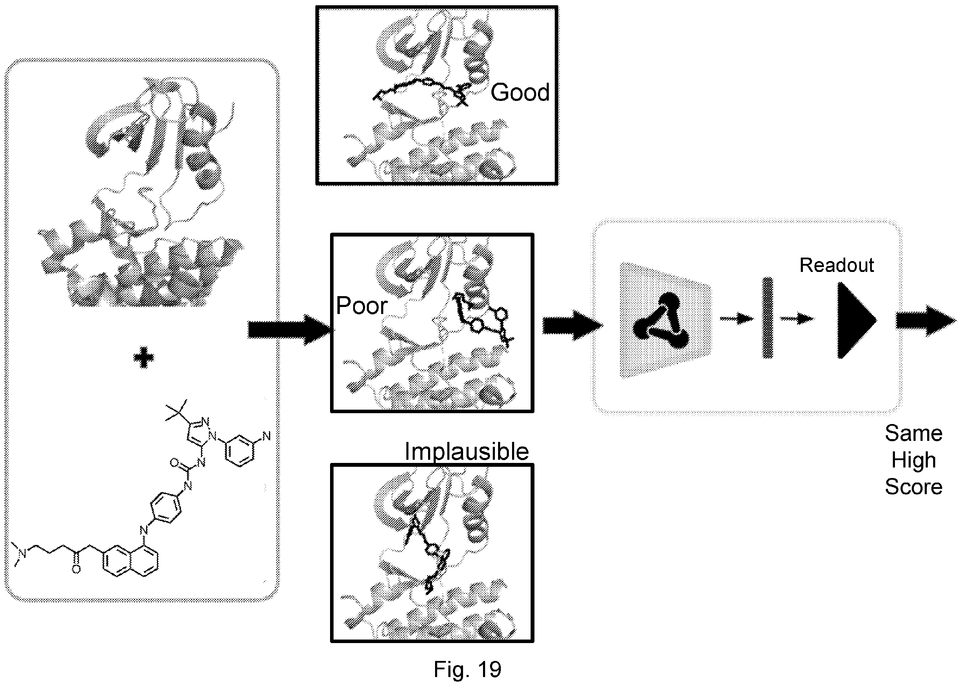

One drawback with vHTS machine learning methods is how the machine learning models invoked in such methods interpret the pose between a compound and a binding site. Models represent a compound and a polymer independently, even though the structural information about the two is provided. Because of that, any provided pose that allows identification of a polymer and a compound gives the same scores. The model is insensitive to the poses. Figure 19 illustrates. It is exemplified in the Picasso problem, where machine learning models such as convolutional neural networks can incorrectly favor poses that have all the right components but are fundamentally incorrect overall. Figure 18 illustrates. Both the pose on the left and the pose on right have the same parts, two eyes, two eyebrows, a nose lips, and the overall shape of a head. Teaching the convolutional neural network that the pose on the left, therefore, is the correct one can prove to be difficult. Because of this, there is an inherent pose insensitivity in conventional vHTS machine learning methods. This pose insensitivity can lead to the incorrect or inaccurate characterization of the interaction between a test compound and a target polymer. For instance, this pose insensitivity can lead a vHTS machine learning approach that provides a categorical activity label for each compound in a screening library to incorrectly label a certain percentage of the compounds in the screening library.

Given the above background, what is needed in the art are methods for imposing pose sensitivity on vHTS machine learning methods.

### SUMMARY

The present disclosure addresses the problems identified in the background by conditioning vHTS machine learning models so that they are pose sensitive. Such models are trained on training compound for which the characterization of the interaction between the respective training compound and the target polymer are known. However, for each such training compounds the vHTS machine learning models are trained on both a positive pose of the training compound and a negative pose of the training compound, where such positive and negative poses are selected using an independent pose generation process. In this way, vHTS machine learning models are trained to be pose sensitive. The invention is defined in the appended claims.

Accordingly, one aspect of the present invention isa computer system according to claim 1 for providing a characterization of an interaction between a test compound and a target polymer. The computer system comprises one or more processors and memory addressable by the one or more processors. The memory stores at least one program for execution by the one or more processors. In some embodiments, the characterization of the interaction between the test compound and the target polymer is a binary activity score. In some embodiments, the target polymer is a protein, a polypeptide, a polynucleic acid, a polyribonucleic acid, a polysaccharide, or an assembly of any combination thereof. In accordance with the present invention, a plurality of atomic coordinates for the target polymer is obtained, wherein the plurality of atomic coordinates comprises atomic coordinates for at least 400 atoms.

In some embodiments, the plurality of atomic coordinates is a set of three-dimensional coordinates {x₁, ..., x_{N}} for a crystal structure of the target polymer resolved at a resolution of 2.5 Å or better or a resolution of 3.3 Å or better.

In some embodiments, the plurality of atomic coordinates for the target polymer comprises an ensemble of three-dimensional coordinates for the target polymer determined by nuclear magnetic resonance, neutron diffraction, or cryo-electron microscopy.

In some embodiments, the characterization of the interaction between the test compound and the target polymer is a binary score, where a first value for the binary score represents an IC₅₀, EC₅₀, Kd, KI, or pKI for the test compound with respect to the target polymer that is above a first threshold, and a second value for the binary score represents an IC₅₀, EC₅₀, Kd, KI, or pKI for the test compound with respect to the target polymer that is below the first threshold.

In accordance with the present invention, a training dataset is obtained that comprises a respective electronic description of each training compound in a plurality of training compounds, wherein the plurality of training compounds comprises at least 100 compounds. Each respective electronic description comprises (i) a corresponding positive pose of the corresponding training compound with respect to the plurality of atomic spatial coordinates coupled with a corresponding first positive interaction score, and (ii) a corresponding negative pose of the corresponding training compound with respect to the plurality of atomic spatial coordinates coupled with a corresponding first negative interaction score.

In some embodiments, the corresponding positive score for the corresponding positive pose of the corresponding training compound with respect to the target polymer is obtained by retrieving a corresponding positive voxel map of the corresponding training compound with respect to the target polymer in the corresponding positive pose, unfolding the corresponding positive voxel map into a corresponding positive vector, and inputting the corresponding positive vector to a neural network thereby obtaining the corresponding positive score for the corresponding positive pose. In some embodiments the neural network comprises more than 500 parameters. In some such embodiments, the corresponding positive vector is a first one-dimensional vector.

In some embodiments, the corresponding negative score for the corresponding negative pose of the corresponding training compound with respect to the target polymer is obtained by retrieving a corresponding negative voxel map of the corresponding training compound with respect to the target polymer in the corresponding negative pose, unfolding the corresponding negative voxel map into a corresponding negative vector, and inputting the corresponding negative vector to the neural network thereby obtaining the corresponding negative score for the corresponding negative pose of the corresponding training compound with respect to the target polymer.

In some such embodiments, the corresponding negative vector is a second one-dimensional vector.

In all embodiments of the invention, the corresponding first positive interaction score and the corresponding first negative interaction score each represent a binding coefficient or an *in silico* pose quality score of the corresponding training compound to the target polymer.

In some embodiments, each training compound in the training dataset satisfies two or more rules, three or more rules, or all four rules of the Lipinski's rule of Five: (i) not more than five hydrogen bond donors, (ii) not more than ten hydrogen bond acceptors, (iii) a molecular weight under 500 Daltons, and (iv) a LogP under 5.

In some embodiments, each training compound in the training dataset is an organic compound that has a molecular weight of less than 500 Daltons, less than 1000 Daltons, less than 2000 Daltons, less than 4000 Daltons, less than 6000 Daltons, less than 8000 Daltons, less than 10000 Daltons, or less than 20000 Daltons.

In accordance with the present invention, at least a first model is trained. The first model has a first plurality of parameters, wherein the first plurality of parameters comprises more than 400 parameters. The training comprises, for each corresponding training compound in the plurality of training compounds: (i) comparing an output from the first model, upon inputting a corresponding positive score for the corresponding positive pose of the corresponding training compound with respect to the target polymer into the first model, with the corresponding first positive interaction score of the corresponding training compound with respect to the target polymer, wherein the corresponding positive score is obtained by inputting the corresponding positive pose into a trained neural network, and (ii) comparing an output from the first model, upon inputting a corresponding negative score for the corresponding negative pose of the corresponding training compound with respect to the target polymer into the first model, with the corresponding first negative interaction score of the corresponding training compound with respect to the target polymer, wherein the corresponding negative score is obtained by inputting the corresponding negative pose into the trained neural network, thereby adjusting the first plurality of parameters, wherein at least an output of the first model provides a binding coefficient or an *in silico* pose quality score for the interaction between the test compound and the target polymer.

In some embodiments, the first model is a first fully connected neural network.

In some embodiments, the training is a regression task in which the first plurality of parameters is adjusted by back-propagation through an associated loss function. In such embodiments, the corresponding first positive interaction score is related to the corresponding first negative interaction score by the expression B = N × A, where A is the corresponding positive interaction score, B is the corresponding negative interaction score, and N is a real number that is greater than zero and less than 1 (*e.g.,* 0.90).

In some such embodiments, the associated loss function is a mean squared error loss function, a mean absolute error loss function, a Huber loss function, a Log-Cosh loss function, or a quantile loss function.

In some such embodiments, the corresponding first positive interaction score and the corresponding first negative interaction score each represent a binding coefficient, and the corresponding first positive interaction score is an *in vitro* measurement of the binding coefficient of the corresponding training compound to the target polymer.

In some such embodiments, the first positive interaction score is an IC₅₀, EC₅₀, Kd, KI, or pKI for the respective training compound with respect to the target polymer.

In some embodiments, each respective electronic description in the training dataset further comprises a corresponding positive activity score for the corresponding positive pose of the corresponding training compound and a corresponding negative activity score for the corresponding negative pose of the corresponding training compound. In such embodiments, the training at least the first model further comprises jointly training a second model with the first model. The second model has a second plurality of parameters. Such training further uses, for each corresponding training compound in the plurality of training compounds, at least: (iii) the corresponding positive score for the corresponding positive pose of the corresponding training compound with respect to the target polymer as input to the second model, against the corresponding positive activity score of the corresponding training compound, and (iv) the corresponding negative score for the corresponding negative pose of the corresponding training compound with respect to the target polymer as input to the second model, against the corresponding negative activity score of the corresponding training compound. In this way, the second plurality of parameters is adjusted so that the second model provides an activity of the interaction between the test compound and the target polymer that is used with the output of the first model, at least in part, to provide the characterization of the interaction between the test compound and the target polymer.

In some such embodiments, the second model is a second fully connected neural network.

In some embodiments, each respective electronic description in the training dataset further comprises a corresponding positive activity score for the corresponding positive pose of the corresponding training compound and a corresponding negative activity score for the corresponding negative pose of the corresponding training compound. In such embodiments, the training at least the first model further comprises jointly training a second model with the first model, where the second model has a second plurality of parameters. The training in such embodiments further uses, for each corresponding training compound in the plurality of training compounds, at least (iii) the corresponding positive score for the corresponding positive pose of the corresponding training compound with respect to the target polymer and the corresponding first positive interaction score as joint input to the second model, against the corresponding positive activity score of the corresponding training compound, and (iv) the corresponding negative score for the corresponding negative pose of the corresponding training compound with respect to the target polymer and the corresponding first negative interaction score as joint input to the second model, against the corresponding negative activity score of the corresponding training compound. In this way the second plurality of parameters is adjusted so that the second model can be used with the output of the first model, at least in part, to provide the characterization of the interaction between the test compound and the target polymer.

In some such embodiments, the corresponding positive activity score is a first binary activity score and the corresponding negative activity score is a second binary activity score. In some embodiments, the corresponding first binary activity score is assigned a value of 1 based on a measured activity of the corresponding compound against the target polymer, and the corresponding second binary activity score is assigned a value of 0. In some such embodiments, the training of the first model is a regression task in which the first plurality of parameters is adjusted by back-propagation through a first associated loss function, and the training of the second model is a classification task in which the second plurality of parameters is adjusted by back-propagation through a second associated loss function. In some such embodiments, the corresponding first positive interaction score and the corresponding first negative interaction score each represent a binding coefficient or an *in silico* pose quality score of the corresponding training compound to the target polymer, and the corresponding positive activity score is a first binary activity score and the corresponding negative activity score is a second binary activity score. In some such embodiments, the first associated loss function is a mean squared error loss function, a mean absolute error loss function, a Huber loss function, a Log-Cosh loss function, or a quantile loss function, and the second associated loss function is a binary cross entropy loss function, a hinge loss function, or a squared hinged loss function. In some such embodiments, the second model is a second fully connected neural network.

In some embodiments, each respective electronic description in the training dataset further comprises a corresponding second positive interaction score for the corresponding positive pose of the corresponding training compound and a corresponding second negative interaction score for the corresponding negative pose of the corresponding training compound. In such embodiments, the respective electronic description in the training dataset further comprises a corresponding positive activity score for the corresponding positive pose of the corresponding training compound and a corresponding negative activity score for the corresponding negative pose of the corresponding training compound. In such embodiments, the training at least the first model further comprises jointly training a second model and a third model with the first model. The second model has a second plurality of parameters and the third model has a third plurality of parameters. In such embodiments, the training further uses, for each corresponding training compound in the plurality of training compounds, at least: (iii) the corresponding positive score for the corresponding positive pose of the corresponding training compound with respect to the target polymer as input to the second model, against the corresponding second positive interaction score of the corresponding training compound with respect to the target polymer, (iv) the corresponding negative score for the corresponding negative pose of the corresponding training compound with respect to the target polymer as input to the second model, against the corresponding second negative interaction score of the corresponding training compound with respect to the target polymer, thereby adjusting the second plurality of parameters, (v) the corresponding positive score for the corresponding positive pose of the corresponding training compound with respect to the target polymer, the output of the first model and second model upon input of the corresponding positive score for the corresponding positive pose of the corresponding training compounds joint input to the third model, against the corresponding positive activity score of the corresponding training compound, and (vi) the corresponding negative score for the corresponding negative pose of the corresponding training compound with respect to the target polymer and the output of the first model and the second model upon input of the corresponding negative score for the corresponding negative pose of the corresponding training compound as joint input to the third model, against the corresponding negative activity score of the corresponding training compound, thereby adjusting the third plurality of parameters of the third model. In such embodiments, the output of the third model provides the characterization of the interaction between the test compound and the target polymer. In some such embodiments, the second model is a second fully connected neural network, and the third model is a third fully connected neural network. In some such embodiments, the corresponding positive activity score is a first binary activity score and the corresponding negative activity score is a second binary activity score. In some embodiments, the corresponding first binary activity score is assigned a value of 1 based on a measured activity of the corresponding compound against the target polymer, and the corresponding second binary activity score is assigned a value of 0. In some such embodiments, the training of the first model is a first regression task in which the first plurality of parameters is adjusted by back-propagation through a first associated loss function, the training of the second model is a second regression task in which the second plurality of parameters is adjusted by back-propagation through a second associated loss function, and the training of the third model is a classification task in which the third plurality of parameters is adjusted by back-propagation through a third associated loss function. In some such embodiments the corresponding first positive interaction score and the corresponding first negative interaction score each represent an *in silico* pose quality score of the corresponding training compound to the target polymer, the corresponding second positive interaction score and the corresponding second negative interaction score each represent a binding coefficient of the corresponding training compound to the target polymer, and the corresponding positive activity score is a first binary activity score and the corresponding negative activity score is a second binary activity score. In some such embodiments, the first associated loss function is a mean squared error loss function, a mean absolute error loss function, a Huber loss function, a Log-Cosh loss function, or a quantile loss function, the second associated loss function is a mean squared error loss function, a mean absolute error loss function, a Huber loss function, a Log-Cosh loss function, or a quantile loss function, and the third associated loss function is a binary cross entropy loss function, a hinge loss function, a squared hinged loss function, or any other loss function described herein as being used as the first or second associated loss function.

Another aspect of the present invention is a method according to claim 14 for characterizing an interaction between a test compound and a target polymer, the method comprising: at a computer system comprising a memory: (A) obtaining a plurality of atomic coordinates for the target polymer, wherein the plurality of atomic coordinates comprises atomic coordinates for at least 400 atoms; (B) obtaining a training dataset comprising a respective electronic description of each training compound in a plurality of training compounds, wherein the plurality of training compounds comprises at least 100 compounds, each respective electronic description comprising: (i) a corresponding positive pose of the corresponding training compound with respect to the plurality of atomic spatial coordinates coupled with a corresponding first positive interaction score that represents a first binding coefficient or an *in silico* pose quality score of the corresponding training compound to the target polymer, and (ii) a corresponding negative pose of the corresponding training compound with respect to the plurality of atomic spatial coordinates coupled with a corresponding first negative interaction score that represents a second binding coefficient or an *in silico* pose quality score of the corresponding training compound to the target polymer, and (C) training at least a first model, wherein the first model has a first plurality of parameters, and wherein the first plurality of parameters comprises more than 400 parameters, the training comprising, for each corresponding training compound in the plurality of training compounds: (i) comparing an output form the first model, upon inputting a corresponding positive score for the corresponding positive pose of the corresponding training compound with respect to the target polymer into the first model, with the corresponding first positive interaction score of the corresponding training compound with respect to the target polymer, wherein the corresponding positive score is obtained by inputting the corresponding positive pose into a trained neural network, and (ii) comparing an output from the second model, upon inputting a corresponding negative score for the corresponding negative pose of the corresponding training compound with respect to the target polymer into the first model, with the corresponding first negative interaction score of the corresponding training compound with respect to the target polymer, wherein the corresponding negative score is obtained by inputting the corresponding negative pose into the trained neural network, thereby adjusting the first plurality of parameters, wherein at least an output of the first model provides a binding coefficient or an *in silico* pose quality score for the interaction between the test compound and the target polymer.

Another aspect of the present invention is a non-transitory computer readable storage medium according to claim 15, wherein the non-transitory computer readable storage medium stores instructions, which when executed by a computer system, cause the computer system to perform a method for characterizing an interaction between a test compound and a target polymer, the method comprising: (A) obtaining a plurality of atomic coordinates for the target polymer, wherein the plurality of atomic coordinates comprises atomic coordinates for at least 400 atoms; (B) obtaining a training dataset comprising a respective electronic description of each training compound in a plurality of training compounds, wherein the plurality of training compounds comprises at least 100 compounds, each respective electronic description comprising: (i) a corresponding positive pose of the corresponding training compound with respect to the plurality of atomic spatial coordinates coupled with a corresponding first positive interaction score that represents a first binding coefficient or an *in silico* pose quality score of the corresponding training compound to the target polymer, and (ii) a corresponding negative pose of the corresponding training compound with respect to the plurality of atomic spatial coordinates coupled with a corresponding first negative interaction score that represents a second binding coefficient or an *in silico* pose quality score of the corresponding training compound to the target polymer, and (C) training at least a first model, wherein the first model has a first plurality of parameters, and wherein the first plurality of parameters comprises more than 400 parameters, the training comprising, for each corresponding training compound in the plurality of training compounds: (i) comparing an output from the first model, upon inputting a corresponding positive score for the corresponding positive pose of the corresponding training compound with respect to the target polymer, into the first model, with the corresponding first positive interaction score of the corresponding training compound with respect to the target polymer, wherein the first corresponding positive score is obtained by inputting the corresponding positive pose into a trained neural network, and (ii) comparing an output from the first model, upon inputting a corresponding negative score for the corresponding negative pose of the corresponding training compound with respect to the target polymer into the first model, with the corresponding first negative interaction score of the corresponding training compound with respect to the target polymer, wherein the corresponding negative score is obtained by inputting the corresponding negative pose into the trained neural network, thereby adjusting the first plurality of parameters, wherein at least an output of the first model provides a binding coefficient or an *in silico* pose quality score for the interaction between the test compound and the target polymer.

### BRIEF DESCRIPTION OF THE DRAWINGS

In the drawings, embodiments of the systems and method of the present disclosure are illustrated by way of example. It is to be expressly understood that the description and drawings are only for the purpose of illustration and as an aid to understanding, and are not intended as a definition of the limits of the systems and methods of the present disclosure.
FIG. 1 illustrates a computer system in accordance with some embodiments of the present disclosure.
FIGS. 2A, 2B, 2C, 2D, 2E, 2F, 2G, 2H, and 21 illustrate methods for characterizing an interaction between a test compound and a target polymer in accordance with some embodiments of the present disclosure.
FIG. 3 is a schematic view of an example training compound in a pose relative to a target polymer in accordance with some embodiments of the present disclosure.
FIG. 4 is a schematic view of a geometric representation of input features in the form of a three-dimensional grid of voxels, in accordance with some embodiments of the present disclosure.
FIG. 5 and FIG. 6 are views of a compound encoded onto a two dimensional grid of voxels, in accordance with some embodiments of the present disclosure.
FIG. 7 is the view of the visualization of FIG. 6, in which the voxels have been numbered, in accordance with some embodiments of the present disclosure.
FIG. 8 is a schematic view of geometric representation of input features in the form of coordinate locations of atom centers, in accordance with some embodiments of the present disclosure.
FIG. 9A is a system for characterizing an interaction between a test compound and a target polymer, where the characterization is a compound binding mode score, and where the system is trained using coupled positive and negative poses for training compounds, in accordance with one embodiment of the present disclosure.
FIG. 9B is a system for characterizing an interaction between a test compound and a target polymer, where the characterization is activity and a compound binding mode score, and where the system is trained using coupled positive and negative poses for training compounds, in accordance with one embodiment of the present disclosure.
FIG. 9C is a system for characterizing an interaction between a test compound and a target polymer, where the characterization is activity, and where the system is trained using coupled positive and negative poses for training compounds, and where the final output model is conditioned on two different pose quality models in accordance with one embodiment of the present disclosure.
FIG. 10 is a system for characterizing an interaction between a test compound and a target polymer, where the characterization is (i) binary-discrete activity and (ii) pKi, and where the system is trained using coupled positive and negative poses for training compounds, in accordance with one embodiment of the present disclosure.
FIG. 11 is a system for characterizing an interaction between a test compound and a target polymer, where the characterization is pKi, and where the pKi is conditioned, in part, on activity, and where the system is trained using coupled positive and negative poses for training compounds, in accordance with one embodiment of the present disclosure.
FIG. 12 is a system for characterizing an interaction between a test compound and a target polymer, where the characterization is activity, and where the activity is conditioned, in part, on both pKi, and a pose quality score, and where the system is trained using coupled positive and negative poses for training compounds, in accordance with one embodiment of the present disclosure.
FIG. 13 is a system for characterizing an interaction between a test compound and a target polymer, where the characterization is activity, and where the activity is conditioned, in part, on both pKi and binding mode score, and where the system is trained using coupled positive and negative poses for training compounds, in accordance with one embodiment of the present disclosure.
FIG. 14 is a system for characterizing an interaction between a test compound and a target polymer, where the characterization is activity and two different compound binding mode scores, and where the system is trained using coupled positive and negative poses for training compounds, in accordance with one embodiment of the present disclosure.
FIG. 15 is a system for characterizing an interaction between a test compound and a target polymer, where the characterization is activity, two different compound binding mode scores and pKi, and where the system is trained using coupled positive and negative poses for training compounds, in accordance with one embodiment of the present disclosure.
FIG. 16A is a system for characterizing an interaction between a test compound and a target polymer, where the characterization is activity, and where the activity is conditioned, in part, on pKi and a binding mode score, and where the system is trained using coupled positive and negative poses for training compounds, in accordance with one embodiment of the present disclosure.
FIG. 16B is a system for characterizing an interaction between a test compound and a target polymer, where the characterization is activity, and where the activity is conditioned, in part, on pKi and two different binding mode scores, and where the system is trained using coupled positive and negative poses for training compounds, in accordance with one embodiment of the present disclosure.
FIG. 17 is a depiction of applying multiple function computation elements (g₁, g₂, ...) to the voxel inputs (x₁, x₂, ... , x₁₀₀) and composing the function computation element outputs together using g(), in accordance with some embodiments of the present disclosure.
FIG. 18 illustrates the insensitivity that machine learning models face when characterizing a pose of a compound with respect to a target polymer in accordance with the prior art.
FIG. 19 illustrates the insensitivity of conventional machine learning models to the quality of the compound-polymer pose, where, as illustrated, the best possible pose receives the same score by a machine learning model as the poor pose, and where an implausible pose receives the same score by the machine learning model as the best possible pose, in accordance with the prior art.
FIG. 20 illustrates Human ZAP 70 protein with annotated ATP binding site (in grey), allosteric site (in red), and a control binding site at the SH2 domain (in blue). PDB ID used: 2ozo.
FIG. 21 illustrates receiver operator curve AUC performance various benchmark in accordance with an embodiment of the present disclosure.
FIG. 22 illustrates a Picasso problem experiment in which 10⁵ diverse compounds (labeled as 0, non-binders) mixed with *c.a.* 300 kinase inhibitors (labeled as 1, binders) were docked and scored with three binding sites i) ATP binding site, ii) allosteric binding site, and iii) binding site at the SH2 domain in accordance with an embodiment of the present disclosure.
FIG. 23 illustrates Median probability drops between good and poor (left panel) or implausible poses (right panel) in accordance with an embodiment of the present disclosure.
FIG. 24 illustrates an active task conditioned on PoseRanker and Vina scores in accordance with an embodiment of the present disclosure.

Like reference numerals refer to corresponding parts throughout the several views of the drawings.

### DETAILED DESCRIPTION

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

It will also be understood that, although the terms first, second, etc. may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject.

The terminology used in the present disclosure is for the purpose of describing particular embodiments only and is not intended to be limiting of the invention. As used in the description of the invention and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

The present disclosure provides systems and methods for characterizing an interaction between a test compound and a polymer using coordinates for the polymer and a training dataset of compounds. Each respective training compound has a positive pose with respect to target polymer coordinates with a positive interaction score. At least some of the respective training compounds in the training dataset of compounds also have a negative pose of the respective training compound with respect to the target polymer coordinates and a negative interaction score. The model is trained by applying, for each respective compound in the training set, at least: (i) a positive score for the positive pose as input to the model, against the positive interaction score of the compound, and (ii), if available, a negative score for the negative pose as input to the model, against the negative interaction score of the compound, thereby adjusting parameters of the model, where at least some of the compounds in the training set have both a positive and negative pose. In some embodiments, at least five percent, ten percent, twenty percent, fifty percent, or seventy percent of the compound in the training set have both a positive pose and a negative pose while the remaining compounds in the training set have only a positive pose. In some embodiments, all of the compound in the training set have both a positive pose and a negative pose.

In some embodiments, the positive score for the positive pose is obtained by forming a corresponding positive voxel map of the respective training compound in the respective positive pose with respect to the polymer. In some embodiments, the corresponding positive voxel map is vectorized and fed into a neural network. In some embodiments, the voxel map is inputted into the neural network without vectorization.

In some embodiments, neural network is convolutional neural network. In some such embodiments, the convolutional neural network comprises an input layer, a plurality of individually weighted convolutional layers, and an output scorer. The convolutional layers include an initial layer and a final layer. Responsive to input, the input layer feeds values into the initial convolutional layer. Each respective convolutional layer, other than the final convolutional layer, feeds intermediate values as a function of the weights of the respective convolutional layer and input values of the respective convolutional layer into another of the convolutional layers. The final convolutional layer feeds values into the scorer as a function of the final layer weights and input values. In this way, the scorer scores the positive pose of the respective compound to arrive at the positive score for the positive pose for the respective compound.

In some embodiments, the negative score for the negative pose is obtained by forming a corresponding negative voxel map of the respective training compound in the respective negative pose with respect to the polymer. In some embodiments, the corresponding negative voxel map is vectorized and fed into the neural network described above *(e.g.,* a convolutional neural network). In some embodiments, the voxel map is inputted into the neural network without vectorization. In this way, the scorer scores the negative pose of the respective compound to arrive at the negative score for the negative pose for the respective compound.

Once the model is trained against the training compounds, the model can be used to characterize the interaction between a test compound and the polymer. In some embodiments, responsive to a positive pose of the test compound and the target polymer being inputted into the neural network, a score of the positive pose is provided by the neural network and the second (or third, fourth, ... x^{th}) model. The score of the positive pose provided by the neural network, upon conditioning via an embedding layer, serves as input into the trained model which, in turn, provides the characterization of the interaction between the test compound and the polymer.

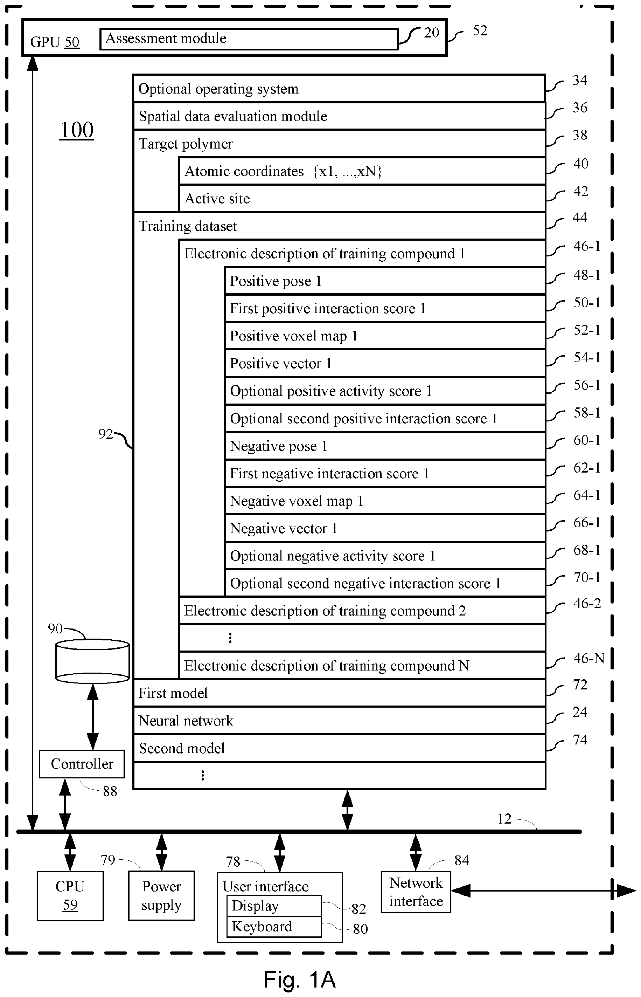

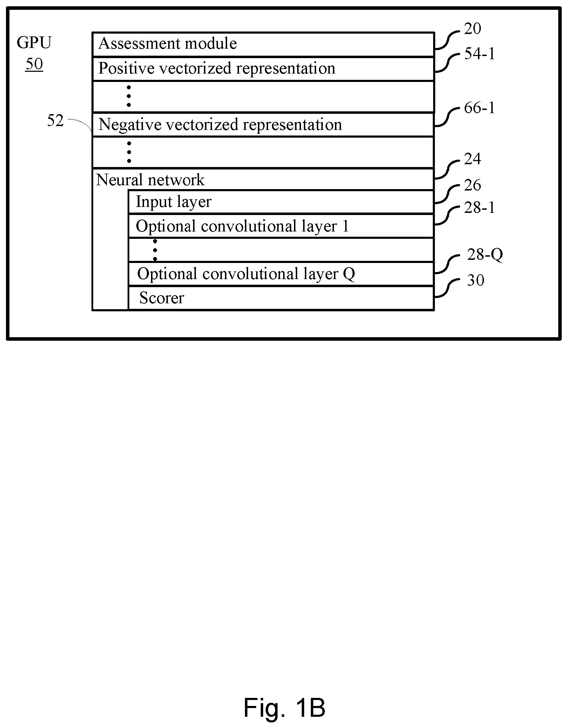

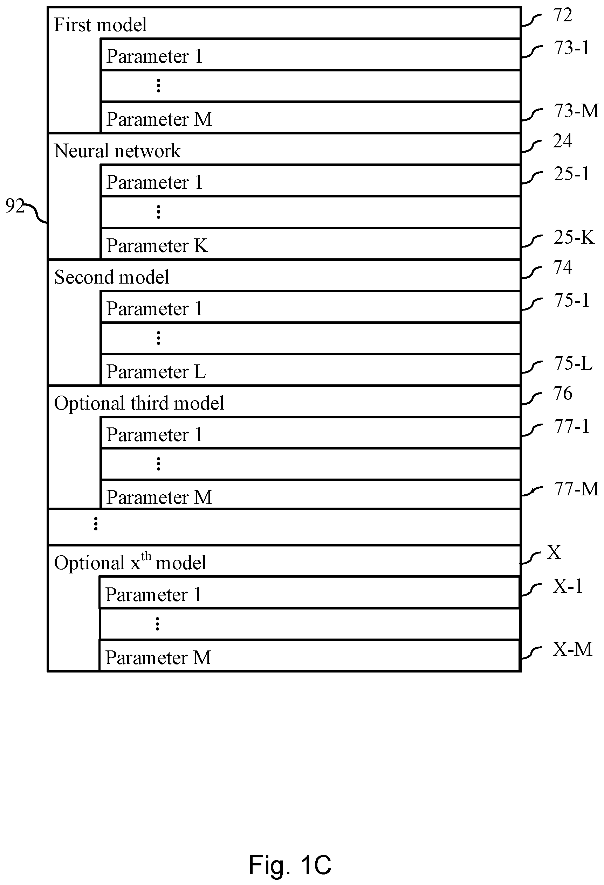

Figure 1 illustrates a computer system 100 for characterizing an interaction between a test compound and a target polymer. For instance, it can be used as a binding affinity prediction system to generate accurate predictions regarding the binding affinity of one or more test compounds with a target polymer.

Referring to Figure 1, in typical embodiments, computer system 100 comprises one or more computers. For purposes of illustration in Figure 1, the computer system 100 is represented as a single computer that includes all of the functionality of the disclosed computer system 100. However, the disclosure is not so limited. The functionality of the computer system 100 may be spread across any number of networked computers and/or reside on each of several networked computers and/or virtual machines. One of skill in the art will appreciate that a wide array of different computer topologies are possible for the computer system 100 and all such topologies are within the scope of the present disclosure.

Turning to Figure 1 with the foregoing in mind, the computer system 100 comprises one or more processing units (CPU's) 59, a network or other communications interface 84, a user interface 78 (*e.g*., including a display 82 and optional keyboard 80 or other form of input device), a memory 92 (*e.g.,* random access memory), one or more magnetic disk storage and/or persistent devices 90 optionally accessed by one or more controllers 88, one or more communication busses 12 for interconnecting the aforementioned components, and a power supply 79 for powering the aforementioned components. Data in memory 92 can be seamlessly shared with non-volatile memory 90 using known computing techniques such as caching. Memory 92 and/or memory 90 can include mass storage that is remotely located with respect to the central processing unit(s) 59. In other words, some data stored in memory 92 and/or memory 90 may in fact be hosted on computers that are external to computer system 100 but that can be electronically accessed by the computer system 100 over an Internet, intranet, or other form of network or electronic cable using network interface 84. In some embodiments, the computer system 100 makes use of a neural network that is run from the memory 52 associated with one or more graphical processing units 50 in order to improve the speed and performance of the system. In some alternative embodiments, the computer system 100 makes use of a neural network that is run from memory 92 rather than memory associated with a graphical processing unit 50.

The memory 92, and/or optionally memory 52, of the computer system 100 stores:
- an optional operating system 34 that includes procedures for handling various basic system services;
- a spatial data evaluation module 36 for characterizing an interaction between a test compound and a target polymer;
- data for a target polymer 38, including structural data (*e.g.,* a plurality of atomic spatial coordinates 40 of the target polymer) and/or optionally active site information 42 of the target polymer;
- a training dataset 44 comprising a respective electronic description 46 of each training compound in a plurality of training compounds, each respective electronic description in at least a subset of the training dataset 44 comprising (i) a corresponding positive pose 48 of the corresponding training compound with respect to the plurality of atomic spatial coordinates 40 coupled with a corresponding first positive interaction score 50, and (ii) a corresponding negative pose 60 of the corresponding training compound with respect to the plurality of atomic spatial coordinates 40 coupled with a corresponding first negative interaction score 62;
- a first model 72 comprising a first plurality of parameters 73, where an output of the first model is used, at least in part, to provide a characterization of the interaction between the test compound and the target polymer;
- an assessment module 20 for applying a neural network 24 to spatial data (*e.g.,* for applying a neural network to test or training compound docked onto a target polymer);
- one or more (optionally) vectorized 54/66 representations of voxel maps;
- a neural network 24 that optionally includes an input layer 26, optionally includes one or more convolutional layers 28, and a terminal scorer 30;
- a second model 74 comprising a second plurality of parameters 75, where an output of the second model is used, at least in part, to (i) provide a characterization of the interaction between the test compound and the target polymer and/or (ii) is used to condition the first model;
- optionally a third model 76 comprising a third plurality of parameters 77, where an output of the third model is used, at least in part, to (i) provide a characterization of the interaction between the test compound and the target polymer and/or (ii) is used to condition the first model and/or the second model; and
- optionally, any number of additional x^{th} models, each such additional x^{th} model comprising a corresponding plurality of parameters, where an output of the additional x^{th} model is used, at least in part, to (i) provide a characterization of the interaction between the test compound and the target polymer and/or (ii) is used to condition any other single model and/or group of models.

In some implementations, one or more of the above identified data elements or modules of the computer system 100 are stored in one or more of the previously mentioned memory devices, and correspond to a set of instructions for performing a function described above. The above identified data, modules or programs (*e.g*., sets of instructions) need not be implemented as separate software programs, procedures or modules, and thus various subsets of these modules may be combined or otherwise re-arranged in various implementations. In some implementations, the memory 92 and/or 90 (and optionally 52) optionally stores a subset of the modules and data structures identified above. Furthermore, in some embodiments the memory 92 and/or 90 (and optionally 52) stores additional modules and data structures not described above.

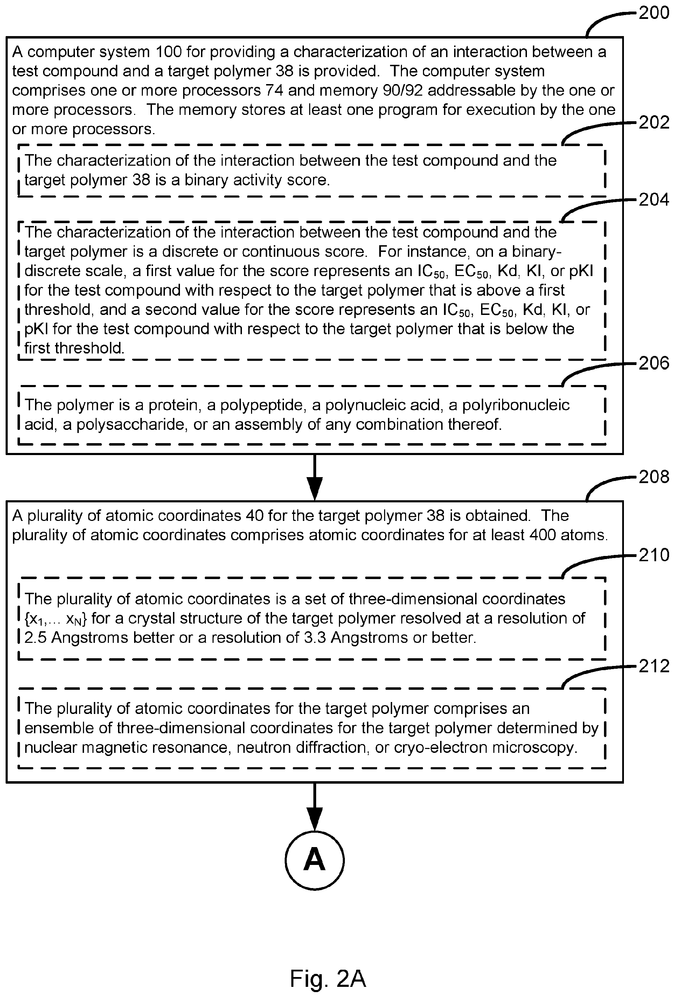

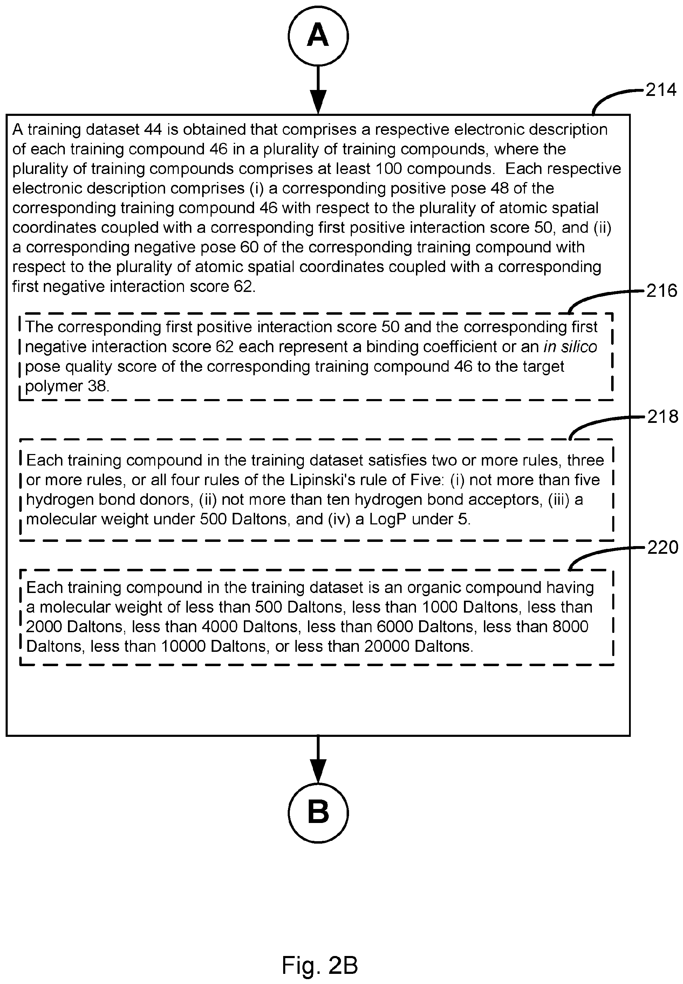

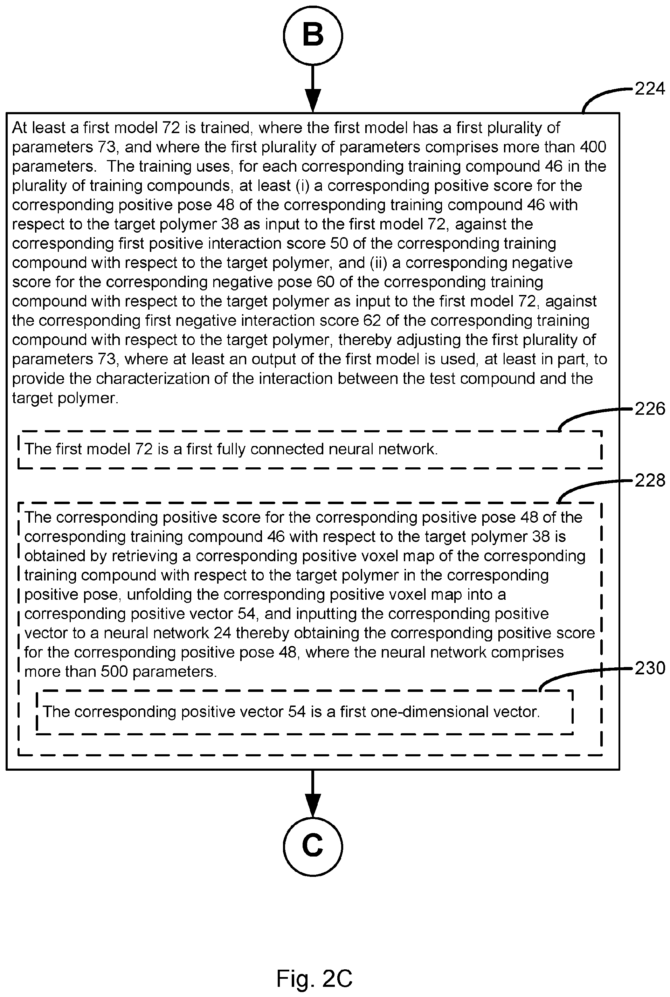

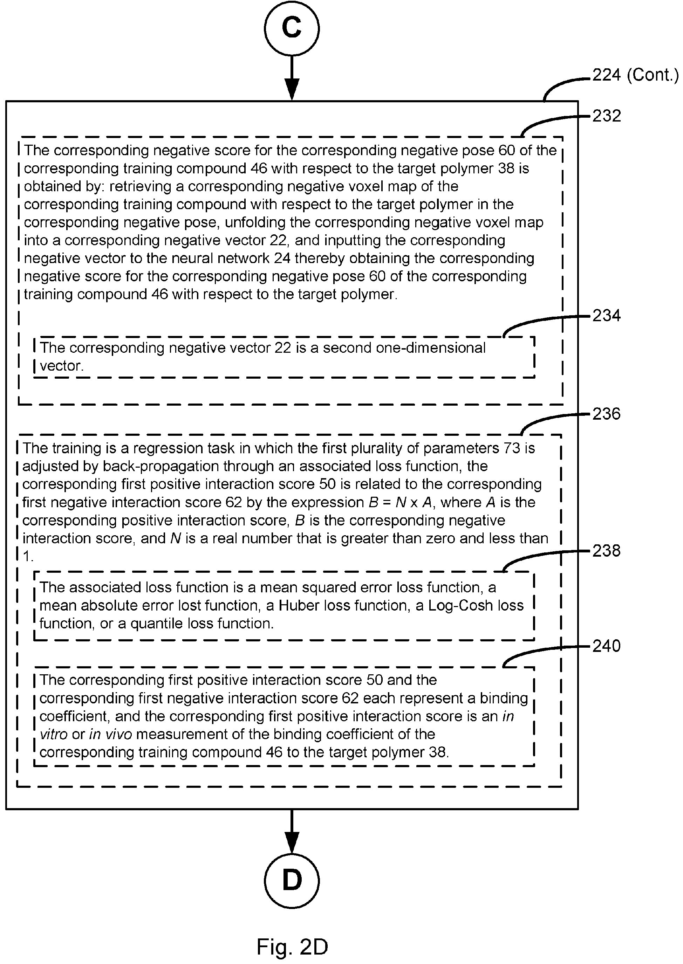

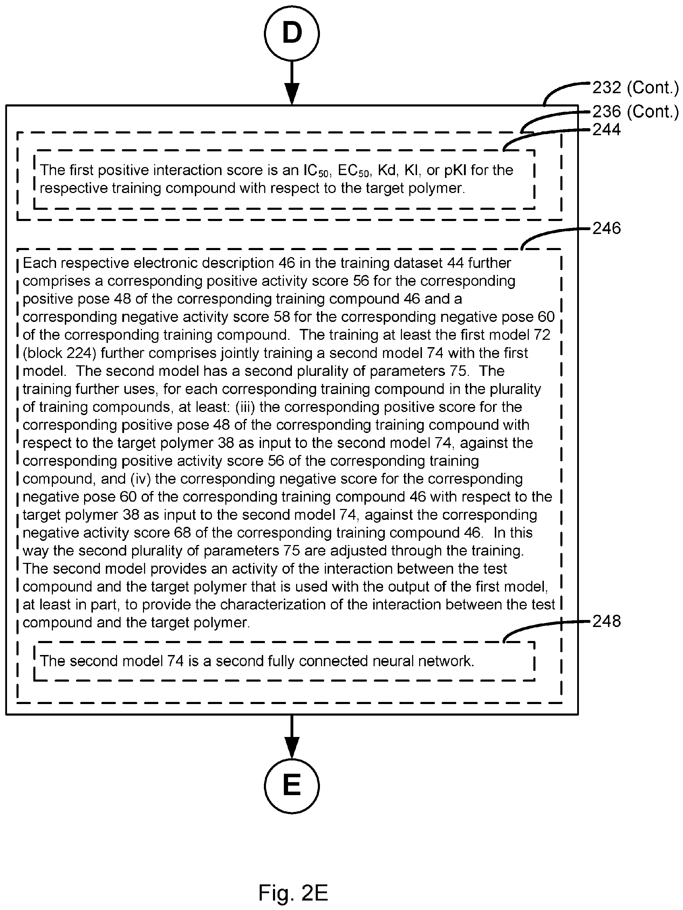

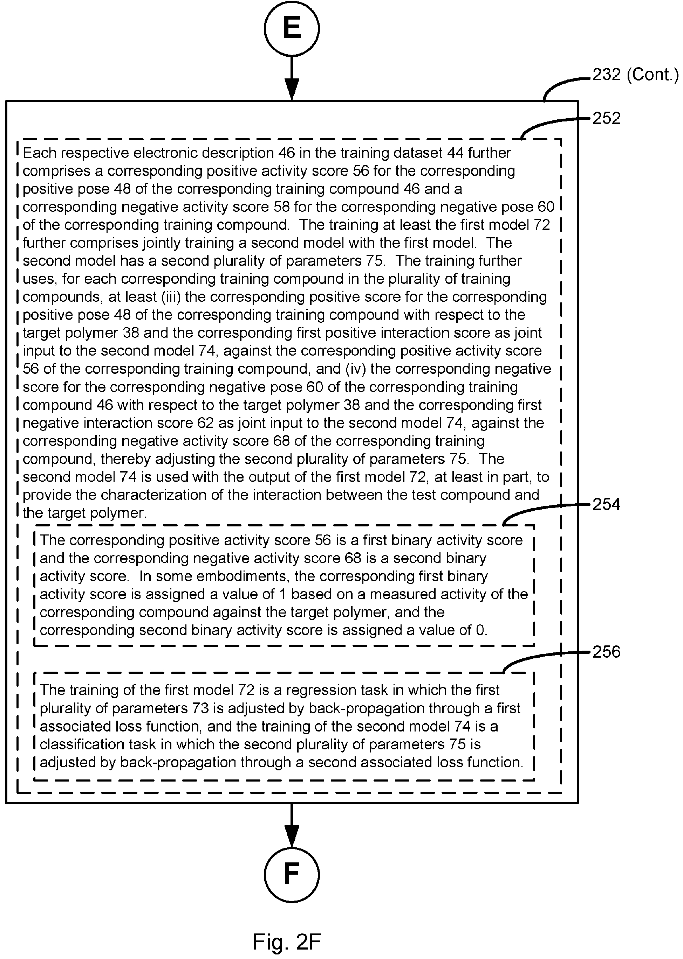

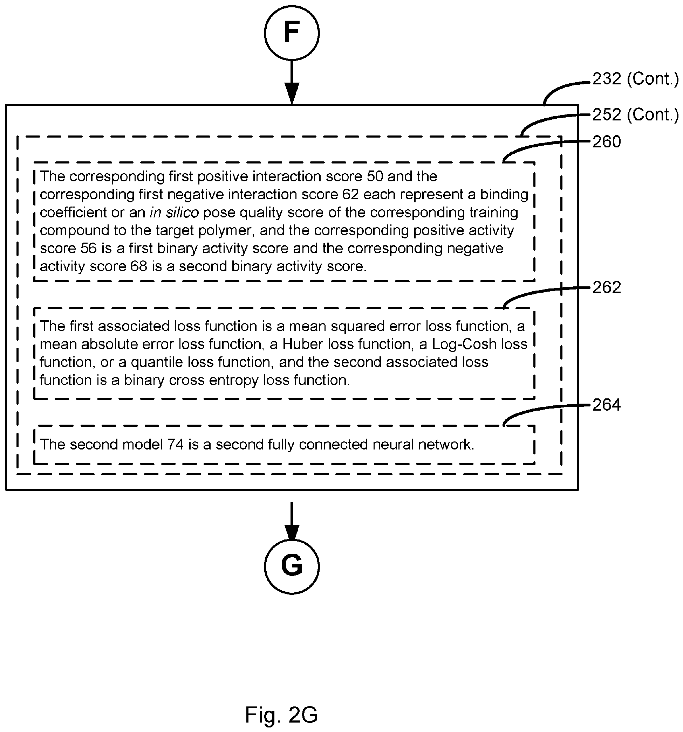

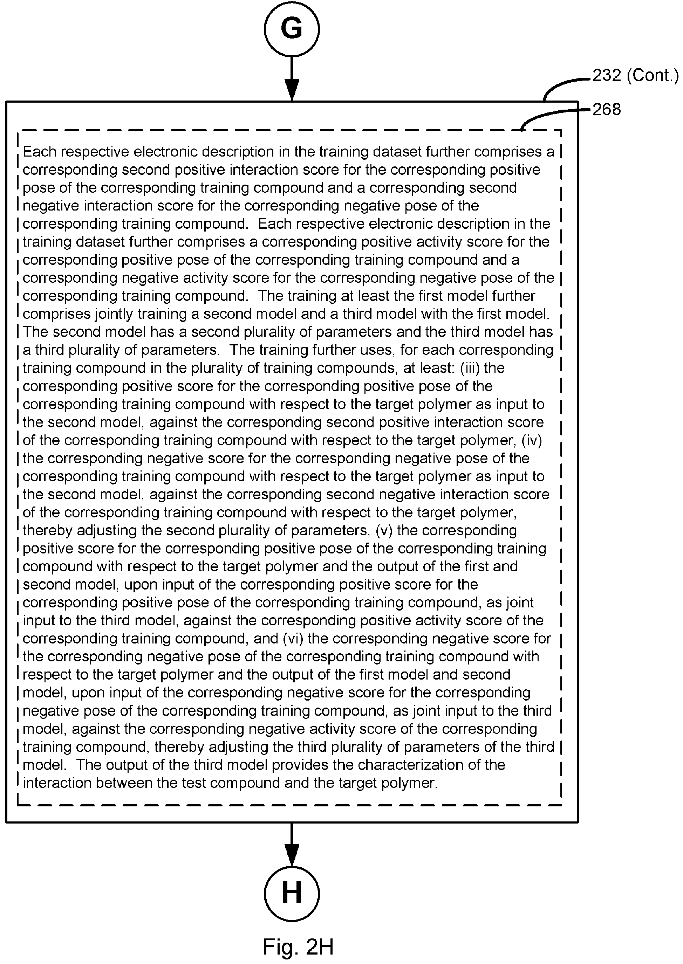

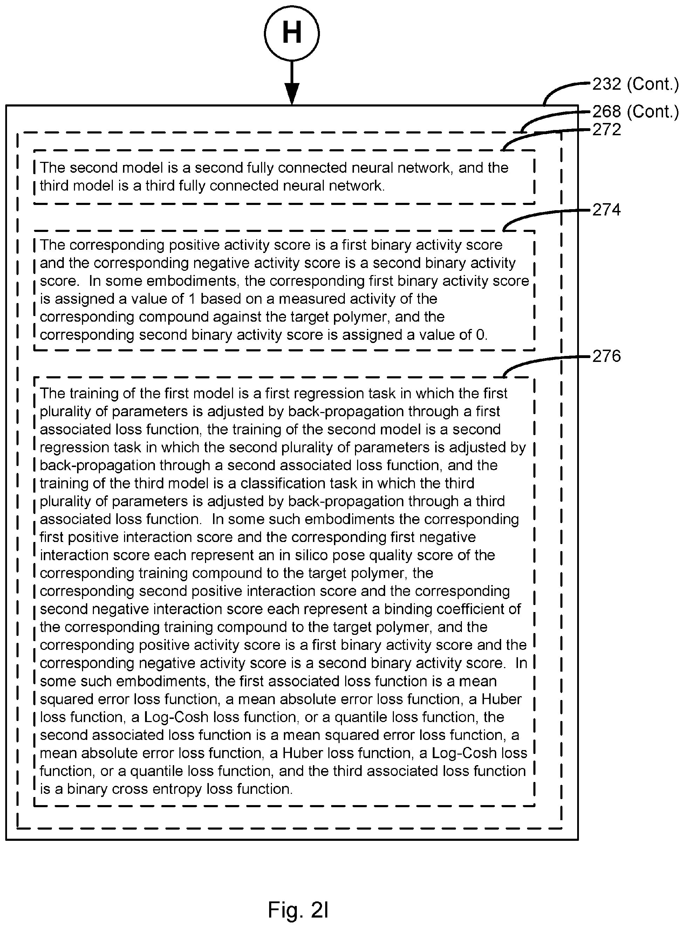

Now that a system for characterizing an interaction between a test compound and a target polymer has been disclosed, methods for performing such characterization is detailed with reference to Figure 2 and discussed below.

*Block 200.* Referring to block 200 of Figure 2A, a computer system 100 is disclosed that provides a characterization of an interaction between a test compound and a target polymer 38. As discussed above in conjunction with Figure 1, the computer system comprises one or more processors 74 and memory 90/92 addressable by the one or more processors. The memory stores at least one program for execution by the one or more processors. The remainder of Figure 2 details features of the at least one program, including the training of the computer system and the use of the trained computer system.

*Blocks 202-204.* Referring to block 202 of Figure 2A, in some embodiments, once trained against reference compounds, the spatial data evaluation module 36 is able to characterize the interaction between a test compound and a target polymer 38. In some such embodiments, this characterization is a discrete (*e.g*., discrete-binary) activity score. In other words, the characterization is categorical. For instance, in some embodiments, the characterization is discrete-binary and the computer system provides one value, *e.g.* a "1", when the test compound is determined, by *in silico* methods implemented in the spatial data evaluation module 36 and discussed in further detail below, to be active against the target polymer and another value, *e.g.* a "0", when the test compound is determined to not be active against the target polymer.

In some embodiments, the characterization is on a discrete scale that is other than binary. For instance, in some embodiments, the characterization provides a first value, *e.g.* a "0", when the test compound is determined, by *in silico* methods implemented in the spatial data evaluation module 36 and discussed in further detail below, to have an activity that falls below a first threshold, a second value, *e.g.* a "1", when the test compound is determined to have an activity that is between a first threshold and a second threshold, and a third value, *e.g.* a "2", when the test compound is determined to have an activity that is above the second threshold. In such embodiments, the first and second threshold are predetermined and constant for a particular experiment (*e.g*., for a particular evaluation of a particular database, set, or collection, of test compounds against a particular target polymer) and are chosen to have values that prove to be useful in identifying suitable test compounds from the particular database, set, or collection of test compounds for activity against the test polymer. For instance, in some embodiments, any of the thresholds disclosed herein are designed to identify 0.1 percent or fewer, 0.5 percent or fewer, 1 percent or fewer, 2 percent or fewer, 5 percent or fewer, 10 percent or fewer, 20 percent or fewer, or 50 percent or fewer of a database of test compounds as being active against the target polymer, where the database of test compounds comprises 100 or more compounds, 1000 or more compounds, 10,000 or more compounds, 100,000 or more compounds, 1 x 10⁶ compounds, 10 x 10⁶ compounds or more.

In alternative embodiments, once trained against reference compounds, the spatial data evaluation module 36 is able to characterize the interaction between a test compound and a target polymer 38 as an activity on a continuous scale. That is, the spatial data evaluation module 36 provides a number on a continuous scale that indicates the activity of the test compound against the target polymer. The activity value on the continuous scale is useful, for instance, in comparing the activity of each test compound in a database of test compounds against the target polymer that was assigned by the trained spatial data evaluation module 36.

Referring to block 204, the disclosed systems and methods are not limited to characterizing the interaction between a test compound and a target polymer 38 as an activity on a continuous scale or discrete scale. In alternative embodiments, the spatial data evaluation module 36 can, in fact, once trained against reference compounds, characterize the interaction between a test compound and a target polymer as an IC₅₀, EC₅₀, Kd, KI, or pKI of the test compound against the target polymer on a continuous scale or a discrete (categorical) scale.

While a binary-discrete scale and a discrete-scale with three possible outcomes has been identified, the present disclosure is not limited to these two examples of discretescales for the characterization of the interaction between a test compound and a target polymer 38. In fact, any discrete scale can be used for the characterization of the interaction between a test compound and a target polymer 38 including, as non-limiting examples, a discrete scale with 4, 5, 6, 7, 8, 9, 10, 11, 12, 13, 14, 15, 16, 17, 18, 19, or 20 different outcomes.

*Block 206.* Referring to block 204 of Figure 2A, in some embodiments, the target polymer 38 is a protein, a polypeptide, a polynucleic acid, a polyribonucleic acid, a polysaccharide, a metalloprotein, or an assembly of any combination thereof. In some embodiments, a target polymer 38 is a large molecule composed of repeating residues. In some embodiments, the target polymer 38 is a natural material. In some embodiments, the target polymer 38 is a synthetic material. In some embodiments, the target polymer 38 is an elastomer, shellac, amber, natural or synthetic rubber, cellulose, Bakelite, nylon, polystyrene, polyethylene, polypropylene, polyacrylonitrile, polyethylene glycol, or a polysaccharide.

In some embodiments, the target polymer 38 is a heteropolymer (copolymer). A copolymer is a polymer derived from two (or more) monomeric species, as opposed to a homopolymer where only one monomer is used. Copolymerization refers to methods used to chemically synthesize a copolymer. Examples of copolymers include, but are not limited to, ABS plastic, SBR, nitrile rubber, styrene-acrylonitrile, styrene-isoprene-styrene (SIS) and ethylene-vinyl acetate. Since a copolymer comprises at least two types of constituent units (also structural units, or particles), copolymers can be classified based on how these units are arranged along the chain. These include alternating copolymers with regular alternating A and B units. *See,* for example, Jenkins, 1996, "Glossary of Basic Terms in Polymer Science," Pure Appl. Chem. 68 (12): 2287-2311. Additional examples of copolymers are periodic copolymers with A and B units arranged in a repeating sequence (e.g. (A-B-A-B-B-A-A-A-A-B-B-B)ₙ). Additional examples of copolymers are statistical copolymers in which the sequence of monomer residues in the copolymer follows a statistical rule. *See,* for example, Painter, 1997, Fundamentals of Polymer Science, CRC Press, 1997, p. 14. Still other examples of copolymers that may be evaluated using the disclosed systems and methods are block copolymers comprising two or more homopolymer subunits linked by covalent bonds. The union of the homopolymer subunits may require an intermediate non-repeating subunit, known as a junction block. Block copolymers with two or three distinct blocks are called diblock copolymers and triblock copolymers, respectively.

In some embodiments, the target polymer 38 comprises 50 or more, 100 or more, 150 or more, 200 or more, 300 or more, 400 or more, 500 or more, 600 or more, 700 or more, 800 or more, 900 or more, or 1000 or more atoms.

In some embodiments, the target polymer 38 is in fact a plurality of polymers *(e.g.,* 2 or more, 3, or more, 10 or more, 100 or more, 1000 or more, or 5000 or more polymers), where the respective polymers in the plurality of polymers do not all have the same molecular weight. In some such embodiments, the target polymers 38 in the plurality of polymers share at least 50 percent, at least 60 percent, at least 70 percent, at least 80 percent, or at least 90 percent sequence identity and fall into a weight range with a corresponding distribution of chain lengths. In some embodiments, the target polymer 38 is a branched polymer molecule comprising a main chain with one or more substituent side chains or branches. Types of branched polymers include, but are not limited to, star polymers, comb polymers, brush polymers, dendronized polymers, ladders, and dendrimers. *See,* for example, Rubinstein et al., 2003, Polymer physics, Oxford ; New York: Oxford University Press. p. 6.

In some embodiments, the target polymer is a polypeptide. As used herein, the term "polypeptide" means two or more amino acids or residues linked by a peptide bond. The terms "polypeptide" and "protein" are used interchangeably herein and include oligopeptides and peptides. An "amino acid," "residue" or "peptide" refers to any of the twenty standard structural units of proteins as known in the art, which include imino acids, such as proline and hydroxyproline. The designation of an amino acid isomer may include D, L, Rand S. The definition of amino acid includes nonnatural amino acids. Thus, selenocysteine, pyrrolysine, lanthionine, 2-aminoisobutyric acid, gamma-aminobutyric acid, dehydroalanine, ornithine, citrulline and homocysteine, as nonlimiting examples, are all considered amino acids. Other variants or analogs of the amino acids are known in the art. Thus, a polypeptide may include synthetic peptidomimetic structures such as peptoids. *See* Simon et al., 1992, Proceedings of the National Academy of Sciences USA, 89, 9367. See also Chin et al., 2003, Science 301, 964; and Chin et al., 2003, Chemistry & Biology 10, 511.

The target polymer 38 evaluated in accordance with some embodiments of the disclosed systems and methods may also have any number of posttranslational modifications. Thus, a target polymer 38 includes those polymers that are modified by acylation, alkylation, amidation, biotinylation, formylation, γ-carboxylation, glutamylation, glycosylation, glycylation, hydroxylation, iodination, isoprenylation, lipoylation, cofactor addition (for example, of a heme, flavin, metal, *etc*.), addition of nucleosides and their derivatives, oxidation, reduction, pegylation, phosphatidylinositol addition, phosphopantetheinylation, phosphorylation, pyroglutamate formation, racemization, addition of amino acids by tRNA (for example, arginylation), sulfation, selenoylation, ISGylation, SUMOylation, ubiquitination, chemical modifications (for example, citrullination and deamidation), and treatment with other enzymes (for example, proteases, phosphotases and kinases). Other types of posttranslational modifications are known in the art and are within the scope of the target polymers 38 of the present disclosure.

In some embodiments, the target polymer 38 is a surfactant. Surfactants are compounds that lower the surface tension of a liquid, the interfacial tension between two liquids, or that between a liquid and a solid. Surfactants may act as detergents, wetting agents, emulsifiers, foaming agents, and dispersants. Surfactants are usually organic compounds that are amphiphilic, meaning they contain both hydrophobic groups (their tails) and hydrophilic groups (their heads). Therefore, a surfactant molecule contains both a water insoluble (or oil soluble) component and a water soluble component. Surfactant molecules will diffuse in water and adsorb at interfaces between air and water or at the interface between oil and water, in the case where water is mixed with oil. The insoluble hydrophobic group may extend out of the bulk water phase, into the air or into the oil phase, while the water soluble head group remains in the water phase. This alignment of surfactant molecules at the surface modifies the surface properties of water at the water/air or water/oil interface.

Examples of ionic surfactants include ionic surfactants such as anionic, cationic, or zwitterionic (ampoteric) surfactants. In some embodiments, the target object 58 is a reverse micelle or liposome.

In some embodiments, the target polymer 38 is a fullerene. A fullerene is any molecule composed entirely of carbon, in the form of a hollow sphere, ellipsoid or tube. Spherical fullerenes are also called buckyballs, and they resemble the balls used in association football. Cylindrical ones are called carbon nanotubes or buckytubes. Fullerenes are similar in structure to graphite, which is composed of stacked graphene sheets of linked hexagonal rings; but they may also contain pentagonal (or sometimes heptagonal) rings.

*Blocks 208-212.* Referring to block 208 of Figure 2A, a plurality of atomic coordinates 40 for the target polymer 38 is obtained. In some embodiments, the plurality of atomic coordinates comprises atomic coordinates for at least 400 atoms of the target polymer. In some embodiments, the plurality of atomic coordinates comprises atomic coordinates for at least 25 atoms, at least 50 atoms, at least 100 atoms, at least 200 atoms, at least 300 atoms, at least 400 atoms, at least 1000 atoms, at least 2000 atoms, or at least 5000 atoms of the target polymer. In some embodiments, only the coordinates of the active site of the target polymer 38 where ligands are expected to bind the target polymer is obtained. Referring to block 210, in some embodiments the plurality of atomic coordinates is a set of three-dimensional coordinates {x₁, ..., x_{N}} for a crystal structure of the target polymer resolved at a resolution of 2.5 Å or better or a resolution of 3.3 Å or better. Referring to block 212, in some embodiments, the plurality of atomic coordinates for the target polymer comprises an ensemble of three-dimensional coordinates for the target polymer determined by nuclear magnetic resonance, neutron diffraction, or cryo-electron microscopy.

In some embodiments, the plurality of atomic coordinates are a set of three-dimensional coordinates {x₁, ..., x_{N}} for a crystal structure of the target polymer 38 resolved (*e.g.,* by X-ray crystallographic techniques) at a resolution of 3.3 Å or better, 3.2 Å or better, 3.1 Å or better, 3.0 Å or better, 2.5 Å or better, 2.2 Å or better, 2.0 Å or better, 1.9 Å or better, 1.85 Å or better, 1.80 Å or better, 1.75 Å or better, or 1.70 Å or better.

In some embodiments, the plurality of atomic coordinates for the target polymer 38 is an ensemble of ten or more, twenty or more or thirty or more three-dimensional coordinates for the target polymer determined by nuclear magnetic resonance where the ensemble has a backbone root mean squared deviation (RMSD) of 1.0 Å or better, 0.9 Å or better, 0.8 Å or better, 0.7 Å or better, 0.6 Å or better, 0.5 Å or better, 0.4 Å or better, 0.3 Å or better, or 0.2 Å or better. In some embodiments, the plurality of atomic coordinates is determined by neutron diffraction or cryo-electron microscopy.

In some embodiments, the target polymer 38 includes two different types of polymers, such as a nucleic acid bound to a polypeptide. In some embodiments, the native target polymer includes two polypeptides bound to each other. In some embodiments, the native target polymer under study includes one or more metal ions (*e.g.* a metalloproteinase with one or more zinc atoms). In such instances, the metal ions and or the organic small molecules may be included in the atomic coordinates 40 for the target polymer.

In some embodiments the target polymer 38 is a polymer and there are ten or more, twenty or more, thirty or more, fifty or more, one hundred or more, between one hundred and one thousand, or less than 500 residues in the target polymer.

In some embodiments, the atomic coordinates of the target polymer 38 are determined using modeling methods such as *ab initio* methods, density functional methods, semi-empirical and empirical methods, molecular mechanics, chemical dynamics, or molecular dynamics.

In some embodiments, the atomic coordinates 40 are represented by the Cartesian coordinates of the centers of the atoms comprising the target polymer 38. In some alternative embodiments, the spatial coordinates 40 for the target polymer 38 are represented by the electron density of the target polymer as measured, for example, by X-ray crystallography. For example, in some embodiments, the atomic coordinates 40 comprise a 2F_{observed}-F_{calculated} electron density map computed using the calculated atomic coordinates of the target polymer 38, where F_{observed} is the observed structure factor amplitudes of the target polymer and Fc is the structure factor amplitudes calculated from the calculated atomic coordinates of the target polymer 38.

In various other embodiments, atomic coordinates 40 for the target polymer 38 are obtained in accordance with block 206 from a variety of sources including, but not limited to, structure ensembles generated by solution NMR, co-complexes as interpreted from X-ray crystallography, neutron diffraction, cryo-electron microscopy, sampling from computational simulations, homology modeling, rotamer library sampling, or any combination thereof.

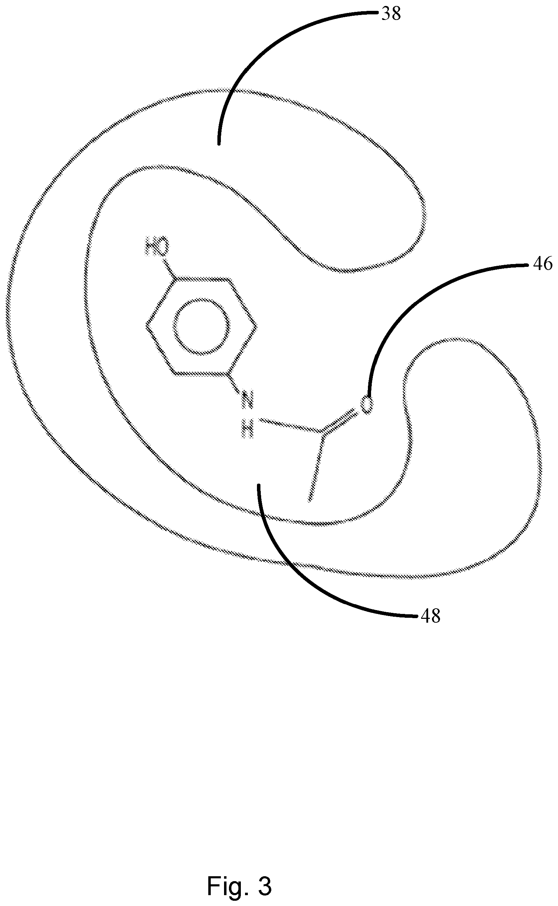

*Block 214.* Referring to block 214 of Figure 2B, a training dataset 44 is obtained that comprises a respective electronic description of each training compound 46 in a plurality of training compounds. In some embodiments, the plurality of training compounds comprises at least 50, 100, 200, 1000, 5000, 10,000, 50,000, 100,000, 1 x 10⁶, 1 x 10⁷, or 1 x 10⁸ training compounds. The respective electronic description of each training compound 46 in at least a subset of the training dataset comprises (i) a corresponding positive pose 48 of the corresponding training compound 46 with respect to the plurality of atomic spatial coordinates coupled with a corresponding first positive interaction score 50, and (ii) a corresponding negative pose 60 of the corresponding training compound with respect to the plurality of atomic spatial coordinates coupled with a corresponding first negative interaction score 62. Figure 3 illustrates a positive pose 48 of a training compound 46 in the active site of a target polymer 38. In some embodiments, some of the training compounds 46 do not have a negative pose 60 and do not have a corresponding first negative interaction score 62. . In some embodiments, some of the training compounds 46 do not have a positive pose 48 and do not have a corresponding first positive interaction score 50. In some embodiments, all of the training compounds 46 have both a positive and negative pose and both a corresponding first positive and first negative interaction score.

In some embodiments, the target polymer 38 is a polymer with an active site, and the positive and negative poses are obtained by docking the training compound into the active site of the polymer. In some embodiments, the training compound is docked onto the target polymer 38 a plurality of times to form a plurality of poses. In some embodiments, each training compound is docked onto the target compound 38 twice, three times, four times, five or more times, ten or more times, fifty or more times, 100 or more times, or a 1000 or more times. Each such docking represents a different pose of the training compound docked onto the target polymer 38. In some embodiments, the target polymer 38 is a polymer with an active site and each training compound is docked into the active site in each of plurality of different ways, each such way representing a different pose. It is expected that many of these poses are not correct, meaning that such poses do not represent true interactions between the training compound and the target polymer that arise in nature.

In some embodiments, each pose of a training compound is determined by AutoDock Vina. *See,* Trott and Olson, "AutoDock Vina: improving the speed and accuracy of docking with a new scoring function, efficient optimization and multithreading," Journal of Computational Chemistry 31 (2010) 455-461. In such embodiments, for a respective training compound, the pose that received the best score by AutoDock Vina is assigned the positive pose 48 and the pose that received the worst score by AutoDock Vina is assigned the negative pose 60. In some embodiments, a different docking program is used to determine the positive pose 48 and the negative pose 60 of a respective training compound. For instance, in some embodiments, Quick Vina 2 (Alhossary et al., 2015, "Fast, accurate, and reliable molecular docking with QuickVina," Bioinformatics 31:13, pp. 2214-2216), VinaLC (Zhang et al., 2013, "Message Passing Interface and Multithreading Hybrid for Parallel Molecular Docking of Large Databases on Petascale High Performance Computing Machines," J. Comput. Chem. DOI: 10.1002/jcc.23214), Smina (Koes et al,, 2013, "Lessons learned in empirical scoring with smina from the CSAR 2011 benchmarking exercise," Journal of chemical information and modeling 53:8, pp. 1893-1904), or Cuina (Morrison et al.., "Efficient GPU Implementation of AutoDock Vina," COMP poster 3432389) is used.

In some embodiments, the positive pose 48 is a positive ensemble of poses and the negative pose 60 is a negative ensemble of poses. For instance, in some embodiments, the positive pose 48 is a corresponding first ensemble of between 2 and 500 structurally similar poses and the negative pose 48 is a corresponding second ensemble of between 2 and 500 structurally similar poses, where the corresponding first ensemble has a better overall docking score than the corresponding second ensemble. Methods for obtaining such ensembles are disclosed in Stafford et al., 2019, "Modeling protein flexibility with conformational sampling improves ligand pose and bioactivity prediction," Abstracts of Papers of the American Chemical Society, Volume 258. In some embodiments each corresponding first ensemble (collectively representing the positive pose 48) is between 2 and 30, between 2 and 20, between 2 and 10, more than 100, between 2 and 1000 structurally similar poses. In some embodiments each corresponding second ensemble (collectively representing the negative pose 48) is between 2 and 30, between 2 and 20, between 2 and 10, more than 100, between 2 and 1000 structurally similar poses.

In some embodiments, each pose (for instance in an ensemble of poses) is scored against several different conformations (*e.g*., between 2 and 100) of the target protein. In some embodiments, each pose (for instance in an ensemble of poses) is scored against a fixed conformation of the target protein.

In some embodiments, training compounds are docked to the target polymer 38 by either random pose generation techniques, or by biased pose generation. In some embodiments, training compounds are docked to the target polymer 38 by Markov chain Monte Carlo sampling. In some embodiments, such sampling allows the full flexibility of training compounds in the docking calculations and a scoring function that is the sum of the interaction energy between the training compound and the target polymer 38 as well as the conformational energy of the training (or test) object. *See,* for example, Liu and Wang, 1999, "MCDOCK: A Monte Carlo simulation approach to the molecular docking problem," Journal of Computer-Aided Molecular Design 13, 435-451. In such embodiments, for a given training compound, the pose that received the best docking score is assigned the positive pose 48 and the pose that received the worst docking score is assigned the positive pose.

In some embodiments, algorithms such as DOCK (Shoichet, Bodian, and Kuntz, 1992, "Molecular docking using shape descriptors," Journal of Computational Chemistry 13(3), pp. 380-397; and Knegtel, Kuntz, and Oshiro, 1997 "Molecular docking to ensembles of protein structures," Journal of Molecular Biology 266, pp. 424-440) are used to find a plurality of poses for each of the training compounds against the target polymer 38. Such algorithms model the target polymer 38 and the training compound as rigid bodies. The docked conformation is searched using surface complementary to find poses.

In some embodiments, algorithms such as AutoDOCK (Morris et al., 2009, "AutoDock4 and AutoDockTools4: Automated Docking with Selective Receptor Flexibility," J. Comput. Chem. 30(16), pp. 2785-2791; Sotriffer et al., 2000, "Automated docking of ligands to antibodies: methods and applications," Methods: A Companion to Methods in Enzymology 20, pp. 280-291; and "Morris et al., 1998, "Automated Docking Using a Lamarckian Genetic Algorithm and Empirical Binding Free Energy Function," Journal of Computational Chemistry 19: pp. 1639-1662) are used to find a plurality of poses for each of the training compounds against the target polymer 38. AutoDOCK uses a kinematic model of the ligand and supports Monte Carlo, simulated annealing, the Lamarckian Genetic Algorithm, and Genetic algorithms. Accordingly, in some embodiments the plurality of different poses (for a given training compound) are obtained by Markov chain Monte Carlo sampling, simulated annealing, Lamarckian Genetic Algorithms, or genetic algorithms, using a docking scoring function.

In some embodiments, algorithms such as FlexX (Rarey et al., 1996, "A Fast Flexible Docking Method Using an Incremental Construction Algorithm," Journal of Molecular Biology 261, pp. 470-489) are used to find a plurality of poses for each training compound against the target polymer. FlexX does an incremental construction of the training compound at the active site of the target polymer 38 using a greedy algorithm. Accordingly, in some embodiments, the plurality of different poses (for a given target compound) are obtained by a greedy algorithm.

In some embodiments, algorithms such as GOLD (Jones et al., 1997, "Development and Validation of a Genetic Algorithm for flexible Docking," Journal Molecular Biology 267, pp. 727-748) are used to find a plurality of poses for each of the training compounds against the target polymer 38. GOLD stands for Genetic Optimization for Ligand Docking. GOLD builds a genetically optimized hydrogen bonding network between the training compound and the target polymer 38.

In some embodiments, molecular dynamics is performed on the target polymer (or a portion thereof such as the active site of the target polymer) and each respective training compound to identify the positive pose 48 and the negative pose 60 for each respective training compound. During the molecular dynamics run, the atoms of the target polymer and the training compound are allowed to interact for a fixed period of time, giving a view of the dynamical evolution of the system. The trajectory of atoms in the target polymer and the training compound are determined by numerically solving Newton's equations of motion for a system of interacting particles, where forces between the particles and their potential energies are calculated using interatomic potentials or molecular mechanics force fields. *See* Alder and Wainwright, 1959, "Studies in Molecular Dynamics. I. General Method," J. Chem. Phys. 31 (2): 459; and Bibcode, 1959, J.Ch.Ph. 31, 459A, doi:10.1063/1.1730376. Thus, in this way, the molecular dynamics run produces a trajectory of the target polymer and the respective training compound over time. This trajectory comprises the trajectory of the atoms in the target polymer and the training compound. In some embodiments, a subset of the plurality of different poses is obtained by taking snapshots of this trajectory over a period of time. In some embodiments, poses are obtained from snapshots of several different trajectories, where each trajectory comprises a different molecular dynamics run of the target polymer interacting with the training compound. In some embodiments, prior to a molecular dynamics run, a training compound is first docked into an active site of the target polymer using a docking technique.

In some embodiments, any pair of poses from among the plurality of poses for a respective training compound against the target polymer, in which one pose in the pair of poses has a better docking score than the other pose in the pair can respectively serve as the positive pose 48 and the negative pose 60 for a respective training compound.

*Block 216.* Several different nonlimiting methods and programs for finding poses and determining *in silico* pose quality scores for such poses have been disclosed above in conjunction with block 214 of Figure 2B. In some embodiments, the first positive interaction score 50 of the positive pose 48 is the *in silico* pose quality score computed for the positive pose 48 with respect to the target polymer 38 by any of these nonlimiting methods and programs or any combination thereof, or by any equivalent or similar program. In some embodiments, the positive pose 48 is an ensemble of poses, as discussed above in block 214, and the first positive interaction score 50 of the positive pose 48 is the *in silico* pose quality score computed for the positive pose 48 with respect to the target polymer 38 by any of these nonlimiting methods and programs. Correspondingly, in some embodiments, the first negative interaction score 62 of the negative pose 60 is the *in silico* pose quality score computed for the negative pose 60 with respect to the target polymer 38 by any of these nonlimiting methods and programs. In some embodiments, the negative pose 60 is an ensemble of poses, as discussed above in block 214, and the first negative interaction score 62 of the negative pose 60 is the *in silico* pose quality score computed for the negative pose 60 with respect to the target polymer 38 by any of these nonlimiting methods and programs.

In some embodiments, rather than using an *in silico* pose quality score for a training compound, the first positive interaction score is a measured binding coefficient, IC₅₀, EC₅₀, Kd, KI, or pKI of the corresponding training compound 46 to the target polymer 38 determined by experimental means. Measured binding coefficients, such as IC₅₀, EC₅₀, Kd, KI, and pKI, are generally described in Huser ed., 2006, High-Throughput-Screening in Drug Discovery, Methods and Principles in Medicinal Chemistry 35; and Chen ed., 2019, A Practical Guide to Assay Development and High- Throughput Screening in Drug Discovery*.*

*Block 218.* Referring to block 218 of Figure 2B, in some embodiments, each training compound in the training dataset satisfies any two or more rules, any three or more rules, or all four rules of the Lipinski's rule of Five: (i) not more than five hydrogen bond donors, (ii) not more than ten hydrogen bond acceptors, (iii) a molecular weight under 500 Daltons, and (iv) a LogP under 5. *See,* Lipinski, 1997, Adv. Drug Del. Rev. 23, 3.

In some embodiments, a training compound satisfies one criterion, or more than one criterion, in addition to Lipinski's Rule of Five. For example, in some embodiments, the training compound has five or fewer aromatic rings, four or fewer aromatic rings, three or fewer aromatic rings, or two or fewer aromatic rings. In some embodiments, a training compound is any organic compound having a molecular weight of less than 2000 Daltons, of less than 4000 Daltons, of less than 6000 Daltons, of less than 8000 Daltons, of less than 10000 Daltons, or less than 20000 Daltons.

However, some embodiments of the disclosed systems and methods have no limitation on the size of training compounds. For instance, in some embodiments, such training compounds are large polymers, such as antibodies.

Referring to block 220, in some embodiments, each training compound in the training dataset is an organic compound having a molecular weight of less than 500 Daltons, less than 1000 Daltons, less than 2000 Daltons, less than 4000 Daltons, less than 6000 Daltons, less than 8000 Daltons, less than 10000 Daltons, or less than 20000 Daltons.

*Blocks 224-226.* Referring to block 224, in the method, at least a first model 72 is trained. The training uses, for each corresponding training compound 46 in at least a first subset of the plurality of training compounds, at least (i) a corresponding positive score for the corresponding positive pose 48 of the corresponding training compound 46 with respect to the target polymer 38 as input to the first model 72, against the corresponding first positive interaction score 50 of the corresponding training compound with respect to the target polymer, and (ii) a corresponding negative score for the corresponding negative pose 60 of the corresponding training compound with respect to the target polymer as input to the first model 72, against the corresponding first negative interaction score 62 of the corresponding training compound with respect to the target polymer, thereby adjusting the first plurality of parameters 73, where at least an output of the first model is used, at least in part, to provide the characterization of the interaction between the test compound and the target polymer. In some such embodiments, the training further uses for each corresponding training compound 46 in a second subset of the plurality of training compounds, at least a corresponding positive score for the corresponding positive pose 48 of the corresponding training compound 46 with respect to the target polymer 38 as input to the first model 72, against the corresponding first positive interaction score 50 of the corresponding training compound with respect to the target polymer. In some embodiments, all of the training compounds have both positive and negative poses. In some embodiments, only some of the training compounds in the plurality of training compounds have both positive and negative poses while other training compounds in the plurality of training compounds have positive poses but no negative poses. In some embodiments, only some of the training compounds in the plurality of training compounds have both positive and negative poses while other training compounds in the plurality of training compounds have either (i) one or more positive poses but no negative poses, or (ii) one or more negative poses but no positive poses.

Referring to block 234, in some embodiments the first model 72 is a first fully connected neural network.

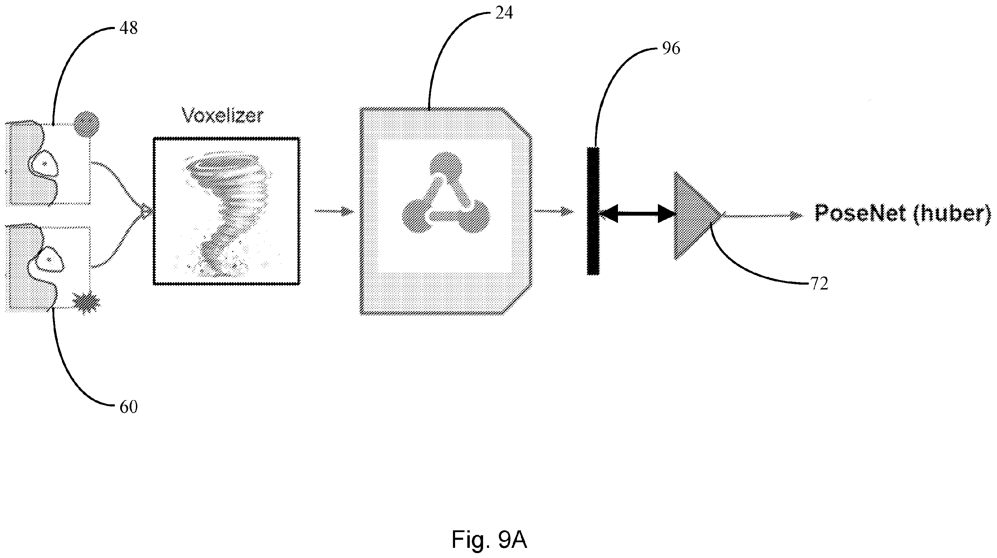

In Figure 9A, the first model 72 provides an estimate of the pose quality of a compound. To train model 72, data in the training set 44 for each corresponding training compound 46 in the plurality of training compounds is used. For each training compound, a corresponding positive score for the corresponding positive pose 48 of the corresponding training compound 46 is obtained with respect to the target polymer 38 as input to the first model 72.

In accordance with the embodiment of Fig. 9A, the corresponding positive score for the corresponding positive pose 48 is the output of neural network 24 upon inputting the positive pose 48 into the neural network 24, as discussed in more detail in block 228 below. As illustrated in Figure 9A, in typical embodiments, the positive score is in the form of an embedding from embedding layer 96, which serves at least the purpose of dimensioning the positive score to the dimensions necessary to serve as input to the first model. The output of the first model 72, upon inputting the corresponding positive score from the neural network 24, is compared against the corresponding first positive interaction score 50 of the corresponding training compound with respect to the target polymer 38. The difference between the output of the first model 72 and the corresponding first positive interaction score 50 is evaluated by a loss function in order to adjust the weights of the first model through 72 back-propagation techniques.

Further, in accordance with the embodiment of Fig. 9A, the corresponding negative score for the corresponding negative pose 60, for those compounds in the training set that have a negative pose, is the output of neural network 24 upon inputting the negative pose 60 into the neural network 24 as discussed in more detail in block 232 below. As illustrated in Figure 9A, in typical embodiments, the negative score is in the form of an embedding from embedding layer 96, which serves at least the purpose of dimensioning the negative score to the dimensions necessary to serve as input to the first model. The output of the first model 72, upon inputting the corresponding negative score from the neural network 24, is compared against the corresponding first negative interaction score 62 of the corresponding training compound with respect to the target polymer 38. The difference between the output of the first model 72 and the corresponding first negative interaction score 62 is also evaluated by the loss function in order to adjust the weights of the first model through back-propagation techniques.

The first model 72 has a first plurality of parameters 73. In some embodiments, the first plurality of parameters comprises more than 100, 200, 300, 400, 500, 600, 700, 800, 900, 1000, 10,000, 50,000, 100,000 or 1 x 10⁶ parameters.

Referring to block 226, in some embodiments, the first model 72 is a fully connected neural network, also known as a multilayer perceptron (MLP). In some embodiments, a MLP is a class of feedforward artificial neural network (ANN) comprising at least three layers of nodes: an input layer, a hidden layer and an output layer. In such embodiments, except for the input nodes, each node is a neuron that uses a nonlinear activation function. More disclosure on suitable MLPs that serve as the first model 72 in some embodiments is found in Vang-mata ed., 2020, Multilayer Perceptrons: Theory and Applications, Nova Science Publishers, Hauppauge, New York.

*Blocks 228-230.* Referring to block 228 of Figure 2B, in some embodiments the corresponding positive score for the corresponding positive pose 48 of the corresponding training compound 46 with respect to the target polymer 38 is obtained by retrieving a corresponding positive voxel map 52 of the corresponding training compound 46 with respect to the target polymer 38 in the corresponding positive pose 48, unfolding the corresponding positive voxel map 52 into a corresponding positive vector 54, and inputting the corresponding positive vector 54 to a neural network 24 in the form of a neural network (e.g., a convolutional neural network, a graph neural network, *etc*.). The graph neural network or the convolutional neural network 24, in turn, provides the corresponding positive score for the corresponding positive pose 48 at output.

In some embodiments, the neural network 24, regardless of whether or not is uses voxel maps, comprises more than 500 parameters, more than 1000 parameters, more than 2000 parameters, more than 5000 parameters, more than 10,000 parameters, more than 100,000 parameters, or more than 1 x 10⁶ parameters.

In some such embodiments, referring to block 230, the corresponding positive vector 54 referenced above is a first one-dimensional vector. In some embodiments, the corresponding positive vector 54 comprises 10 or more elements, 20 or more elements, 100 or more elements, 500 or more elements, 1000 or more elements, or 10,000 or more elements.

In some embodiments, the neural network 24 is any of the convolutional neural networks 24 disclosed in Wallach et al., 2015, "AtomNet: A Deep Convolutional Neural Network for Bioactivity Prediction in Structure-based Drug Discovery," arXiv:1510.02855v1, or United States Patents Nos. 11,080,570; 10,546,237; 10,482,355; 10,002,312; or 9,373059. More details on obtaining a corresponding positive score for the corresponding positive pose 48 of the corresponding training compound 46 with respect to the target polymer 38 using a convolutional neural network is disclosed below in the section entitled "Using a convolutional neural network to obtain scores for poses."

In some embodiments, the neural network 24 is an equivariant neural network. Nonlimiting examples of the equivariant convolutional neural network are disclosed in Thomas et al., 2018, "Tensor field networks: Rotation- and translation-equivariant neural networks for 3D point clouds," arXiv: 1802.08219; Anderson et al., 2019, "Cormorant: Covariant Molecular Neural Networks," Neural Information Processing Systems; Johannes et al., 2020, "Directional Message Passing For Molecular Graphs," International Conference on Learning Representations; Townshend et al., 2021, "ATOM3D: Tasks On Molecules in Three Dimensions," International Conference on Learning Representations; Jing et al., 2009, "Learning from Protein Structure with Geometric Vector Perceptrons," arXiv: 2009.01411; and Satorras et al., 2021, "E(n) Equivariant Graph Neural Networks," arXiv: 2102 09844.

In some embodiments, the neural network 24 is a graph neural network (*e.g.*, graph convolutional neural network). Nonlimiting examples of graph convolutional neural networks are disclosed in Behler Parrinello, 2007, "Generalized Neural-Network Representation of High Dimensional Potential-Energy Surfaces," Physical Review Letters 98, 146401; Chmiela et al., 2017, "Machine learning of accurate energy-conserving molecular force fields," Science Advances 3(5):e1603015; Schütt et al., 2017, "SchNet: A continuousfilter convolutional neural network for modeling quantum interactions," Advances in Neural Information Processing Systems 30, pp. 992-1002; Feinberg et al., 2018, "PotentialNet for Molecular Property Prediction," ACS Cent. Sci. 4, 11, 1520-1530; and Stafford et al., "AtomNet PoseRanker: Enriching Ligand Pose Quality for Dynamic Proteins in Virtual High Throughput Screens," https://chemrxiv.org/engage/chemrxiv/article-details/614b905e39ef6a1c36268003.

In some embodiments the neural network 24 is any of the graph neural networks disclosed in United States Provisional Patent Application No. 63/336,841, entitled "Characterization of Interactions Between Compounds and Polymers Using Pose Ensembles," filed May 10, 2022.

*Blocks 232-234.* Referring to block 232 of Figure 2D, in some embodiments the corresponding negative score for the corresponding negative pose 60 of the corresponding training compound 46 with respect to the target polymer 38 is obtained in the same manner that the corresponding positive score for the corresponding positive pose 48 of the corresponding training compound 46 with respect to the target polymer 38 was obtained. For instance, in some embodiments, it is obtained by retrieving a corresponding negative voxel map of the corresponding training compound with respect to the target polymer in the corresponding negative pose 60, unfolding the corresponding negative voxel map into a corresponding negative vector 66, and inputting the corresponding negative vector to the neural network 24 thereby obtaining the corresponding negative score for the corresponding negative pose 60 of the corresponding training compound 46 with respect to the target polymer 38. In some such embodiments, referring to block 234 of Figure 2D, the corresponding negative vector 66 is a second one-dimensional vector.

*Blocks 236-244.* Referring to block 236 of Figure 2D, in some embodiments, the training of model 72 is a regression task in which the first plurality of parameters 73 of the first model 72 is adjusted by back-propagation through an associated loss function, the corresponding first positive interaction score 50 is related to the corresponding first negative interaction score 62 by the expression *B = N* × *A,* where *A* is the corresponding positive interaction score, B is the corresponding negative interaction score, and N is a real number that is greater than zero and less than 1. The training of model 72 as a regression task is suitable in instances where the first positive interaction score is a measured property of the respective training compound from a wet lab (*e.g.* an *in vivo* or *in vitro*) assay. Examples of such measured properties of the respective training compound include, but are not limited to IC₅₀, EC₅₀, Kd, KI, or pKI for the respective training compound with respect to the target polymer. In such embodiments, it is reasonable to assign the first positive interaction score 50 the measured property of the training compound. The question then becomes, for training purposes, what to assign the first negative interaction score 62 for the training compound, given the measured property of the training compound. In accordance with block 236 of Figure 2D, in some embodiments, the negative interaction score 62 is assigned a fixed discounted value *N* of the measured property. By fixed, what is meant is the same value *N* is applied to each first positive interaction score 50 for each respective training compound to calculate the value for the corresponding first negative interaction score 62. Thus, if the value for *N* is 0.90, for each respective training compound, the corresponding first negative interaction score 62 has a value that is 0.90 of the corresponding first positive interaction score 50. In some embodiments, *N* is a value between 0.10 and 0.99. In some embodiments, Nis a value between 0.20 and 0.95. In some embodiments, Nis a value between 0.30 and 0.90. In some embodiments, *N* is a value between 0.25 and 0.85. In some embodiments, *N* is a value between 0.60 and 0.95. In some alternative embodiments, the negative interaction score 62 is assigned a logarithm of the measured property. Thus, in such embodiments, for each respective training compound, the corresponding first negative interaction score is a logarithm of the corresponding first positive interaction score 50. The logarithm can be in any base, such as the natural logarithm, base 10, *etc.*

Referring to block 238, in some embodiments, the associated loss function described above with respect to block 232 is any suitable regression task loss function. Examples of such loss functions include, but are not limited to, a mean squared error loss function, a mean absolute error loss function, a Huber loss function, a Log-Cosh loss function, or a quantile loss function. See Wang et al., 2020, "A Comprehensive Survey of Loss Functions in Machine Learning," Annals of Data Science, https://doi.org/10.1007/s40745-020-00253-5, last accessed September 15, 2021.

Referring to block 240 of Figure 2D, in some specific embodiments, the corresponding first positive interaction score 50 and the corresponding first negative interaction score 62 each represent a binding coefficient, and the corresponding first positive interaction score is an *in vitro* or *in vivo* measurement of the binding coefficient of the corresponding training compound 46 to the target polymer 38.

Referring to block 244 of Figure 2E, in some embodiments, the first positive interaction score is an IC₅₀, EC₅₀, Kd, KI, or pKI for the respective training compound with respect to the target polymer. Measured binding coefficients are generally described in Huser ed., 2006, High-Throughput-Screening in Drug Discovery, Methods and Principles in Medicinal Chemistry 35; and Chen ed., 2019, A Practical Guide to Assay Development and High-Throughput Screening in Drug Discovery*.*

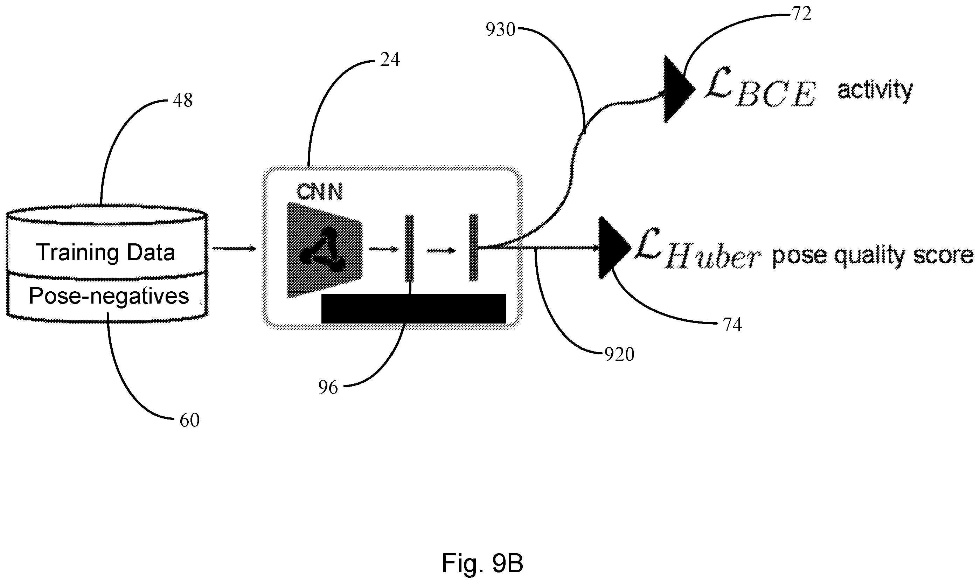

*Blocks 246-248.* Referring to block 246 of Figure 2E, in some embodiments, each respective electronic description 46 in at least a subset of the electronic descriptions 46 in the training dataset 44 further comprises a corresponding positive activity score 56 for the corresponding positive pose 48 of the corresponding training compound 46 and a corresponding negative activity score 58 for the corresponding negative pose 60 of the corresponding training compound. In some embodiments, at least some of the training compounds do not have a negative activity score 58. With reference to Figure 9B, in some embodiments, the training at least the first model 72 further comprises jointly training a second model 74 with the first model.

Like the first model 72, the second model 74 has a plurality of parameters 75 (second plurality of parameters). In some embodiments, the second plurality of parameters comprises more than 100, 200, 300, 400, 500, 600, 700, 800, 900, 1000, 10,000, 50,000, 100,000 or 1 x 10⁶ parameters.

In the embodiment of Figure 9B, the second model 74 provides an estimate of the pose quality of a compound. To train the second model 74, data in the training set 44 for each corresponding training compound 46 in the plurality of training compounds is used. For each training compound, a corresponding positive score for the corresponding positive pose 48 of the corresponding training compound 46 is obtained with respect to the target polymer 38 as input to the second model 74.

In accordance with the embodiment of Fig. 9B, the corresponding positive score for the corresponding positive pose 48 is the output of neural network 24 upon inputting the positive pose 48 into the neural network 24. As illustrated in Figure 9B, in typical embodiments, the positive score is in the form of an embedding from embedding layer 96, which serves at least the purpose of dimensioning the positive score to the dimensions necessary to serve as input to the second model. The output of the second model 74, upon inputting the corresponding positive score from the neural network 24 into the second model 74 as indicated by edge 920, is compared against the corresponding first positive interaction score 50 of the corresponding training compound with respect to the target polymer 38. The difference between the output of the second model 74 and the corresponding first positive interaction score 50 is evaluated by a loss function in order to adjust the weights of the second model through 74 back-propagation techniques.

Further, in accordance with the embodiment of Fig. 9B, the corresponding negative score for the corresponding negative pose 60, for those compounds in the training set that have a negative pose, is the output of neural network 24 upon inputting the negative pose 60 into the neural network 24. As illustrated in Figure 9B, in typical embodiments, the negative score is in the form of an embedding from embedding layer 96, which serves at least the purpose of dimensioning the negative score to the dimensions necessary to serve as input to the second model. The output of the second model 74, upon inputting the corresponding negative score from the neural network 24 into the second model 74 as indicated by edge 920, is compared against the corresponding first negative interaction score 62 of the corresponding training compound with respect to the target polymer 38. The difference between the output of the second model 74 and the corresponding first negative interaction score 62 is also evaluated by the loss function in order to adjust the plurality of parameters 75 of the second model through back-propagation techniques.

Further in the embodiment illustrated in Figure 9B, for each corresponding training compound in the plurality of training compounds, the training of block 224 further uses at least: (iii) the corresponding positive score for the corresponding positive pose 48 of the corresponding training compound 46 with respect to the target polymer 38 as input to the first model 72 (indicated by edge 930 in Figure 9B), against the corresponding positive activity score 56 of the corresponding training compound and (iv) the corresponding negative score for the corresponding negative pose 60 of the corresponding training compound 46 with respect to the target polymer 38 (again indicated by edge 930 in Figure 9B) as input to the first model 72, against the corresponding negative activity score 68 of the corresponding training compound 46, for at least a subset of the training compounds. In this way, the first plurality of parameters 73 of the first model are adjusted during the training.

Thus, in the embodiments of Figure 9B, the second model 74 is trained against the respective first positive and first negative interaction scores 50/62 while the first model 72 is trained against the positive and negative activity scores 56/68. In some such embodiments the first positive and first negative interaction scores 50/62 are docking scores and the positive and negative activity scores are binary-discrete activity values. For instance, one of the two possible values for a binary-discrete activity value would indicate that the corresponding training inhibits an activity of the target polymer while the other of the two possible values for the binary-discrete activity value would indicate that the corresponding training does not inhibit that activity of the target polymer.

As illustrated in Figure 9B, once trained, the pose of a test compound is introduced into the neural network 24 to yield a score for the pose of the test compound against the target polymer. This score of the pose of the test compound with respect to the target polymer is inputted into both the second model 74 (to provide a characterization of the interaction between the test compound and the target polymer in the form of a pose quality score) as well as the first model 72 (to provide a characterization of the interaction between the test compound and the target polymer in the form of an activity of the interaction between the test compound and the target polymer 38). Thus, in the embodiment of Figure 9B, the characterization of the interaction between the test compound and the target polymer is both an activity score (*e.g.,* a discrete-binary score or a scalar score) and a pose quality score.

Referring to block 248, in some such embodiments, the first model 72 and the second model 74 are each fully connected neural networks, also known as multilayer perceptrons (MLP). In some embodiments, a MLP is a class of feedforward artificial neural network (ANN) comprising at least three layers of nodes: an input layer, a hidden layer and an output layer. In such embodiments, except for the input nodes, each node is a neuron that uses a nonlinear activation function. More disclosure on suitable MLPs that serve as the first model 72 in some embodiments is found in Vang-mata ed., 2020, Multilayer Perceptrons: Theory and Applications, Nova Science Publishers, Hauppauge, New York.

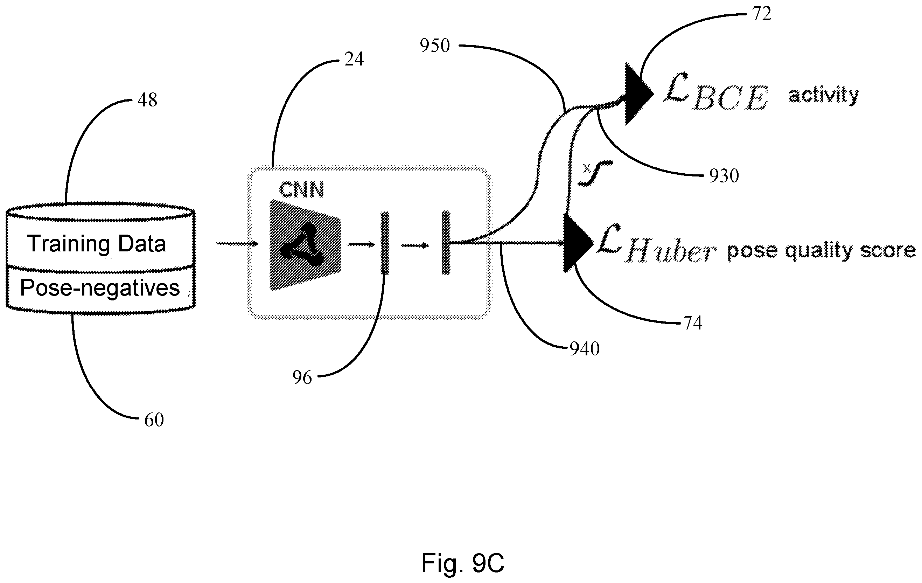

*Blocks 252-256.* Referring to block 252 of Figure 2F, and as illustrated in Figure 9C, in some embodiments, each respective electronic description 46 in at least a subset of the training dataset 44 further comprises a corresponding positive activity score 56 for the corresponding positive pose 48 of the corresponding training compound 46 and a corresponding negative activity score 58 for the corresponding negative pose 60 of the corresponding training compound. In such embodiments, the training described above in block 224 (the training at least the first model 72) further comprises jointly training a second model 74 with the first model 72. The second model 74 has a second plurality of parameters 75.

In the embodiment of Figure 9C, the second model 74 provides an estimate of the pose quality of a compound. To train the second model 74, data in the training set 44 for each corresponding training compound 46 in the plurality of training compounds is used. For each training compound, a corresponding positive score for the corresponding positive pose 48 of the corresponding training compound 46 is obtained with respect to the target polymer 38 as input to the second model 74.

In accordance with the embodiment of Fig. 9C, the corresponding positive score for the corresponding positive pose 48 is the output of neural network 24 upon inputting the positive pose 48 into the neural network 24. As illustrated in Figure 9C, in typical embodiments, the positive score is in the form of an embedding from embedding layer 96, which serves at least the purpose of dimensioning the positive score to the dimensions necessary to serve as input to the first model and the second model. The output of the second model 74, upon inputting the corresponding positive score from the neural network 24 into the second model 74 as indicated by edge 940, is compared against the corresponding first positive interaction score 50 of the corresponding training compound with respect to the target polymer 38. The difference between the output of the second model 74 and the corresponding first positive interaction score 50 is evaluated by a loss function in order to adjust the weights of the second model through 74 back-propagation techniques.

Further, in accordance with the embodiment of Fig. 9C, the corresponding negative score for the corresponding negative pose 60, for those compounds in the training set that have a negative pose, is the output of neural network 24 upon inputting the negative pose 60 into the neural network 24. As illustrated in Figure 9C, in typical embodiments, the negative score is in the form of an embedding from embedding layer 96, which serves at least the purpose of dimensioning the negative score to the dimensions necessary to serve as input to both the first model and the second model. The output of the second model 74, upon inputting the corresponding negative score from the neural network 24 into the second model 74 as indicated by edge 940, is compared against the corresponding first negative interaction score 62 of the corresponding training compound with respect to the target polymer 38. The difference between the output of the second model 74 and the corresponding first negative interaction score 62 is also evaluated by the loss function in order to adjust the plurality of parameters 75 of the second model through back-propagation techniques.

The training in accordance with Figure 9C further uses, for each corresponding training compound 46 in at least a subset of the plurality of training compounds, at least the corresponding positive score for the corresponding positive pose 48 of the corresponding training compound with respect to the target polymer 38 provided by both the model 24 (through edge 950) and the second model 74 (through edge 930) as joint input to the first model 72, against the corresponding positive activity score 56 of the corresponding training compound, and the corresponding negative score for the corresponding negative pose 60 of the corresponding training compound 46 with respect to the target polymer 38 provided by both the model 24 (again through edge 950) and the second model 74 (again through edge 930), against the corresponding negative activity score 68 of the corresponding training compound. In this way the first plurality of parameters 73 of the first model 72 are adjusted (*e.g.,* through back-propagation methods using a loss function).

The second model 74 is used with the output of the first model 72, at least in part, to provide the characterization of the interaction between the test compound and the target polymer. For instance, as illustrated in Figure 9C, once trained, the pose of a test compound is introduced into the neural network 24 to yield a score for the pose of the test compound against the target polymer 38. This score with respect to the target polymer is inputted into both the first model 72 (through edge 950) as well as the second model 74 (through edge 940). Further, the output of the second model 74 (which is a calculation of the interaction score, such as pose quality score, pKA, *etc*.) of the test compound is inputted into the first model 72 through edge 930. Thus, the first model 72 receives both the output of the second model and the output of model 24 in response to input of the pose of the test compound into model 24. The first model 72 uses both of these inputs to determine the characterization of the interaction between the test compound and the target polymer. In some embodiments this characterization is an activity score of the test compound. In some embodiments, this activity score is a discrete-binary score, for instance where a "1" indicates the test compound is active against the target polymer and a "0" indicates that the test compound is inactive against the target polymer. In some embodiments, the activity score provided by the first model 72 is scalar. The conditioning of the discrete-binary activity score of the first model 72 on both the output of model 24 and the second model 74 serves to improve the performance of the first model at characterizing test compounds.

Referring to block 254 of Figure 2F, in some such embodiments the corresponding positive activity score 56 is a first binary activity score and the corresponding negative activity score 68 is a second binary activity score. In some embodiments, the corresponding first binary activity score is assigned a value of 1 based on a measured activity of the corresponding compound against the target polymer based on satisfying an activity criterion, and the corresponding second binary activity score is assigned a value of 0 based on not satisfying an activity criterion. In some embodiments, these activity values for the training compounds are obtained by *in vivo* or *in vitro* assays. Such assays are generally described in in Huser ed., 2006, High-Throughput-Screening in Drug Discovery, Methods and Principles in Medicinal Chemistry 35; and Chen ed., 2019, A Practical Guide to Assay Development and High-Throughput Screening in Drug Discovery*.*

Referring to block 256 of Figure 2F, in some embodiments the training of the second model 74 is a regression task in which the second plurality of parameters 75 is adjusted by back-propagation through a second associated loss function. Non-limiting examples of loss functions suitable for the regression task include, but are not limited to, a mean squared error loss function, a mean absolute error loss function, a Huber loss function, a Log-Cosh loss function, or a quantile loss function. *See,* Wang et al., 2020, "A Comprehensive Survey of Loss Functions in Machine Learning," Annals of Data Science, https://doi.org/10.1007/s40745-020-00253-5, last accessed September 15, 2021. Further, in some embodiments, the training of the first model 72 is a classification task in which the first plurality of parameters 73 is adjusted by back-propagation through a first associated loss function. Non-limiting examples of loss functions suitable for the classification task include, but are not limited to, a binary cross entropy loss function, a hinge loss function, or a squared hinged loss function.

In some embodiments the output of the first model is a discrete value that is other than binary. For instance a first output value of the second model (in response to inputting a pose into classifier 24 of the configuration illustrated in Figure 9C) indicates poor activity of the test compound against the target polymer, a second output value indicates intermediate activity of the test compound against the target polymer, and a third output value indicates good activity for the test compound against the target polymer. In some such embodiments, the loss function used to train the first classifier can be a multiclass classification loss function such as a multi-class cross-entropy loss function, a sparse multiclass cross-entropy loss function, or a Kullback Leibler Divergence loss function.

*Block 260.* Referring to block 260 of Figure 2G, in some embodiments the corresponding first positive interaction score 50 and the corresponding first negative interaction score 62 each represent a binding coefficient or an *in silico* pose quality score of the corresponding training compound to the target polymer, and the corresponding positive activity score 56 is a first binary activity score and the corresponding negative activity score 68 is a second binary activity score.

*Block 262.* Referring to block 262 of Figure 2G, in some embodiments the first associated loss function is a mean squared error loss function, a mean absolute error loss function, a Huber loss function, a Log-Cosh loss function, or a quantile loss function, and the second associated loss function is a binary cross entropy loss function, a hinge loss function, or a squared hinged loss function.

*Block 264.* Referring to block 264 of Figure 2G, in some embodiments the second model 74 is a second fully connected neural network, also known as a multilayer perceptron (MLP). In some embodiments, a MLP is a class of feedforward artificial neural network (ANN) comprising at least three layers of nodes: an input layer, a hidden layer and an output layer. In such embodiments, except for the input nodes, each node is a neuron that uses a nonlinear activation function. More disclosure on suitable MLPs that serve as the first model 72 in some embodiments is found in Vang-mata ed., 2020, Multilayer Perceptrons: Theory and Applications, Nova Science Publishers, Hauppauge, New York.

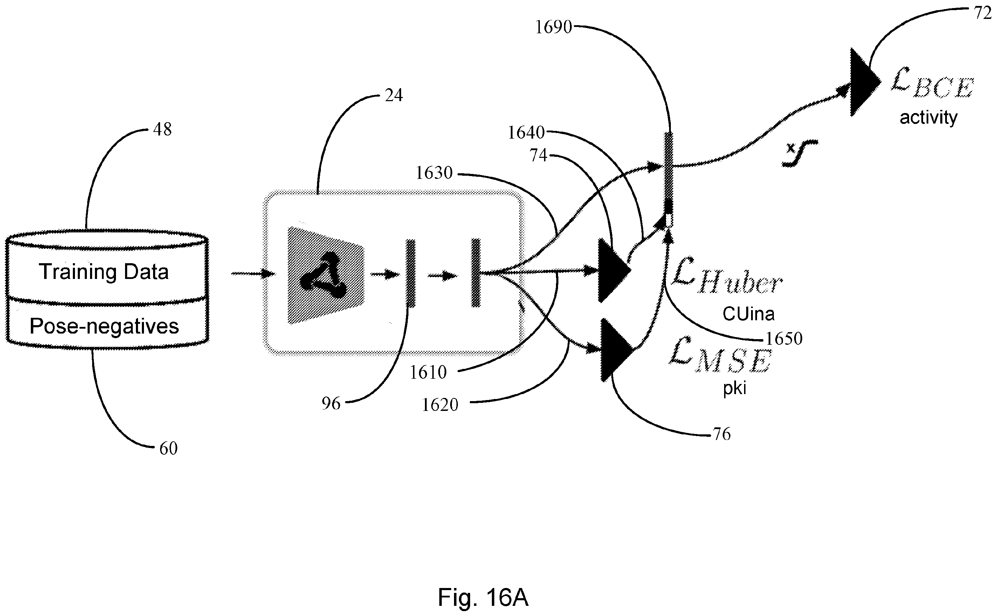

*Blocks 268-276.* Referring to block 268 of Figure 2H, and as illustrated in Figure 16A, in some embodiments each respective electronic description in the training dataset further comprises a corresponding second positive interaction score for the corresponding positive pose 48 of the corresponding training compound 46 and a corresponding second negative interaction score for the corresponding negative pose 60 of the corresponding training compound. Further, each respective electronic description in the training dataset also comprises a corresponding positive activity score 56 for the corresponding positive pose 48 of the corresponding training compound 46 and a corresponding negative activity score 68 for the corresponding negative pose 60 of the corresponding training compound.

In such embodiments, the training at least the first model 72, second model 74, and third model 76 are jointly trained.

The second model 74 has a second plurality of parameters 75. In some embodiments, the second plurality of parameters comprises more than 100, 200, 300, 400, 500, 600, 700, 800, 900, 1000, 10,000, 50,000, 100,000 or 1 x 10⁶ parameters.

The third model 76 has a third plurality of parameters 77. In some embodiments, the third plurality of parameters comprises more than 100, 200, 300, 400, 500, 600, 700, 800, 900, 1000, 10,000, 50,000, 100,000 or 1 x 10⁶ parameters.

The model co-training uses, for each corresponding training compound 46 in at least a subset of the plurality of training compounds, at least: (i) the corresponding positive score for the corresponding positive pose 48 of the corresponding training compound with respect to the target polymer 38 provided by the model 24 (through edge 1610) as input to the second model 74, against the corresponding first positive interaction score 50 of the corresponding training compound with respect to the target polymer 38, and (ii) the corresponding negative score for the corresponding negative pose 60 of the corresponding training compound 46 with respect to the target polymer 38 provided by the model 24 (again through edge 1610) as input to the second model 74, against the corresponding first negative interaction score 62 of the corresponding training compound 46 with respect to the target polymer 38, thereby adjusting the second plurality of parameters of the second model.

The model co-training further uses, for each corresponding training compound 46 in at least a subset of the plurality of training compounds, at least: the corresponding positive score for the corresponding positive pose 48 of the corresponding training compound with respect to the target polymer 38 provided by the model 24 (through edge 1620) as input to the third model 76, against the corresponding second positive interaction score 58 of the corresponding training compound with respect to the target polymer 38, and (ii) the corresponding negative score for the corresponding negative pose 60 of the corresponding training compound 46 with respect to the target polymer 38 provided by the model 24 (again through edge 1620) as input to the third model 76, against the corresponding second negative interaction score 70 of the corresponding training compound 46 with respect to the target polymer 38, thereby adjusting the third plurality of parameters 77 of the third model 76.

The model co-training further uses, for each corresponding training compound 46 in at least a subset of the plurality of training compounds, at least: (i) the corresponding positive score for the corresponding positive pose 48 of the corresponding training compound with respect to the target polymer 38 provided by the model 24 (through edge 1630), (ii) an output of the second model 74 through edge 1640 upon input into the second model 74 of the corresponding positive score for the corresponding positive pose 48 of the corresponding training compound with respect to the target polymer 38 provided by the model 24, and (iii) an output of the third model 76 through edge 1650 upon input into the third model 76 of the corresponding positive score for the corresponding positive pose 48 of the corresponding training compound with respect to the target polymer 38 provided by the model 24, as collective input to the first model 72, against the corresponding positive activity score of the corresponding training compound with respect to the target polymer 38, and at least: (i) the corresponding negative score for the corresponding negative pose of the corresponding training compound with respect to the target polymer 38 provided by the model 24 (through edge 1630), (ii) an output of the second model 74 through edge 1640 upon input into the second model 74 of the corresponding negative score for the corresponding negative pose of the corresponding training compound with respect to the target polymer 38 provided by the model 24, and (iii) an output of the third model 76 through edge 1650 upon input into the third model 76 of the corresponding negative score for the corresponding negative pose of the corresponding training compound with respect to the target polymer 38 provided by the model 24, as collective input to the first model 72, against the corresponding negative activity score of the corresponding training compound with respect to the target polymer 38,, thereby adjusting the first plurality of parameters of the first model.

The first model 74 is used to provide the characterization of the interaction between the test compound and the target polymer. For instance, as illustrated in Figure 16A, once trained, the pose of a test compound is introduced into the neural network 24 to yield a score for the pose of the test compound against the target polymer 38. This score with respect to the target polymer is inputted into the first model 72 (through edge 1630), the second model 74 (through edge 1610), and the third model (through edge 1620). Further, the output of the second model 74 (which is a calculation of the interaction score, such as pose quality score, *etc.)* of the test compound is inputted into the first model 72 through edge 1640. Further still, the output of the third model 76 (which is a calculation of the interaction score, such as pKA, *etc.)* of the test compound is inputted into the first model 72 through edge 1650. Thus, the third model receives the output of the first model, the second model, and model 24 in response to input of the pose of the test compound into model 24. The first model 72 uses each of these inputs collectively to determine the characterization of the interaction between the test compound and the target polymer. In some embodiments this characterization is an activity score of the test compound. In some embodiments, this activity score is a discrete-binary score, for instance where a "1" indicates the test compound is active against the target polymer and a "0" indicates that the test compound is inactive against the target polymer. In some embodiments, the activity score provided by the third model 74 is scalar. The conditioning of the discrete-binary activity score of the first model 72 on the output of model 24, the second model 74, and the third model 76 serves to improve the performance of the first model at characterizing test compounds by forcing this first model to consider binding mode when computing activity, thus addressing the Picasso problem that arises in machine learning. As such, the output of the first model provides the characterization of the interaction between the test compound and the target polymer.

In some embodiments, with reference to Figure 16A, the embedding 96 produced by the neural network 24 is used to predict three outputs: the activity (through the first model 72), a CUina pose quality score (through the second model 74), and a pKi score (through the third model 76). This is performed in two stages in the embodiment illustrated in Figure 16A. First, the CUina and pKi score predictions are computed by passing the score for the pose of the test compound against the target polymer 38 (as embedding 96) from the neural network 24 through the second model 74 and the third models 76. Second, a conditioned embedding 1690 is formed by concatenating (i) the input embedding 96 (score for the pose of the test compound against the target polymer 38 from the neural network score), (ii) the resulting second model 74 score prediction from the first stage, and (iii) the third model 76 score prediction from the first stage. This embedding 1690 is then passed to the first model 72, which is the form of a multilayer perceptron, to compute the activity prediction for the test compound. In some embodiments, the embedding 1690, rather than simply concatenating (i) the input embedding 96 (score for the pose of the test compound against the target polymer 38 from the neural network score), (ii) the resulting second model 74 score prediction from the first stage, and (iii) the third model 76 score prediction from the first stage, these three sources are multiplied against each other and the product of the multiplication is inputted into the third model as embedding 1690. In some embodiments, the embedding 1690 of Figure 16A, rather than simply concatenating (i) the input embedding 96 (score for the pose of the test compound against the target polymer 38 from the neural network score), (ii) the resulting second model 74 score prediction from the first stage, and (iii) the third model 76 score prediction from the first stage, multiplies these three sources against each other and the product of the multiplication is inputted into the third model as embedding 1690. In some embodiments, the embedding 1690, rather than concatenating, transforms each of the three sources in embedding 1690 and this transformation serves as input to the first model 72. More generally, embedding 1690 is capable of performing any mathematical function on all or any part of any of the inputs to embedding 1690, including but not limited to multiplication, concatenation, linear or nonlinear transformation in order to form a condition embedding that is passed on to the first model 72.

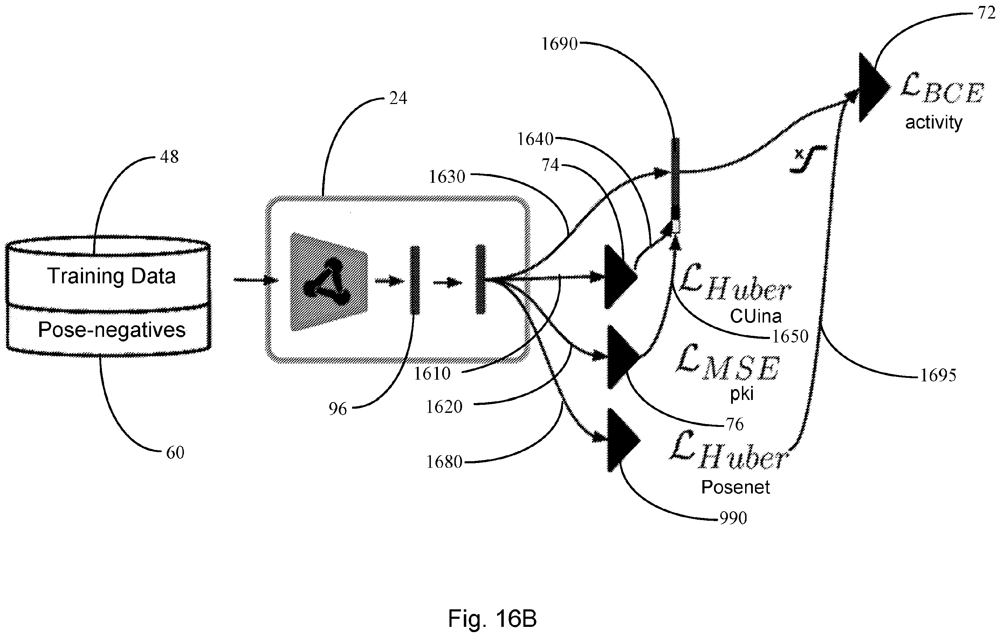

Referring to Figure 16B, it is possible to condition the first model 72 on additional models as well. Thus, in Figure 16B, the first model 72 is conditioned, in addition to the output of network 24, on the output of a second model 74 that has been trained on, for example, CUina scores of the training compounds, a third model 76 that has been trained on, for example, pKi scores of the training compounds, and a fourth model 990 that has been trained on, for example, PoseNet scores of the training compound.

Referring to block 272 of Figure 2I, in some such embodiments the first model, the second model 74, the third model 76, and the fourth model 990 are each a fully connected neural network. Such fully connected neural networks are also known as multilayer perceptrons (MLP). In some embodiments, a MLP is a class of feedforward artificial neural network (ANN) comprising at least three layers of nodes: an input layer, a hidden layer and an output layer. In such embodiments, except for the input nodes, each node is a neuron that uses a nonlinear activation function. More disclosure on suitable MLPs that serve as the first model 72 in some embodiments is found in Vang-mata ed., 2020, Multilayer Perceptrons: Theory and Applications, Nova Science Publishers, Hauppauge, New York.

Referring to block 274 of Figure 2I, in some embodiments the corresponding positive activity score provided by the first model 72 is a first binary activity score and the corresponding negative activity score provided by the first model 72 is a second binary activity score. In some embodiments, the corresponding first binary activity score is assigned a value of "1" based on a measured activity of the corresponding training compound against the target polymer, and the corresponding second binary activity score is assigned a value of "0".

Referring to block 276 of Figure 2I, in some embodiments the training of the second model 74 is a regression task in which the second plurality of parameters associated with the second model is adjusted by back-propagation through a second associated loss function. Further, in some embodiments, the training of the third model 76 is a regression task in which the third plurality of parameters associated with the third model is adjusted by back-propagation through a third associated loss function. Further, in some embodiments, the training of the fourth model 990 is a regression task in which the fourth plurality of parameters associated with the fourth model 990 is adjusted by back-propagation through a fourth associated loss function. Non-limiting examples of loss functions suitable for these regression tasks include, but are not limited to, a mean squared error loss function, a mean absolute error loss function, a Huber loss function, a Log-Cosh loss function, or a quantile loss function. *See,* Wang et al., 2020, "A Comprehensive Survey of Loss Functions in Machine Learning," Annals of Data Science, https.//doi.org/10.1007/s40745-020-00253-5, last accessed September 15, 2021. Further, still, in some embodiments, the training of the first model 72 is a classification task in which the first plurality of parameters associated with the first model 72 is adjusted by back-propagation through a first associated loss function. Non-limiting examples of loss functions suitable for the classification task include, but are not limited to, a binary cross entropy loss function, a hinge loss function, or a squared hinged loss function.

In some such embodiments the corresponding first positive interaction score and the corresponding first negative interaction score each represent an *in silico* pose quality score of the corresponding training compound to the target polymer, the corresponding second positive interaction score and the corresponding second negative interaction score each represent a binding coefficient of the corresponding training compound to the target polymer, and the corresponding positive activity score is a first binary activity score and the corresponding negative activity score is a second binary activity score. In some such embodiments, the second, third, and fourth associated loss functions are each independently a mean squared error loss function, a mean absolute error loss function, a Huber loss function, a Log-Cosh loss function, or a quantile loss function, while the first associated loss function is a binary cross entropy loss function, a hinge loss function, or a squared hinged loss function.

In some embodiments, with reference to Figure 16B, the embedding 96 produced by the neural network 24 is used to predict four outputs: the activity (through the first model 72), a CUina pose quality score (through the second model 74), a pKi score (through the third model 76), and a PoseNet score (through the fourth model 990). This is performed in two stages in the embodiment illustrated in Figure 16B. First, CUina, pKi, and PoseNet score predictions are computed by passing the score for the pose of the test compound against the target polymer 38 (as embedding 96) from the neural network 24 through the second model 74, the third model 76, and the fourth model 990. Second, a conditioned embedding 1690 is formed by concatenating (i) the input embedding 96 (score for the pose of the test compound against the target polymer 38 from the neural network score), (ii) the resulting second model 74 score prediction from the first stage, and (iii) the third model 76 score prediction from the first stage. This embedding 1690, along with the output of the fourth model, is then passed to the first model 72, which is the form of a multilayer perceptron, to compute the activity prediction for the test compound. In some embodiments, the embedding 1690 of Figure 16B, rather than simply concatenating (i) the input embedding 96 (score for the pose of the test compound against the target polymer 38 from the neural network score), (ii) the resulting second model 74 score prediction from the first stage, and (iii) the third model 76 score prediction from the first stage, multiplies these three sources against each other and the product of the multiplication is inputted into the third model as embedding 1690. In some embodiments, the embedding 1690, rather than concatenating, transforms each of the three sources in embedding 1690 and this transformation serves as input to the first model 72. More generally, embedding 1690 is capable of performing any mathematical function on all or any part of any of the inputs to embedding 1690, including but not limited to multiplication, concatenation, linear or nonlinear transformation in order to form a condition embedding that is passed on to the first model 72.

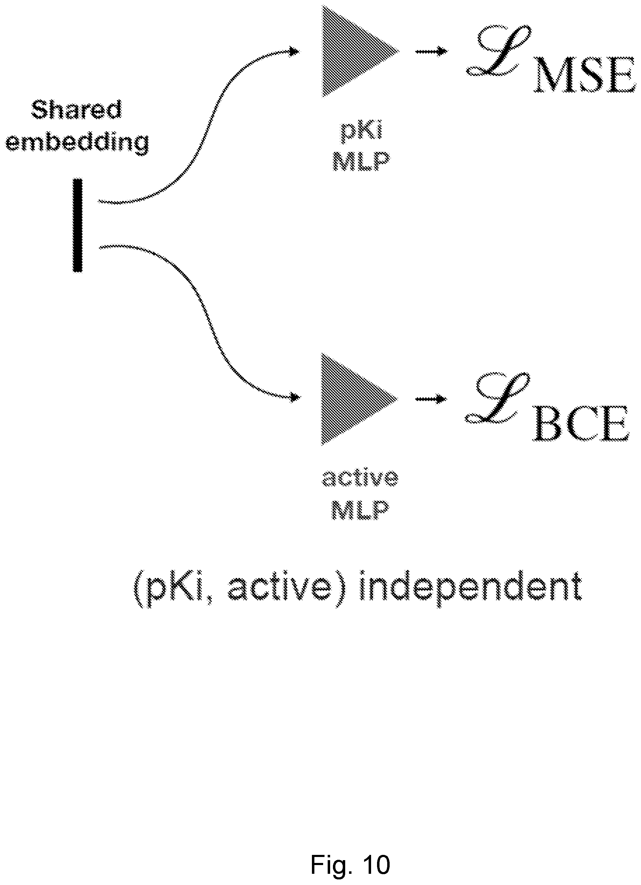

Figure 10 is a system for characterizing an interaction between a test compound and a target polymer, where the characterization is (i) binary-discrete activity and (ii) pKi, and where the system is trained using coupled positive and negative poses for training compounds, in accordance with one embodiment of the present disclosure. Although not shown in Figure 10 the shared embedding layer receives the output from neural network 24 upon input of a voxelated pose of a compound into the neural network 24. In the system of Figure 10, the pKi model and the activity model are independent of each other. In some embodiments, the pKi model is trained as a regression task using a loss function such as mean squared error, whereas the activity model is trained as a classification task using a loss function such as binary cost entropy.

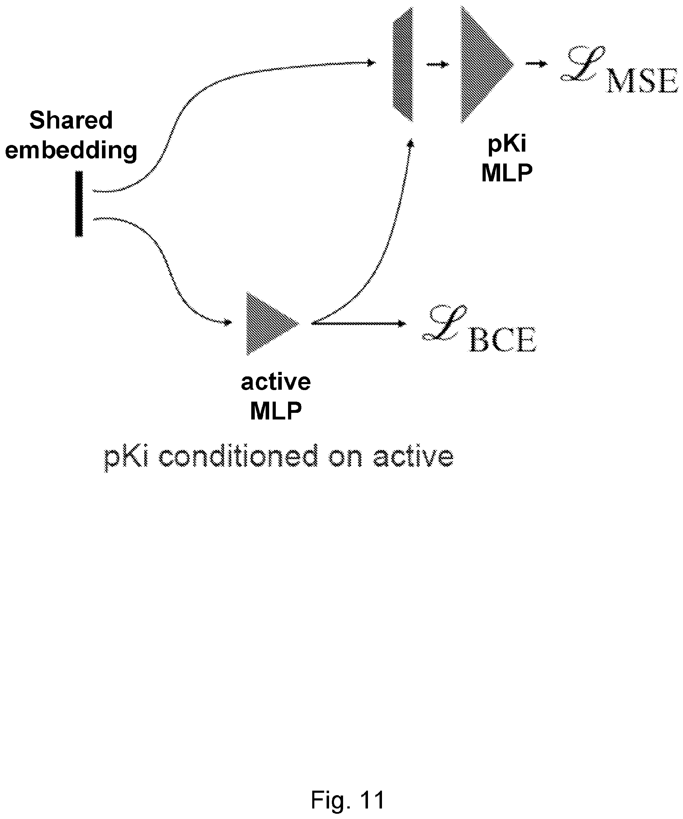

Figure 11 is a system for characterizing an interaction between a test compound and a target polymer, where the characterization is pKi, and where the pKi is conditioned, in part, on activity, and where the system is trained using coupled positive and negative poses for training compounds, in accordance with one embodiment of the present disclosure. Although not shown in Figure 11 the shared embedding layer receives the output from the neural network 24 upon input of a voxelated pose of a compound into the neural network 24. In the system of Figure 11, the pKi model is conditioned on the activity model. In some embodiments, the pKi model is trained as a regression task using a loss function such as mean squared error, whereas the activity model is trained as a classification task using a loss function such as binary cost entropy.

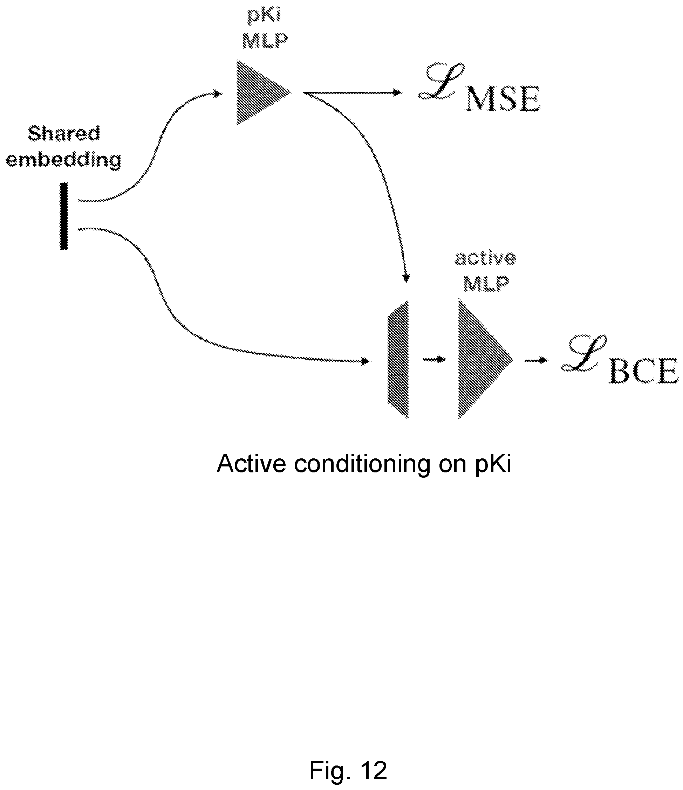

Figure 12 is a system for characterizing an interaction between a test compound and a target polymer, where the characterization is activity, and where the activity is conditioned, in part, on both pKi, and a pose quality score, and where the system is trained using coupled positive and negative poses for training compounds, in accordance with one embodiment of the present disclosure. Although not shown in Figure 12, the shared embedding layer receives the output from the neural network 24 upon input of a voxelated pose of a compound into the neural network 24. In the system of Figure 12, the activity model is conditioned on the pKi model. In some embodiments, the pKi model is trained as a regression task using a loss function such as mean squared error, whereas the activity model is trained as a classification task using a loss function such as binary cost entropy.

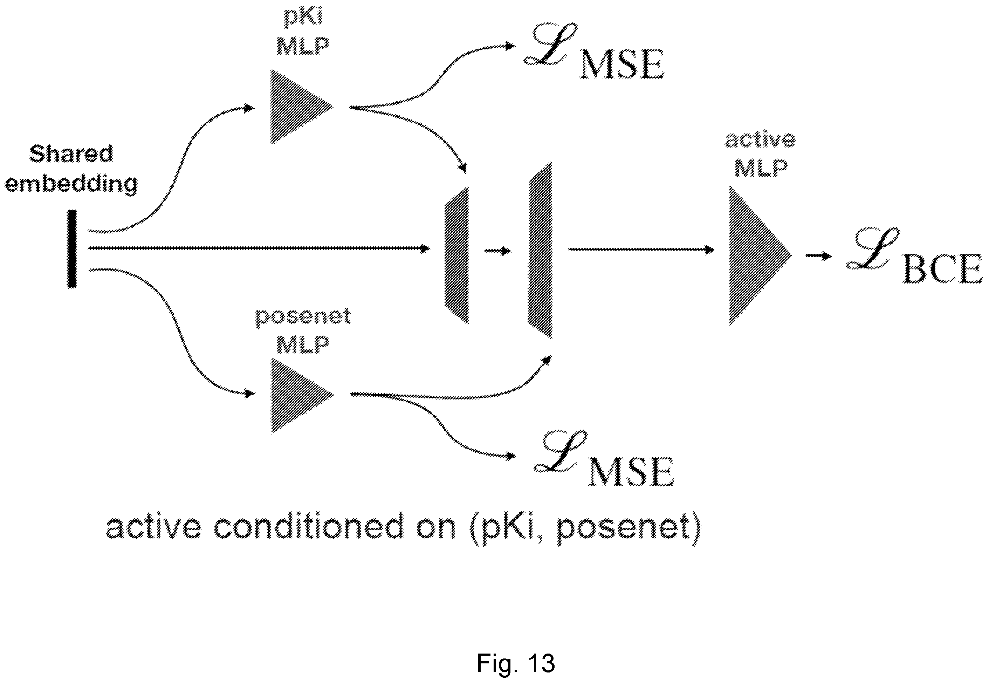

Figure 13 is a system for characterizing an interaction between a test compound and a target polymer, where the characterization is activity, and where the activity is conditioned, in part, on both pKi and binding mode score, and where the system is trained using coupled positive and negative poses for training compounds, in accordance with one embodiment of the present disclosure. Although not shown in Figure 13, the shared embedding layer receives the output from the neural network 24 upon input of a voxelated pose of a compound into the neural network 24. In the system of Figure 13, the activity model is conditioned on both a pKi model and a posenet model. In some embodiments, the pKi model and the posenet model is trained as a regression task using a loss function such as mean squared error, whereas the activity model is trained as a classification task using a loss function such as binary cost entropy.

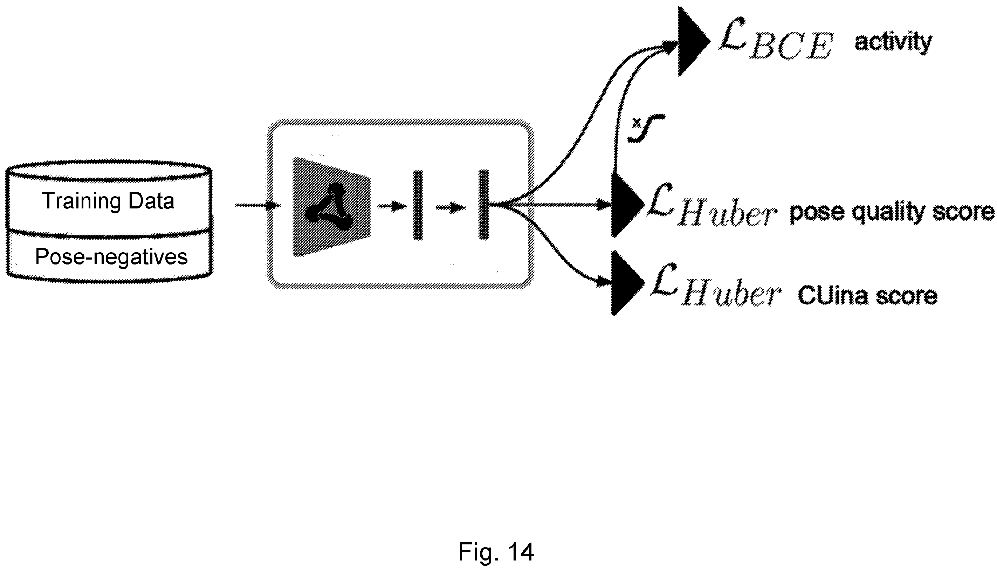

FIG. 14 is a system for characterizing an interaction between a test compound and a target polymer, where the characterization is activity and two different compound binding mode scores, and where the system is trained using coupled positive and negative poses for training compounds, in accordance with one embodiment of the present disclosure. In the system of Figure 14, the activity model is conditioned on a pose quality score model. In some embodiments, the pose quality model is trained as a regression task using a loss function such as mean squared error, whereas the activity model is trained as a classification task using a loss function such as binary cost entropy.

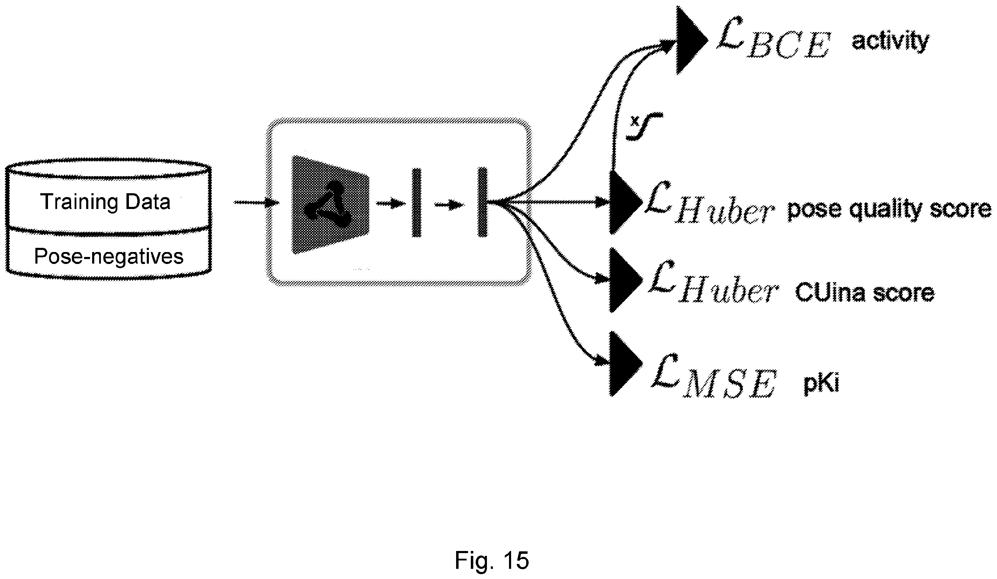

FIG. 15 is a system for characterizing an interaction between a test compound and a target polymer, where the characterization is activity, two different compound binding mode scores and pKi, and where the system is trained using coupled positive and negative poses for training compounds, in accordance with one embodiment of the present disclosure. In the system of Figure 15, the activity model is conditioned on a pose quality score model. In some embodiments, the pose quality model is trained as a regression task using a loss function such as mean squared error, whereas the activity model is trained as a classification task using a loss function such as binary cost entropy.

***Representative test compounds and training compounds.*** The significant difference between test compounds and training compounds is that the training compounds are labeled (*e.g.*, with complementary binding data obtained from wet lab binding assays, *etc*.) and such labeling is used to train the neural network 24 and other models of the present disclosure, whereas the test compounds are not labeled and the neural network 24 and other models of the present disclosure is used to classify test compounds. In other words, the training compounds are already classified by labels, and such classification is used to train the neural network 24 and other models of the present disclosure so that the models of the present disclosure may then classify the test compounds. The test compounds are typically not classified prior to application of the neural network 24 and other models of the present disclosure. In typical embodiments, the classifications associated with the training compounds is binding data against the target polymer 38 obtained by wet lab binding assays.

***Training the predictive model.*** In some embodiments, where a deep neural network is implemented (*e.g*., the neural network 24), the network 24 is trained to receive the geometric data input and to output a prediction (probability) of whether or not a given test compound binds to a target polymer. For instance, in some embodiments, the training compounds, which have known binding data against the target polymer (because of their associated binding data) are sequentially run through the neural network 24 and models of the present disclosure using the techniques discussed above in relation to Figure 2 and the neural network 24 provides a single value for each respective training compound.

In some such embodiments, the systems of the present disclosure output one of two possible activity classes for each training object against a given target compound. For instance, the single value provided for each respective training compound by the systems of the present disclosure is in a first activity class (*e.g.,* binders) when it is below a predetermined threshold value and is in a second activity class (*e.g.,* nonbinders) when the number is above the predetermined threshold value. The activity classes assigned by the systems of the present disclosure are compared to the actual activity classes as represented by the training compound binding data. In typical non-limiting embodiments, such training compound binding data is from independent web lab binding assays. Errors in activity class assignments made by the systems of the present disclosure, as verified against the binding data, are then back-propagated through the weights of the each of the models of the systems of the present disclosure (e.g., 24, 72, 74, *etc*.) in order to train the system. For instance, the filter weights of respective filters in the optional convolutional layers 28 of the network are adjusted in such back-propagation. In an exemplary embodiment, the neural network 24 is trained against the errors in the activity class assignments made by the system, in view of the binding data, by stochastic gradient descent with the AdaDelta adaptive learning method (Zeiler, 2012 "ADADELTA: an adaptive learning rate method,"' CoRR, vol. abs/1212.5 701), and the back propagation algorithm provided in Rumelhart et al., 1988, "Neurocomputing: Foundations of research," ch. Learning Representations by Back-propagating Errors, pp. 696-699, Cambridge, MA, USA: MIT Press. In some such embodiments, the two possible activity classes are respectively a binding constant greater than a given threshold amount (*e.g.,* an IC₅₀, EC₅₀, or KI for the training compound with respect to the target polymer that is greater than one nanomolar, ten nanomolar, one hundred nanomolar, one micromolar, ten micromolar, one hundred micromolar, or one millimolar) and a binding constant that is below the given threshold amount (*e.g.,* an IC₅₀, EC₅₀, or KI for the training compound with respect to the target compound that is less than one nanomolar, ten nanomolar, one hundred nanomolar, one micromolar, ten micromolar, one hundred micromolar, or one millimolar).

In some embodiments, the systems of the present disclosure output one of a plurality of possible activity classes (*e.g.,* three or more activity classes, four or more activity classes, five or more activity classes) for each training compound against a given target polymer. For instance, the single value provided for each respective training compound by the systems of the present disclosure is in a first activity class when the number falls into a first range, is in a second activity class when the number falls into a second range, is in a third activity class when the number falls into a third range, and so forth. The activity classes assigned by the systems of the present disclosure are compared to the actual activity classes as represented by the training compound binding data of other forms of training data. Errors in activity class assignments made by the systems of the present disclosure, as verified against the binding data (or other forms of measured or independently calculated data), are then used to train the systems of the present disclosure using the techniques discussed above. In some embodiments, each respective classification in the plurality of classifications is an IC₅₀, EC₅₀, pkA, or KI range for the training compound with respect to the target polymer.

In some embodiments, classification of a plurality of training compounds by the systems of the present disclosure is compared to the training data (*e.g.,* binding data or other independently measured data for the training compounds) using non-parametric techniques. For instance, the systems of the present disclosure are used to rank order the plurality of training compounds with respect to a given property (*e.g.,* binding against a given target polymer) and this rank order is compared to the rank order provided by the training data that is acquired by wet lab binding assays for the plurality of training compounds. This gives rise to the ability to train the systems of the present disclosure on the errors in the calculated rank order using the system error correction techniques discussed above. In some embodiments, the error (differences) between the ranking by the training compounds by the systems of the present disclosure and the ranking of the training compounds as determined by the binding data (or other independently measured data for the training compounds) is computed using a Wilcoxon Mann Whitney function (Wilcoxon signed-rank test) or other non-parametric test and this error is back-propagated through the systems of the present disclosure (*e.g.,* model 72, model 74, model 24, *etc.*) in order to further train the system using the error correction techniques discussed above.

In an embodiment where the deep learning techniques utilizes a neural network 24 as described above, the training of the system, including the network 24, to improve the accuracy of its prediction may involve modifying the weights in the filters in the optional convolutional layers 28 as well as the biases in the network layers. The weights and biases may be further constrained with various forms of regularization such as L1, L2, weight decay, and dropout.

In an embodiment, the neural network 24 or any of the models disclosed herein may optionally, where training data is labeled (*e.g.,* with binding data), have their parameters (*e.g.,* weights) tuned (adjusted to potentially minimize the error between the system's predicted binding affinities and/or categorizations and the training data's reported binding affinities and/or categorizations. Various methods may be used to minimize error function, such as gradient descent methods, which may include, but are not limited to, log-loss, sum of squares error, hinge-loss methods. These methods may include second-order methods or approximations such as momentum, Hessian-free estimation, Nesterov's accelerated gradient, adagrad, etc. Unlabeled generative pretraining and labeled discriminative training may also be combined.

Input geometric data may be grouped into training examples. For example, it is often the case that a single set of molecules, cofactors, and protein has multiple geometric measurements, where each "snapshot" describes alternative conformations and poses that the target polymer and the training compound may adopt. Similarly, in instances where the target polymer is a protein, different tautomers for the protein sidechains, cofactors, and the training compounds may also be sampled. Because these states all contribute to the behavior of the biological system, as per the Boltzmann distribution, a system to predict binding affinity may be configured to consider these states together (for instance by taking the weighted average of these samplings). Optionally, these training examples may be labeled with binding information. If quantitative binding information is available (e.g., binding data), such labels may be the numerical binding affinities. Alternatively, the training examples may be assigned labels from a set of two or more ordered categories (*e.g.,* two categories of binders and nonbinders, or several possibly-overlapping categories describing the ligands as binders of potencies < 1 molar, < 1 millimolar, < 100 micromolar, < 10 micromolar, < 1 micromolar, < 100 nanomolar, < 10 nanomolar, < 1 nanomolar). Training binding data may be derived or received from a variety of sources, such as experimental measurements, computed estimates, expert insight, or presumption (for example, a random pair of molecule and protein are highly unlikely to bind).

***Using a neural network 24 to obtain scores for poses.*** In order to score a pose using a neural network 24, in some embodiments a voxel map is created for the pose (*e.g.,* a positive voxel map 52 for a positive pose / a negative voxel map 64 for a negative pose 60). In some embodiments, a voxel map is created by (i) sampling the training compound, in either a positive pose 48 (or an ensemble thereof) or a negative pose (or an ensemble thereof), and the target polymer 38 on a three-dimensional grid basis thereby forming a corresponding three dimensional uniform space-filling honeycomb comprising a corresponding plurality of space filling (three-dimensional) polyhedral cells and (ii) populating, for each respective three-dimensional polyhedral cell in the corresponding plurality of three-dimensional cells, a voxel (discrete set of regularly-spaced polyhedral cells) in the respective voxel map based upon a property (*e.g.,* chemical property) of the respective three-dimensional polyhedral cell. Thus, for a particular training compound, two voxel maps are created, a positive voxel map 52 and a negative voxel map 65. Examples of space filling honeycombs include cubic honeycombs with parallelepiped cells, hexagonal prismatic honeycombs with hexagonal prism cells, rhombic dodecahedra with rhombic dodecahedron cells, elongated dodecahedra with elongated dodecahedron cells, and truncated octahedra with truncated octahedron cells.

In some embodiments, the space filling honeycomb is a cubic honeycomb with cubic cells and the dimensions of such voxels determine their resolution. For example, a resolution of 1Å may be chosen meaning that each voxel, in such embodiments, represents a corresponding cube of the geometric data with 1Å dimensions (*e.g.,* 1Å x 1Å x 1Å in the respective height, width, and depth of the respective cells). However, in some embodiments, finer grid spacing (*e.g.,* 0.1Å or even 0.01Å) or coarser grid spacing (*e.g.* 4Å) is used, where the spacing yields an integer number of voxels to cover the input geometric data. In some embodiments, the sampling occurs at a resolution that is between 0.1 Å and 10 Å. As an illustration, for a 40Å input cube, with a 1Å resolution, such an arrangement would yield 40 * 40 * 40 = 64,000 input voxels.

In some embodiments, a characteristic of an atom incurred in the sampling (i) is placed in a single voxel in the respective voxel map, and each voxel in the plurality of voxels represents a characteristic of a maximum of one atom. In some embodiments, the characteristic of the atom consists of an enumeration of the atom type. As one example, some embodiments of the disclosed systems and methods are configured to represent the presence of every atom in a given voxel of the voxel map 40 as a different number for that entry, *e.g.,* if a carbon is in a voxel, a value of 6 is assigned to that voxel because the atomic number of carbon is 6. However, such an encoding could imply that atoms with close atomic numbers will behave similarly, which may not be particularly useful depending on the application. Further, element behavior may be more similar within groups (columns on the periodic table), and therefore such an encoding poses additional work for the neural network 24 to decode.

In some embodiments, the characteristic of the atom is encoded in the voxel as a binary categorical variable. In such embodiments, atom types are encoded in what is termed a "one-hot" encoding: every atom type has a separate channel. Thus, in such embodiments, each voxel has a plurality of channels and at least a subset of the plurality of channels represent atom types. For example, one channel within each voxel may represent carbon whereas another channel within each voxel may represent oxygen. When a given atom type is found in the three-dimensional grid element corresponding to a given voxel, the channel for that atom type within the given voxel is assigned a first value of the binary categorical variable, such as "1", and when the atom type is not found in the three-dimensional grid element corresponding to the given voxel, the channel for that atom type is assigned a second value of the binary categorical variable, such as "0" within the given voxel.

While there are over 100 elements, most are not encountered in biology. However, even representing the most common biological elements (*e.g.,* H, C, N, O, F, P, S, Cl, Br, I, Li, Na, Mg, K, Ca, Mn, Fe, Co, Zn) may yield 18 channels per voxel or 10,483 * 18 = 188,694 inputs to the receptor field. As such, in some embodiments, each respective voxel in a voxel map comprises a plurality of channels, and each channel in the plurality of channels represents a different property that may arise in the three-dimensional space filling polyhedral cell corresponding to the respective voxel. The number of possible channels for a given voxel is even higher in those embodiments where additional characteristics of the atoms (for example, partial charge, presence in ligand versus protein target, electronegativity, or SYBYL atom type) are additionally presented as independent channels for each voxel, necessitating more input channels to differentiate between otherwise-equivalent atoms.

In some embodiments, each voxel has five or more input channels. In some embodiments, each voxel has fifteen or more input channels. In some embodiments, each voxel has twenty or more input channels, twenty-five or more input channels, thirty or more input channels, fifty or more input channels, or one hundred or more input channels. In some embodiments, each voxel has five or more input channels selected from the descriptors found in Table 1 below. For example, in some embodiments, each voxel has five or more channels, each encoded as a binary categorical variable where each such channel represents a SYBYL atom type selected from Table 1 below. For instance, in some embodiments, each respective voxel in a voxel map includes a channel for the C.3 (sp3 carbon) atom type meaning that if the grid in space for a given test object - target object (or training object - target object) complex represented by the respective voxel encompasses an sp3 carbon, the channel adopts a first value (*e.g.,* "1") and is a second value (*e.g.* "0") otherwise.

**Table 1 - SYBYL Atom Types**

| **SYBYL ATOM TYPE** | **DESCRIPTION** |
|---|---|
| C.3 | sp3 carbon |
| C.2 | sp2 carbon |
| C.ar | aromatic carbon |
| C.1 | sp carbon |
| N.3 | sp3 nitrogen |
| N.2 | sp2 nitrogen |
| N.1 | sp nitrogen |
| O.3 | sp3 oxygen |
| O.2 | sp2 oxygen |
| S.3 | sp3 sulfur |
| N.ar | aromatic nitrogen |
| P.3 | sp3 phosphorous |
| H | hydrogen |
| Br | bromine |
| Cl | chlorine |
| F | fluorine |
| I | iodine |
| S.2 | sp2 sulfur |
| N.pl3 | pl3 trigonal planar nitrogen |
| LP | lone pair |
| Na | sodium |
| K | potassium |
| Ca | calcium |
| Li | lithium |
| Al aluminum | aluminum |
| Si | silicon |
| N.am | amide nitrogen |
| S.o | sulfoxide sulfur |
| S.o2 | sulfone sulfur |
| N.4 | positively charged nitrogen |
| O.co2 | oxygen in carboxylate or phosphate group |
| C.cat | carbocation, used only in a guadinium group |
| H.spc | hydrogen in SPC water model |
| O.spc | oxygen in SPC water model |
| H.t3p | hydrogen in TIP3P water model |
| O.t3p | oxygen in TIP3P water model |
| ANY | any atom |
| HEV | heavy (non H) atom |
| HET | heteroatom (N, O, S, P) |
| HAL | halogen |
| Mg | magnesium |
| Cr.oh | hydroxy chromium |
| Cr.th | chromium |
| Se | selenium |
| Fe | iron |
| Cu | copper |
| Zn | zinc |
| Sn | tin |
| Mo | molybdenum |
| Mn | manganese |
| Co.oh | hydroxy cobalt |

In some embodiments, each voxel comprises ten or more input channels, fifteen or more input channels, or twenty or more input channels selected from the descriptors found in Table 1 above. In some embodiments, each voxel includes a channel for halogens.

In some embodiments, a first structural protein-ligand interaction fingerprint (SPLIF) score is generated for the positive pose 48 of a respective training compound and a second SPLIF is generated for the negative pose 60 of the training compounds. In such embodiments, these SPLIF scores are used as additional input into the underlying neural network or is individually encoded in the voxel map. For a description of SPLIFs, *see* Da and Kireev, 2014, J. Chem. Inf. Model. 54, pp. 2555-2561, "Structural Protein-Ligand Interaction Fingerprints (SPLIF) for Structure-Based Virtual Screening: Method and Benchmark Study". A SPLIF implicitly encodes all possible interaction types that may occur between interacting fragments of the training compound and the target polymer 38 *(e.g., π-π,* CH-π, *etc.).* In the first step, a training compound - target polymer 38 is inspected for intermolecular contacts. Two atoms are deemed to be in a contact if the distance between them is within a specified threshold (*e.g.,* within 4.5 Å). For each such intermolecular atom pair, the respective training atom and target polymer atoms are expanded to circular fragments, *e.g.,* fragments that include the atoms in question and their successive neighborhoods up to a certain distance. Each type of circular fragment is assigned an identifier. In some embodiments, such identifiers are coded in individual channels in the respective voxels. In some embodiments, the Extended Connectivity Fingerprints up to the first closest neighbor (ECFP2) as defined in the Pipeline Pilot software can be used. *See,* Pipeline Pilot, ver. 8.5, Accelrys Software Inc., 2009. ECFP retains information about all atom/bond types and uses one unique integer identifier to represent one substructure (*e.g.,* circular fragment). The SPLIF fingerprint encodes all the circular fragment identifiers found. In some embodiments, the SPLIF fingerprint is not encoded individual voxels but serves as a separate independent input in the neural network 24 discussed below.

In some embodiments, rather than or in addition to SPLIFs, structural interaction fingerprints (SIFt) are computed for each pose (positive pose 48 and negative pose 60) of a given training compound to a target polymer and independently provided as input into the neural network 24 or are encoded in the voxel map. For a computation of SIFts, see Deng et al., 2003, "Structural Interaction Fingerprint (SIFt): A Novel Method for Analyzing Three-Dimensional Protein-Ligand Binding Interactions," J. Med. Chem. 47 (2), pp. 337-344.

In some embodiments, rather than or in addition to SPLIFs and SIFTs, atom-pairs-based interaction fragments (APIFs) are computed for each pose (positive pose 48 and negative pose 60) of a given training compound to the target polymer 38 and independently provided as input into the neural network 24 or are individually encoded in the voxel map. For a computation of APIFs, see Perez-Nueno et al., 2009, "APIF: a new interaction fingerprint based on atom pairs and its application to virtual screening," J. Chem. Inf. Model. 49(5), pp. 1245-1260.

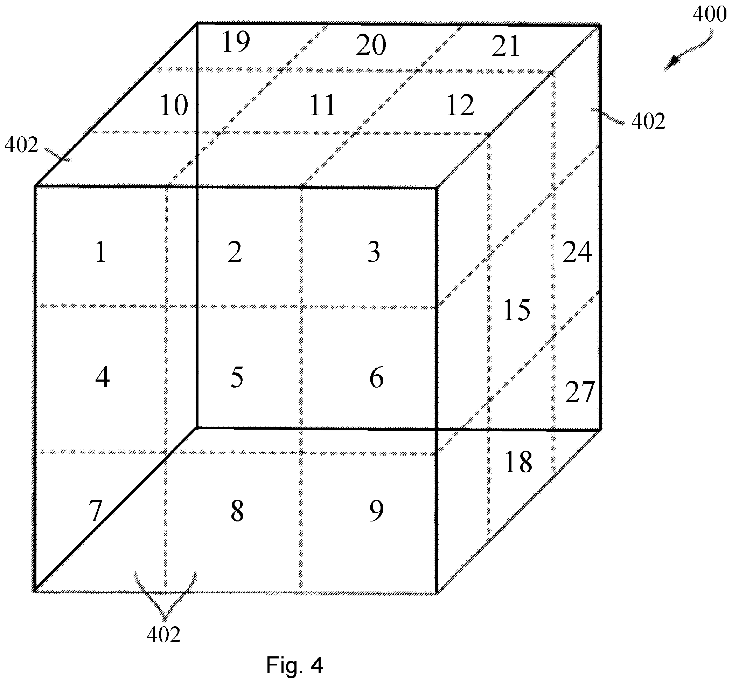

The data representation may be encoded in a way that enables the expression of various structural relationships associated with molecules/proteins for example. The geometric representation may be implemented in a variety of ways and topographies, according to various embodiments. The geometric representation is used for the visualization and analysis of data. For example, in an embodiment, geometries may be represented using voxels laid out on various topographies, such as 2-D, 3-D Cartesian / Euclidean space, 3-D non-Euclidean space, manifolds, etc. For example, FIG. 4 illustrates a sample three-dimensional grid structure 400 including a series of sub-containers, according to an embodiment. Each sub-container 402 may correspond to a voxel. A coordinate system may be defined for the grid, such that each sub-container has an identifier. In some embodiments of the disclosed systems and methods, the coordinate system is a Cartesian system in 3-D space, but in other embodiments of the system, the coordinate system may be any other type of coordinate system, such as a oblate spheroid, cylindrical or spherical coordinate systems, polar coordinates systems, other coordinate systems designed for various manifolds and vector spaces, among others. In some embodiments, the voxels may have particular values associated to them, which may, for example, be represented by applying labels, and/or determining their positioning, among others.

Because neural networks require a fixed input size, some embodiments of the disclosed systems and methods crop the geometric data (the target-test or target-training object complex) to fit within an appropriate bounding box. For example, a cube of 25 - 40Å to a side, may be used. In some embodiments in which the target and/or test objects have been docketed into the active site of target objects 58, the center of the active site serves as the center of the cube.

While in some embodiments a square cube of fixed dimensions centered on the active site of the target polymer 38 is used to partition the space into the voxel grid, the disclosed systems are not so limited. In some embodiments, any of a variety of shapes is used to partition the space into the voxel grid. In some embodiments, polyhedra, such as rectangular prisms, polyhedra shapes, etc. are used to partition the space.

In an embodiment, the grid structure may be configured to be similar to an arrangement of voxels. For example, each sub-structure may be associated with a channel for each atom being analyzed. Also, an encoding method may be provided for representing each atom numerically.

In some embodiments, the voxel map takes into account the factor of time (e.g. along a molecular dynamics run of the training compound pose and the target polymer) and may thus be in four dimensions (X, Y, Z, and time).

In some embodiments, other implementations such as pixels, points, polygonal shapes, polyhedrals, or any other type of shape in multiple dimensions (*e.g.* shapes in 3D, 4D, and so on) may be used instead of voxels.

In some embodiments, the geometric data is normalized by choosing the origin of the X, Y and Z coordinates to be the center of mass of a binding site of the target polymer 38 as determined by a cavity flooding algorithm. For representative details of such algorithms, see Ho and Marshall, 1990, "Cavity search: An algorithm for the isolation and display of cavity-like binding regions," Journal of Computer-Aided Molecular Design 4, pp. 337-354; and Hendlich et al., 1997, "Ligsite: automatic and efficient detection of potential small molecule-binding sites in proteins," J. Mol. Graph. Model 15:6. Alternatively, in some embodiments, the origin of the voxel map is centered at the center of mass of the entire co-complex (of the training compound docked in the respective pose - positive pose 48 or negative pose 60- bound to the target polymer). In some embodiments, the origin of the voxel map is centered at the center of mass of the training compound. In some embodiments, the origin of the voxel map is centered at the center of mass of the target polymer 38. The basis vectors may optionally be chosen to be the principal moments of inertia of the entire co-complex, of just the target polymer, or of just the training compounds. In some embodiments, the target polymer 38 has an active site, and the sampling samples the training compound, in both the positive pose 48 and the negative pose 60, and the active site on the three-dimensional grid basis in which a center of mass of the active site is taken as the origin and the corresponding three dimensional uniform honeycomb for the sampling represents a portion of the polymer and the training compound centered on the center of mass. In some embodiments, the uniform honeycomb is a regular cubic honeycomb and the portion of the polymer and the test object is a cube of predetermined fixed dimensions. Use of a cube of predetermined fixed dimensions, in such embodiments, ensures that a relevant portion of the geometric data is used and that each voxel map is the same size. In some embodiments, the predetermined fixed dimensions of the cube are N Å x N Å x N Å, where N is an integer or real value between 5 and 100, an integer between 8 and 50, or an integer between 15 and 40. In some embodiments, the uniform honeycomb is a rectangular prism honeycomb and the portion of the polymer and the training compound is a rectangular prism predetermined fixed dimensions Q Å x R Å x S Å, wherein Q is a first integer between 5 and 100, R is a second integer between 5 and 100, S is a third integer or real value between 5 and 100, and at least one number in the set {Q, R, S} is not equal to another value in the set {Q, R, S}.

In an embodiment, every voxel has one or more input channels, which may have various values associated with them, which in a simple implementation could be on/off, and may be configured to encode for a type of atom. Atom types may denote the element of the atom, or atom types may be further refined to distinguish between other atom characteristics. Atoms present may then be encoded in each voxel. Various types of encoding may be utilized using various techniques and/or methodologies. As an example encoding method, the atomic number of the atom may be utilized, yielding one value per voxel ranging from one for hydrogen to 118 for ununoctium (or any other element).

However, as discussed above, other encoding methods may be utilized, such as "one-hot encoding," where every voxel has many parallel input channels, each of which is either on or off and encodes for a type of atom. Atom types may denote the element of the atom, or atom types may be further refined to distinguish between other atom characteristics. For example, SYBYL atom types distinguish single-bonded carbons from double-bonded, triple-bonded, or aromatic carbons. For SYBYL atom types, see Clark et al., 1989, "Validation of the General Purpose Tripos Force Field, 1989, J. Comput. Chem. 10, pp. 982-1012.

In some embodiments, each voxel further includes one or more channels to distinguish between atoms that are part of the target polymer 38 or cofactors versus part of the training compound. For example, in one embodiment, each voxel further includes a first channel for the target polymer 38 and a second channel for the training compound. When an atom in the portion of space represented by the voxel is from the target polymer 38, the first channel is set to a value, such as "1", and is zero otherwise (*e.g.,* because the portion of space represented by the voxel includes no atoms or one or more atoms from the training compound). Further, when an atom in the portion of space represented by the voxel is from the training compound, the second channel is set to a value, such as "1", and is zero otherwise (*e.g.,* because the portion of space represented by the voxel includes no atoms or one or more atoms from the target polymer 38). Likewise, other channels may additionally (or alternatively) specify further information such as partial charge, polarizability, electronegativity, solvent accessible space, and electron density. For example, in some embodiments, an electron density map for the target object overlays the set of three-dimensional coordinates, and the creation of the voxel map further samples the electron density map. Examples of suitable electron density maps include, but are not limited to, multiple isomorphous replacement maps, single isomorphous replacement with anomalous signal maps, single wavelength anomalous dispersion maps, multi-wavelength anomalous dispersion maps, and 2Fo-Fc maps (260). *See* McRee, 1993, Practical Protein Crystallography, Academic Press.

In some embodiments, voxel encoding in accordance with the disclosed systems and methods may include additional optional encoding refinements. The following two are provided as examples.

In a first encoding refinement, the required memory may be reduced by reducing the set of atoms represented by a voxel (*e.g.,* by reducing the number of channels represented by a voxel) on the basis that most elements rarely occur in biological systems. Atoms may be mapped to share the same channel in a voxel, either by combining rare atoms (which may therefore rarely impact the performance of the system) or by combining atoms with similar properties (which therefore could minimize the inaccuracy from the combination). In some embodiments, two, three, four, five, six, seven, eight, nine, or ten different atoms share the same channel in a voxel.

An encoding refinement is to have voxels represent atom positions by partially activating neighboring voxels. This results in partial activation of neighboring neurons in the subsequent neural network and moves away from one-hot encoding to a "several-warm" encoding. For example, it may be illustrative to consider a chlorine atom, which has a van der Waals diameter of 3.5Å and therefore a volume of 22.4Å³ when a 1Å³ grid is placed, voxels inside the chlorine atom will be completely filled and voxels on the edge of the atom will only be partially filled. Thus, the channel representing chlorine in the partially-filled voxels will be turned on proportionate to the amount such voxels fall inside the chlorine atom. For instance, if fifty percent of the voxel volume falls within the chlorine atom, the channel in the voxel representing chlorine will be activated fifty percent. This may result in a "smoothed" and more accurate representation relative to the discrete one-hot encoding. Thus, in some embodiments, a characteristic of an atom incurred in the sampling is spread across a subset of voxels in the voxel map and this subset of voxels comprises two or more voxels, three or more voxels, five or more voxels, ten or more voxels, or twenty-five or more voxels. In some embodiments, the characteristic of the atom consists of an enumeration of the atom type *(e.g.,* one of the SYBYL atom types).

Thus, voxelation (rasterization) of the geometric data (the docking of a test or training object onto a target object) that has been encoded is based upon various rules applied to the input data.

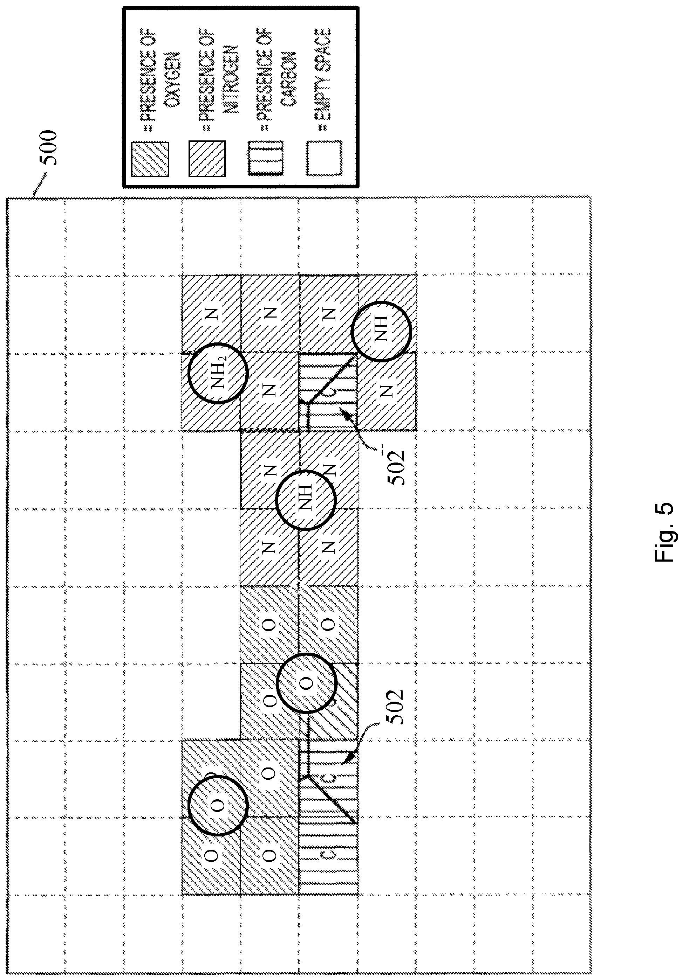

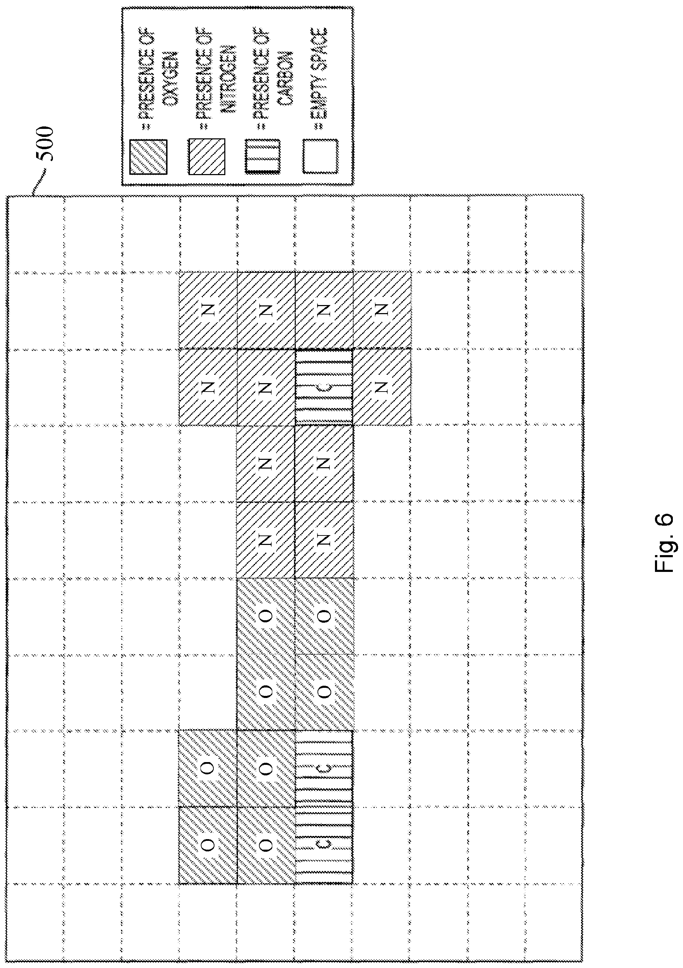

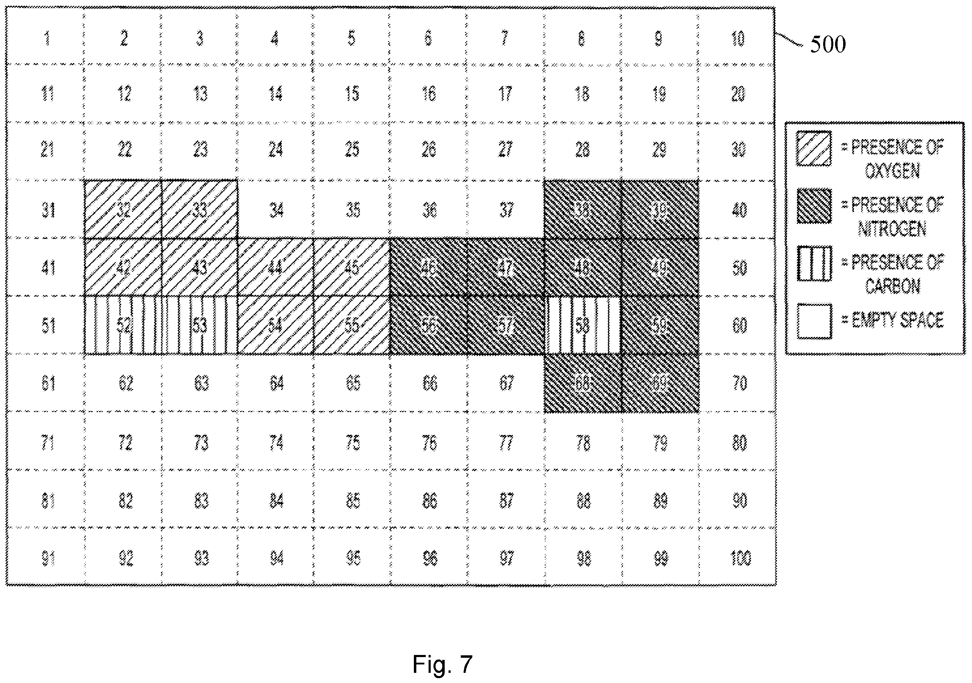

FIG. 5 and FIG. 6 provide views of two molecules 502 encoded onto a two dimensional grid 500 of voxels, according to some embodiments. FIG. 5 provides the two molecules superimposed on the two dimensional grid. FIG. 6 provides the one-hot encoding, using the different shading patterns to respectively encode the presence of oxygen, nitrogen, carbon, and empty space. As noted above, such encoding may be referred to as "one-hot" encoding. FIG. 6 shows the grid 500 of FIG. 5 with the molecules 502 omitted. FIG. 7 provides a view of the two dimensional grid of voxels of FIG. 6, where the voxels have been numbered.

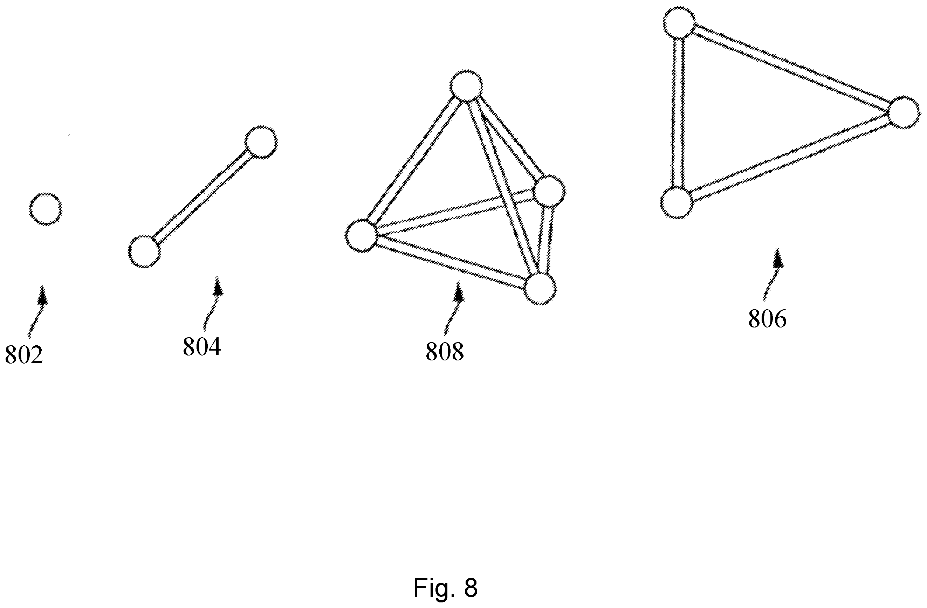

In some embodiments, feature geometry is represented in forms other than voxels. FIG. 8 provides a view of various representations in which features (*e.g.,* atom centers) are represented as 0-D points (representation 802), 1-D points (representation 804), 2-D points (representation 806), or 3-D points (representation 808). Initially, the spacing between the points may be randomly chosen. However, as the predictive model is trained, the points may be moved closer together, or farther apart.

In some embodiments, the input representation can be in the form of 1D-array of features including, but not limited to, three-dimensional coordinates.

In some embodiments, the neural network 24 is a graph convolutional neural network. Nonlimiting examples of graph convolutional neural networks are disclosed in Behler Parrinello, 2007, "Generalized Neural-Network Representation of High Dimensional Potential-Energy Surfaces," Physical Review Letters 98, 146401; Chmiela, et al., 2017, "Machine learning of accurate energy-conserving molecular force fields," Science Advances 3(5):e1603015; Schütt et al., 2017, "SchNet: A continuous-filter convolutional neural network for modeling quantum interactions," Advances in Neural Information Processing Systems 30, pp. 992-1002; Feinberg et al., 2018, "PotentialNet for Molecular Property Prediction," ACS Cent. Sci. 4, 11, 1520-1530; and Stafford et al., "AtomNet PoseRanker: Enriching Ligand Pose Quality for Dynamic Proteins in Virtual High Throughput Screens," https://chemrxiv.org/engage/chemrxiv/article-details/614b905e39ef6a1c36268003.

In some embodiments, the neural network is an equivariant neural network. Nonlimiting examples of the equivariant convolutional neural network are disclosed in Thomas et al., 2018, "Tensor field networks: Rotation- and translation-equivariant neural networks for 3D point clouds," arXiv: 1802.08219; Anderson et al., 2019, "Cormorant: Covariant Molecular Neural Networks," Neural Information Processing Systems; Johannes et al., 2020, "Directional Message Passing For Molecular Graphs," International Conference on Learning Representations; Townshend et al., 2021, "ATOM3D: Tasks On Molecules in Three Dimensions," International Conference on Learning Representations; Jing et al., 2009, "Learning from Protein Structure with Geometric Vector Perceptrons," arXiv: 2009.01411; and Satorras et al., 2021, "E(n) Equivariant Graph Neural Networks," arXiv: 2102 09844.

In some embodiments the neural network 24 is any of the graph neural networks disclosed in United States Provisional Patent Application No. 63/336,841, entitled "Characterization of Interactions Between Compounds and Polymers Using Pose Ensembles," filed May 10, 2022.

***Unfolding a voxel map into a corresponding vector.*** Each voxel map *(e.g.,* positive voxel map 52 and negative voxel map 64) is optionally unfolded into a corresponding vector (*e.g.* positive vector 54 and negative vector 66 for each training compound in the training dataset 40). In some embodiments, each such vector is a one-dimensional vector. For instance, in some embodiments, a cube of 20 Å on each side is centered on the active site of the target polymer 38 and is sampled with a three-dimensional fixed grid spacing of 1 Å to form corresponding voxels of a voxel map that hold in respective channels basic of the voxel structural features such as atom types as well as, optionally, more complex training compound- target polymer descriptors, as discussed above. In some embodiments, the voxels of this three-dimensional voxel map are unfolded into a one-dimensional floating point vector.

In some embodiments, the vectorized representation of voxel maps (*e.g.* positive vector 54 and negative vector 66 for each training compound in the training dataset 40) are subjected to a neural network 24. In some embodiments, as illustrated in Figure 1B, the vectorized representation of voxel maps are stored in the GPU memory 52 along with a assessment module 20, and a neural network 24. This provides the advantage of processing the vectorized representation of voxel maps through the neural network 24 at faster speeds. However, in other embodiments, any or all of the vectorized representations of voxel maps (*e.g.* positive vector 54 and negative vector 66 for each training compound in the training dataset 40), the assessment module 20, and the neural network 24 are in memory 92 of system 100 or simply are addressable by system 92 across a network. In some embodiments, any or all of the vectorized representation of voxel maps, the assessment module 20, and the neural network 24 are in a cloud computing environment.

In some embodiments, the vectors (*e.g.* positive vector 54 and negative vector 66 for each training compound in the training dataset 40) is provided to the graphical processing unit memory 52, where the graphical processing unit memory includes a network architecture that includes a neural network 24 comprising an input layer 26 for sequentially receiving the plurality of vectors, optionally a plurality of convolutional layers 28, and a scorer 30. In some embodiments, the optional plurality of convolutional layers includes an initial convolutional layer and a final convolutional layer. In some embodiments, the neural network 24 is not in GPU memory but is in the general purpose memory of system 100. In some embodiments, the voxel maps are not vectorized before being input into network 24.

In some embodiments that make user of convolutional layers 28, a convolutional layer 28 in the plurality of convolutional layers comprises a set of learnable filters (also termed kernels). Each filter has fixed three-dimensional size that is convolved (stepped at a predetermined step rate) across the depth, height and width of the input volume of the convolutional layer, computing a dot product (or other functions) between entries (weights, or more generally parameters) of the filter and the input thereby creating a multi-dimensional activation map of that filter. In some embodiments, the filter step rate is one element, two elements, three elements, four elements, five elements, six elements, seven elements, eight elements, nine elements, ten elements, or more than ten elements of the input space. Thus, consider the case in which a filter has size 5³. In some embodiments, this filter will compute the dot product (or other mathematical function) between a contiguous cube of input space that has a depth of five elements, a width of five elements, and a height of five elements, for a total number of values of input space of 125 per voxel channel.

The input space to the initial convolutional layer (*e.g.,* the output from the input layer 26) is formed from either a voxel map or a vectorized representation of the voxel map (*e.g.* positive vector 54 and negative vector 66 for each training compound in the training dataset 40). In some embodiments, the vectorized representation of the voxel map is a one-dimensional vectorized representation of the voxel map that serves as the input space to the initial convolutional layer. Nevertheless, when a filter convolves its input space and the input space is a one-dimensional vectorized representation of the voxel map, the filter still obtains from the one-dimensional vectorized representation those elements that represent a corresponding contiguous cube of fixed space in the target polymer 38 - training compound complex. In some embodiments, the filter uses bookkeeping techniques to select those elements from within the one-dimensional vectorized representation that form the corresponding contiguous cube of fixed space in the target polymer 38 - training compound complex. Thus, in some instances, this necessarily involves taking a non-contiguous subset of elements in the one-dimensional vectorized representation in order to obtain the element values of the corresponding contiguous cube of fixed space in the target polymer 38 - training compound complex.

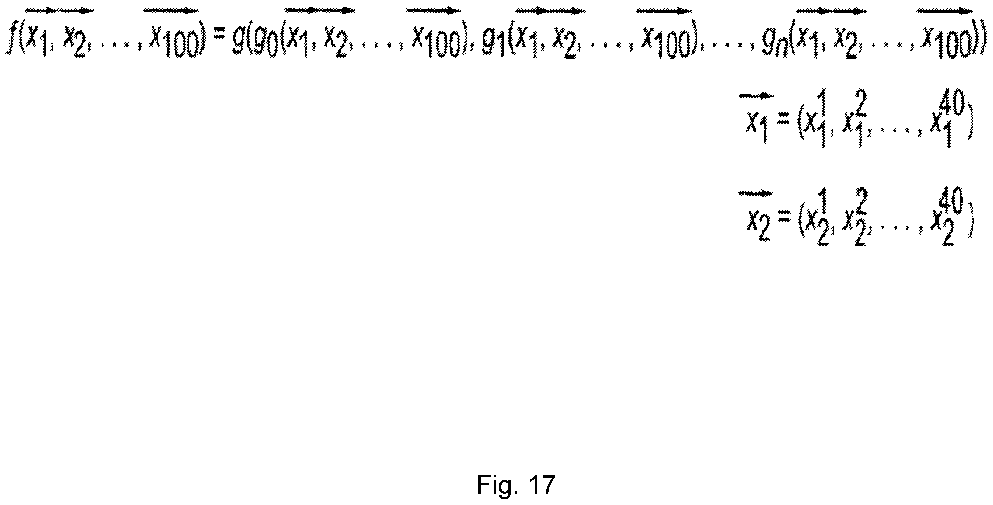

In some embodiments, the filter is initialized (*e.g.,* to Gaussian noise) or trained to have 125 corresponding weights (per input channel) in which to take the dot product (or some other form of mathematical operation such as the function disclosed in Figure 14) of the 125 input space values in order to compute a first single value (or set of values) of the activation layer corresponding to the filter. In some embodiment the values computed by the filter are summed, weighted, and/or biased. To compute additional values of the activation layer corresponding to the filter, the filter is then stepped (convolved) in one of the three dimensions of the input volume by the step rate (stride) associated with the filter, at which point the dot product (or some other form of mathematical operation such as the mathematical function disclosed in Figure 17) between the filter weights and the 125 input space values (per channel) is taken at the new location in the input volume is taken. This stepping (convolving) is repeated until the filter has sampled the entire input space in accordance with the step rate. In some embodiments, the border of the input space is zero padded to control the spatial volume of the output space produced by the convolutional layer. In typical embodiments, each of the filters of the convolutional layer canvas the entire three-dimensional input volume in this manner thereby forming a corresponding activation map. The collection of activation maps from the filters of the convolutional layer collectively form the three-dimensional output volume of one convolutional layer, and thereby serves as the three-dimensional (three spatial dimensions) input of a subsequent convolutional layer. Every entry in the output volume can thus also be interpreted as an output of a single neuron (or a set of neurons) that looks at a small region in the input space to the convolutional layer and shares parameters with neurons in the same activation map. Accordingly, in some embodiments, a convolutional layer in the plurality of convolutional layers has a plurality of filters and each filter in the plurality of filters convolves (in three spatial dimensions) a cubic input space of N³ with stride Y, where N is an integer of two or greater (*e.g.,* 2, 3, 4, 5, 6, 7, 8, 9, 10, or greater than 10) and Y is a positive integer (*e.g.* 1, 2, 3, 4, 5, 6, 7, 8, 9, 10, or greater than 10).

Each layer in the plurality of convolutional layers is associated with a different set of weights, or more generally a different set of parameters. With more particularity, each layer in the plurality of convolutional layers includes a plurality of filters and each filter comprises an independent plurality of parameters (*e.g.,* weights). In some embodiments, a convolutional layer has 128 filters of dimension 5³ and thus the convolutional layer has 128 x 5 x 5 x 5 or 16,000 parameters (*e.g.,* weights) per channel in the voxel map. Thus, if there are five channels in the voxel map, the convolutional layer will have 16,000 x 5 parameters (*e.g.,* weights), or 80,000 parameters (*e.g.,* weights). In some embodiments some or all such parameters (and, optionally, biases) of every filter in a given convolutional layer may be tied together, *e.g.* constrained to be identical.

Responsive to input of a respective vector (e.g., positive vector 54 or negative vector 66), the input layer 26 feeds a first plurality of values into the initial convolutional layer as a first function of values in the respective vector, where the first function is optionally computed using a graphical processing unit 50. In some embodiments, the computer system 100 has more than one graphical processing unit 50.

Each respective convolutional layer 28, other than the final convolutional layer, feeds intermediate values, as a respective second function of (i) the different set of parameters (*e.g.,* weights) associated with the respective convolutional layer and (ii) input values received by the respective convolutional layer, into another convolutional layer in the plurality of convolutional layers. In some embodiments the second function is computed using the graphical processing unit 50. For instance, in some embodiments, each respective filter of the respective convolutional layer 28 canvasses the input volume (in three spatial dimensions) to the convolutional layer in accordance with the characteristic three-dimensional stride of the convolutional layer and at each respective filter position, takes the dot product (or some other mathematical function) of the filter parameters (*e.g.,* weights) of the respective filter and the values of the input volume (contiguous cube that is a subset of the total input space) at the respect filter position thereby producing a calculated point (or a set of points) on the activation layer corresponding to the respective filter position. The activation layers of the filters of the respective convolutional layer collectively represent the intermediate values of the respective convolutional layer.

The final convolutional layer feeds final values, as a third function of (i) the different set of parameters (*e.g.,* weights) associated with the final convolutional layer and (ii) input values received by the final convolutional layer that is optionally computed using the graphical processing unit 50, into the scorer. For instance, each respective filter of the final convolutional layer 28 canvasses the input volume (in three spatial dimensions) to the final convolutional layer in accordance with the characteristic three-dimensional stride of the convolutional layer and at each respective filter position, takes the dot product (or some other mathematical function) of the filter weights of the filter and the values of the input volume at the respect filter position thereby calculating a point (or a set of points) on the activation layer corresponding to the respective filter position. The activation layers of the filters of the final convolutional layer collectively represent the final values that are fed to the scorer 30.

In some embodiments, the convolutional neural network has one or more activation layers. In some embodiments, the activation layer is a layer of neurons that applies the non-saturating activation function f(x) = max(0, x). It increases the nonlinear properties of the decision function and of the overall network without affecting the receptive fields of the convolution layer. In other embodiments, the activation layer has other functions to increase nonlinearity, for example, the saturating hyperbolic tangent function f(x) = tanh, f(x) = | tanh(x) | , and the sigmoid function f(x) = (1 +e^{-x})⁻¹. Nonlimiting examples of other activation functions found in other activation layers in some embodiments for the neural network may include, but are not limited to, logistic (or sigmoid), softmax, Gaussian, Boltzmann-weighted averaging, absolute value, linear, rectified linear, bounded rectified linear, soft rectified linear, parameterized rectified linear, average, max, min, some vector norm LP (for p=1, 2, 3, ... ,∞), sign, square, square root, multiquadric, inverse quadratic, inverse multi quadric, polyharmonic spline, and thin plate spline.

The network 24 learns filters within the convolutional layers 28 that activate when they see some specific type of feature at some spatial position in the input. In some embodiments, the initial parameters (*e.g.,* weights) of each filter in a convolutional layer are obtained by training the convolutional neural network against a compound training library. Accordingly, the operation of the convolutional neural network 24 may yield more complex features than the features historically used to conduct binding affinity prediction. For example, a filter in a given convolutional layer of the network 24 that serves as a hydrogen bond detector may be able to recognize not only that a hydrogen bond donor and acceptor are at a given distance and angles, but also recognize that the biochemical environment around the donor and acceptor strengthens or weakens the bond. Additionally, the filters within the network 24 may be trained to effectively discriminate binders from non-binders in the underlying data.

As described above, in some embodiments the neural network 24 is configured to develop three-dimensional convolutional layers. The input region to the lowest level convolutional layer 28 may be a cube (or other contiguous region) of voxel channels from the receptive field. Higher convolutional layers 28 evaluate the output from lower convolutional layers, while still having their output be a function of a bounded region of voxels which are close together (in 3-D Euclidean distance).

In an embodiment, the network 24 is configured to apply regularization techniques to reduce the tendency of the models to overfit the training data.

Zero or more of the network layers in network 24 may consist of pooling layers. As in a convolutional layer, a pooling layer is a set of function computations that apply the same function over different spatially-local patches of input. For pooling layers, the output is given by a pooling operators, e.g. some vector norm LP for p=1, 2, 3, ..., ∞, over several voxels. Pooling is typically done per channel, rather than across channels. Pooling partitions the input space into a set of three-dimensional boxes and, for each such sub-region, outputs the maximum. The pooling operation provides a form of translation invariance. The function of the pooling layer is to progressively reduce the spatial size of the representation to reduce the amount of parameters and computation in the network, and hence to also control overfitting. In some embodiments a pooling layer is inserted between successive convolutional 28 layers in network 24. Such a pooling layer operates independently on every depth slice of the input and resizes it spatially. In addition to max pooling, the pooling units can also perform other functions, such as average pooling or even L2-norm pooling.

Zero or more of the layers in network 24 may consist of normalization layers, such as local response normalization or local contrast normalization, which may be applied across channels at the same position or for a particular channel across several positions. These normalization layers may encourage variety in the response of several function computations to the same input.

In some embodiments, the scorer 30 comprises a plurality of fully-connected layers and an evaluation layer where a fully-connected layer in the plurality of fully-connected layers feeds into the evaluation layer. Neurons in a fully connected layer have full connections to all activations in the previous layer, as seen in regular neural networks. Their activations can hence be computed with a matrix multiplication followed by a bias offset. In some embodiments, each fully connected layer has 512 hidden units, 1024 hidden units, or 2048 hidden units. In some embodiments there are no fully connected layers, one fully connected layer, two fully connected layers, three fully connected layers, four fully connected layers, five fully connected layers, six or more fully connected layers or ten or more fully connected layers in the scorer.

In some embodiments, the evaluation layer discriminates between a plurality of activity classes. In some embodiments, the evaluation layer comprises a logistic regression cost layer over a two activity classes, three activity classes, four activity classes, five activity classes, or six or more activity classes.

In some embodiments, the evaluation layer comprises a logistic regression cost layer over a plurality of activity classes. In some embodiments, the evaluation layer comprises a logistic regression cost layer over a two activity classes, three activity classes, four activity classes, five activity classes, or six or more activity classes.

In some embodiments, the evaluation layer discriminates between two activity classes and the first activity classes (first classification) represents an IC₅₀, EC₅₀ or KI for the training compound with respect to the target polymer that is above a first binding value, and the second activity class (second classification) is an IC₅₀, EC₅₀, or KI for the training compound with respect to the target polymer that is below the first binding value. In some embodiments, the first binding value is one nanomolar, ten nanomolar, one hundred nanomolar, one micromolar, ten micromolar, one hundred micromolar, or one millimolar.

In some embodiments, the evaluation layer comprises a logistic regression cost layer over two activity classes and the first activity classes (first classification) represents an IC₅₀, EC₅₀ or KI for the training compound with respect to the target polymer that is above a first binding value, and the second activity class (second classification) is an IC₅₀, EC₅₀, or KI for the training compound with respect to the target polymer that is below the first binding value. In some embodiments, the first binding value is one nanomolar, ten nanomolar, one hundred nanomolar, one micromolar, ten micromolar, one hundred micromolar, or millimolar.

In some embodiments, the evaluation layer discriminates between three activity classes and the first activity classes (first classification) represents an IC₅₀, EC₅₀ or KI for the training compound with respect to the target polymer that is above a first binding value, the second activity class (second classification) is an IC₅₀, EC₅₀, or KI for the training compound with respect to the target polymer that is between the first binding value and a second binding value, and the third activity class (third classification) is an IC₅₀, EC₅₀, or KI for the training compound with respect to the target polymer that is below the second binding value, where the first binding value is other than the second binding value.

In some embodiments, the evaluation layer comprises a logistic regression cost layer over three activity classes and the first activity classes (first classification) represents an IC₅₀, EC₅₀ or KI for the training compound with respect to the target polymer that is above a first binding value, the second activity class (second classification) is an IC₅₀, EC₅₀, or KI for the training compound with respect to the target polymer that is between the first binding value and a second binding value, and the third activity class (third classification) is an IC₅₀, EC₅₀, or KI for the training compound with respect to the target polymer that is below the second binding value, where the first binding value is other than the second binding value.

In some embodiments, the scorer 30 comprises a fully connected single layer or multilayer perceptron. In some embodiments the scorer comprises a support vector machine, random forest, nearest neighbor. In some embodiments, the scorer 30 assigns a numeric score indicating the strength (or confidence or probability) of classifying the input into the various output categories. In some cases, the categories are binders and nonbinders or, alternatively, the potency level (IC₅₀, EC₅₀ or KI potencies of e.g., < 1 molar, < 1 millimolar, < 100 micromolar, < 10 micromolar, < 1 micromolar, < 100 nanomolar, < 10 nanomolar, < 1 nanomolar).

### Use Cases.

The following are sample use cases provided for illustrative purposes only that describe some applications of some embodiments of the present disclosure. Other uses may be considered, and the examples provided below are non-limiting and may be subject to variations, omissions, or may contain additional elements.

*Hit discovery.* Pharmaceutical companies spend millions of dollars on screening compounds to discover new prospective drug leads. Large compound collections are tested to find the small number of compounds that have any interaction with the disease target of interest. Unfortunately, wet lab screening suffers experimental errors and, in addition to the cost and time to perform the assay experiments, the gathering of large screening collections imposes significant challenges through storage constraints, shelf stability, or chemical cost. Even the largest pharmaceutical companies have only between hundreds of thousands to a few millions of compounds, versus the tens of millions of commercially available molecules and the hundreds of millions of simulate-able molecules.

A potentially more efficient alternative to physical experimentation is virtual high throughput screening. In the same manner that physics simulations can help an aerospace engineer to evaluate possible wing designs before a model is physically tested, computational screening of molecules can focus the experimental testing on a small subset of high-likelihood molecules. This may reduce screening cost and time, reduces false negatives, improves success rates, and/or covers a broader swath of chemical space.

In this application, a protein target may be provided as input to the system. A large set of molecules may also be provided. For each molecule, a binding affinity is predicted against the protein target. The resulting scores may be used to rank the molecules, with the best-scoring molecules being most likely to bind the target protein. Optionally, the ranked molecule list may be analyzed for clusters of similar molecules; a large cluster may be used as a stronger prediction of molecule binding, or molecules may be selected across clusters to ensure diversity in the confirmatory experiments.

*Off-target side-effect prediction.* Many drugs may be found to have side-effects. Often, these side-effects are due to interactions with biological pathways other than the one responsible for the drug's therapeutic effect. These off-target side-effects may be uncomfortable or hazardous and restrict the patient population in which the drug's use is safe. Off-target side effects are therefore an important criterion with which to evaluate which drug candidates to further develop. While it is important to characterize the interactions of a drug with many alternative biological targets, such tests can be expensive and time-consuming to develop and run. Computational prediction can make this process more efficient.

In applying an embodiment of the invention, a panel of biological targets may be constructed that are associated with significant biological responses and/or side-effects. The system may then be configured to predict binding against each protein in the panel in turn. Strong activity (that is, activity as potent as compounds that are known to activate the off-target protein) against a particular target may implicate the molecule in side-effects due to off-target effects.

*Toxicity prediction.* Toxicity prediction is a particularly-important special case of off-target side-effect prediction. Approximately half of drug candidates in late stage clinical trials fail due to unacceptable toxicity. As part of the new drug approval process (and before a drug candidate can be tested in humans), the FDA requires toxicity testing data against a set of targets including the cytochrome P450 liver enzymes (inhibition of which can lead to toxicity from drug-drug interactions) or the hERG channel (binding of which can lead to QT prolongation leading to ventricular arrhythmias and other adverse cardiac effects).

In toxicity prediction, the system may be configured to constrain the off-target proteins to be key antitargets (e.g. CYP450, hERG, or 5-HT_{2B} receptor). The binding affinity for a drug candidate may then be predicted against these proteins. Optionally, the molecule may be analyzed to predict a set of metabolites (subsequent molecules generated by the body during metabolism/degradation of the original molecule), which can also be analyzed for binding against the antitargets. Problematic molecules may be identified and modified to avoid the toxicity or development on the molecular series may be halted to avoid wasting additional resources.

*Potency optimization.* One of the key requirements of a drug candidate is strong binding against its disease target. It is rare that a screen will find compounds that bind strongly enough to be clinically effective. Therefore, initial compounds seed a long process of optimization, where medicinal chemists iteratively modify the molecular structure to propose new molecules with increased strength of target binding. Each new molecule is synthesized and tested, to determine whether the changes successfully improved binding. The system may be configured to facilitate this process by replacing physical testing with computational prediction.

In this application, the disease target and a set of lead molecules may be input into the system. The system may be configured to produce binding affinity predictions for the set of leads. Optionally, the system could highlight differences between the candidate molecules that could help inform the reasons for the predicted differences in binding affinity. The medicinal chemist user can use this information to propose a new set of molecules with, hopefully, improved activity against the target. These new alternative molecules may be analyzed in the same manner.

*Selectivity optimization.* As discussed above, molecules tend to bind a host of proteins at a variety of strengths. For example, the binding pockets of protein kinases (which are popular chemotherapy targets) are very similar and most kinase inhibitors affect many different kinases. This means that various biological pathways are simultaneously modified, which yields a "dirty" medicinal profile and many side-effects. The critical challenge in the design of many drugs, therefore, is not activity *per se* but specificity: the ability to selectively target one protein (or a subset of proteins) from a set of possibly-closely related proteins.

The system can reduce the time and cost of optimizing the selectivity of a candidate drug. In this application, a user may input two sets of proteins. One set describes proteins against which the compound should be active, while the other set describes proteins against which the compound should be inactive. The system may be configured to make predictions for the molecule against all of the proteins in both sets, establishing a profile of interaction strengths. Optionally, these profiles could be analyzed to suggest explanatory patterns in the proteins. The user can use the information generated by the system to consider structural modifications to a molecule that would improve the relative binding to the different protein sets, and to design new candidate molecules with better specificity. Optionally, the system could be configured to highlight differences between the candidate molecules that could help inform the reasons for the predicted differences in selectivity. The proposed candidates can be analyzed iteratively, to further refine the specificity of their activity profiles.

Fitness function for automated molecular design: Automated tools to perform the preceding optimizations are valuable. A successful molecule requires optimization and balance among potency, selectivity, and toxicity. "Scaffold hopping" (when the activity of a lead compound is preserved but the chemical structure is significantly altered) can yield improved pharmacokinetics, pharmacodynamics, toxicity, or intellectual property profiles. Algorithms exist to iteratively suggest new molecules, such as random generation of molecules, growth of molecular fragments to fill a given binding site, genetic algorithms to "mutate" and "cross-breed" a population of molecules, and swapping of pieces of a molecule with bioisosteric replacements. The drug candidates generated by each of these methods must be evaluated against the multiple objectives described above (potency, selectivity, toxicity) and, in the same way that the technology can be informative on each of the preceding manual settings (binding prediction, selectivity, side-effect and toxicity prediction), it can be incorporated in an automated molecular design system.

*Drug repurposing.* All drugs have side-effects and, from time to time, these side-effects are beneficial. The best known example might be aspirin, which is generally used as a headache treatment but is also taken for cardiovascular health. Drug repositioning can significantly reduce the cost, time, and risk of drug discovery because the drugs have already been shown to be safe in humans and have been optimized for rapid absorption and favorable stability in patients. Unfortunately, drug repositioning has been largely serendipitous. For example, sildenafil (Viagra), was developed as a hypertension drug and was unexpectedly observed to be an effective treatment for erectile dysfunction. Computational prediction of off-target effects can be used in the context of drug repurposing to identify compounds that could be used to treat alternative diseases.

In this application, as in off-target side-effect prediction, the user may assemble a set of possible target proteins, where each protein is linked to a disease. That is, inhibition of each protein would treat a (possibly different) disease; for example, inhibitors of Cyclooxygenase-2 can provide relief from inflammation, whereas inhibitors of Factor Xa can be used as anticoagulants. These proteins are annotated with the binding affinity of approved drugs, if any exist. We then assemble a set of molecules, restricting the set to molecules that have been approved or investigated for use in humans. Finally, for each pair of protein and molecule, the user may use the system to predict the binding affinity. Candidates for drug repurposing may be identified if the predicted binding affinity of the molecule is close to the binding affinity of effective drugs for the protein.

*Drug resistance prediction.* Drug resistance is an inevitable outcome of pharmaceutical use, which puts selection pressure on rapidly dividing and mutating pathogen populations. Drug resistance is seen in such diverse disease agents as viruses (HIV), exogenous microorganisms (MRSA), and disregulated host cells (cancers). Over time, a given medicine will become ineffective, irrespective of whether the medicine is antibiotics or chemotherapies. At that point, the intervention can shift to a different medicine that is, hopefully, still potent. In HIV, there are well-known disease progression pathways that are defined by which mutations the virus will accumulate while the patient is being treated.

There is considerable interest in *predicting* how disease agents adapt to medical intervention. One approach is to characterize which mutations will occur in the disease agent while under treatment. Specifically, the protein target of a medicine needs to mutate so as to avoid binding the drug while simultaneously continuing to bind its natural substrate.

In this application, a set of possible mutations in the target protein may be proposed. For each mutation, the resulting protein shape may be predicted. For each of these mutant protein forms, the system may be configured to predict a binding affinity for both the natural substrate and the drug. The mutations that cause the protein to no longer bind to the drug but also to continue binding to the natural substrate are candidates for conferring drug resistance. These mutated proteins may be used as targets against which to design drugs, e.g. by using these proteins as inputs to one of these other prediction use cases.

*Personalized medicine.* Ineffective medicines should not be administered. In addition to the cost and hassle, all medicines have side-effects. Moral and economic considerations make it imperative to give medicines only when the benefits outweigh these harms. It may be important to be able to predict when a medicine will be useful. People differ from one another by a handful of mutations. However, small mutations may have profound effects. When these mutations occur in the disease target's active (orthosteric) or regulatory (allosteric) sites, they can prevent the drug from binding and, therefore, block the activity of the medicine. When a particular person's protein structure is known (or predicted), the system can be configured to predict whether a drug will be effective or the system may be configured to predict when the drug will *not* work.

For this application, the system may be configured to receive as input the drug's chemical structure and the specific patient's particular expressed protein. The system may be configured to predict binding between the drug and the protein and, if the drug's predicted binding affinity that particular patient's protein structure is too weak to be clinically effective, clinicians or practitioners may prevent that drug from being fruitlessly prescribed for the patient.

*Drug trial design.* This application generalizes the above personalized medicine use case to the case of patient populations. When the system can predict whether a drug will be effective for a particular patient phenotype, this information can be used to help design clinical trials. By excluding patients whose particular disease targets will not be sufficiently affected by a drug, a clinical trial can achieve statistical power using fewer patients. Fewer patients directly reduces the cost and complexity of clinical trials.

For this application, a user may segment the possible patient population into subpopulations that are characterized by the expression of different proteins (due to, for example, mutations or isoforms). The system may be configured to predict the binding strength of the drug candidate against the different protein types. If the predicted binding strength against a particular protein type indicates a necessary drug concentration that falls below the clinically-achievable in-patient concentration (as based on, for example, physical characterization in test tubes, animal models, or healthy volunteers), then the drug candidate is predicted to fail for that protein subpopulation. Patients with that protein may then be excluded from a drug trial.

*Agrochemical design.* In addition to pharmaceutical applications, the agrochemical industry uses binding prediction in the design of new pesticides. For example, one desideratum for pesticides is that they stop a single species of interest, without adversely impacting any other species. For ecological safety, a person could desire to kill a weevil without killing a bumblebee.

For this application, the user could input a set of protein structures, from the different species under consideration, into the system. A subset of proteins could be specified as the proteins against which to be active, while the rest would be specified as proteins against which the molecules should be inactive. As with previous use cases, some set of molecules (whether in existing databases or generated de novo) would be considered against each target, and the system would return the molecules with maximal effectiveness against the first group of proteins while avoiding the second.

*Materials science.* To predict the behavior and properties of new materials, it may be useful to analyze molecular interactions. For example, to study solvation, the user may input a repeated crystal structure of a given small molecule and assess the binding affinity of another instance of the small molecule on the crystal's surface. To study polymer strength, a set of polymer strands may be input analogously to a protein target structure, and an oligomer of the polymer may be input as a small molecule. Binding affinity between the polymer strands may therefore be predicted by the system.

*Simulation.* Simulators often measure the binding affinity of a molecule to a protein, because the propensity of a molecule to stay in a region of the protein is correlates to its binding affinity there. An accurate description of the features governing binding could be used to identify regions and poses that have particularly high or low binding energy. The energetic description can be folded into Monte Carlo simulations to describe the motion of a molecule and the occupancy of the protein binding region. Similarly, stochastic simulators for studying and modeling systems biology could benefit from an accurate prediction of how small changes in molecule concentrations impact biological networks.

### EXAMPLES

*AtomNet^{®} Carbon: Learning physics and geometry confers pose-sensitivity onto structure-based virtual high-throughput screening architectures.*

Molecular bioactivity is an ensemble property, determined by enthalpic and entropic components of receptor-compound complex formation. Structure-based deep learning methods have been successful in activity prediction, but can be insensitive to the docked poses, decreasing the reliability of hit detection. Furthermore, structure-based deep learning methods often ignore the entropic contribution to the change in free energy. Ensemble approaches are successful when the ensemble is sensitive to poses. This example describes a deep learning multi-task architecture, with the increased sensitivity to the docked poses.

### 1 Introduction

Vast, make-on-demand chemical libraries like ENAMINE or Mcule have transformed the scale of pharmaceutical, structure-based virtual high-throughput screening (vHTS) campaigns [1]. To identify a 'hit' from a library of candidate molecules, structure-based virtual screening methods predict binding affinity between a protein and a ligand from their docked, bound complex, thereby assuming that experimentally observed affinities correlate with protein-ligand interactions. Conventional methods use empirical, physics-based approaches, which attempt to calculate the binding free energy of complex formation. By contrast, machine learning (ML) and deep learning (DL) approaches are trained on large data sets using explicit (ML) or implicit (DL) features and labels to predict activity. These statistical models generally outperform physics-based approaches in retrospective tests for predicting activity.

Early structure-based DL methods in vHTS centered on convolutional neural networks (CNNs), representing protein-ligand structures by a 3D grid to predict activity[2-5]. Although generally effective [6], a drawback of CNNs is that they are not rotationally invariant, and require more parameters than alternative representations. Consequently, graph convolutional networks [7], or more generally, message passing neural networks [8-10] have gained popularity. Recent studies have suggested that the performance of structure-based machine learning methods is partly driven by proteochemometric-like features [11, 12, 5]. Rather than responding to specific interactions between the ligand and the binding site, the model learns a general ligand-protein signature. This deficiency manifests itself by a drop in predictive performance when the model is confronted with a previously unseen binding site on the same protein, especially when that site partially overlaps with a canonical site. For example, the model may highly rank ATP-competitive binders for an allosteric site on kinases. This limitation critically hinders discovery of new chemical matter, or the ability to target novel sites on proteins.

Simultaneous training on ligand pose quality and affinity can improve pose sensitivity [13]. Here, we build on that observation and present a multi-task architecture for bioactivity prediction, which simultaneously evaluates bioactivity and the physics-based vina [14] score of the pose. We furthermore condition its bioactivity task on pose quality. Finally, we expose the model to poor poses while negating the bioactivity label for bioactive molecules, thus presenting poor poses of true binders as negative examples. We demonstrate that our architecture improves pose-sensitivity on several rigorous benchmarks.

### 2.1 Neural Network Architectures

The system of this example includes is a graph-neural network based architecture with position dependent edges. This is an example of convolutional neural network 24 of the present disclosure. In this example, we consider only receptor atoms within 7 Å of any ligand atom. We use two graph convolutional layers where (ligand and receptor) atoms are neighbors if they are within 4 Å of each other. We then extract ligand-only features and follow with two more ligand-only layers. This ligand-only layer is pooled using a sum-pooling layer. The pooled features are then used as an embedding for the multi-task multilayer perceptrons (first model 72, second model 74, ...) at the top of the network. The embedding produced by the graph neural network is used to predict three outputs in this example: the activity, the PoseRanker pose quality score, and the Vina docking score. This is performed in two stages. First, the PoseRanker and Vina score predictions are computed by passing the embedding through two independent multilayer perceptrons. A conditioned embedding is then formed by concatenating the input embedding with the PoseRanker score prediction, and passed to a third multilayer perceptron to compute the activity prediction [15]. Section 4.3 provided details of model training parameters.

### 2.2 Data

The training dataset consisted of binding affinity measurements collected from publicly available sources like Chembl or Pubchem and commercial databases like Reaxys or Liceptor. In this example, we considered only quantitative measurements with pKi 2 (0; 11). Compounds were labeled as active if their measured pKi (or IC50) is less than 10µM; otherwise they were labeled as inactive. The number of measured active compounds is larger than inactive ones, so we augmented the training dataset by randomly assigning each of the active compounds as decoys for another, dissimilar protein target. Additionally, for some models we used pose-negatives - active compounds provided with poor poses and labeled as inactive, see Section 4.2 for details. We excluded a set of 12 diverse proteins (D12) from the training, and it set served as the hold-out test set. Additionally from the training set, we excluded all close homologs of the D12 set proteins (>95% sequence similarly). The training set covers more than 3800 diverse proteins, and counts 4.8M (5.8M) datapoints without (with) pose-negatives. The hold out set counts c.a. 33000 compounds, distributed over 12 proteins. Every compound is docked with the disclosure architecture, CUina [16], and the best available pose (as ranked by the PoseRanker model [10]) was used for scoring with the DL models.

### 2.3 Numerical experiments

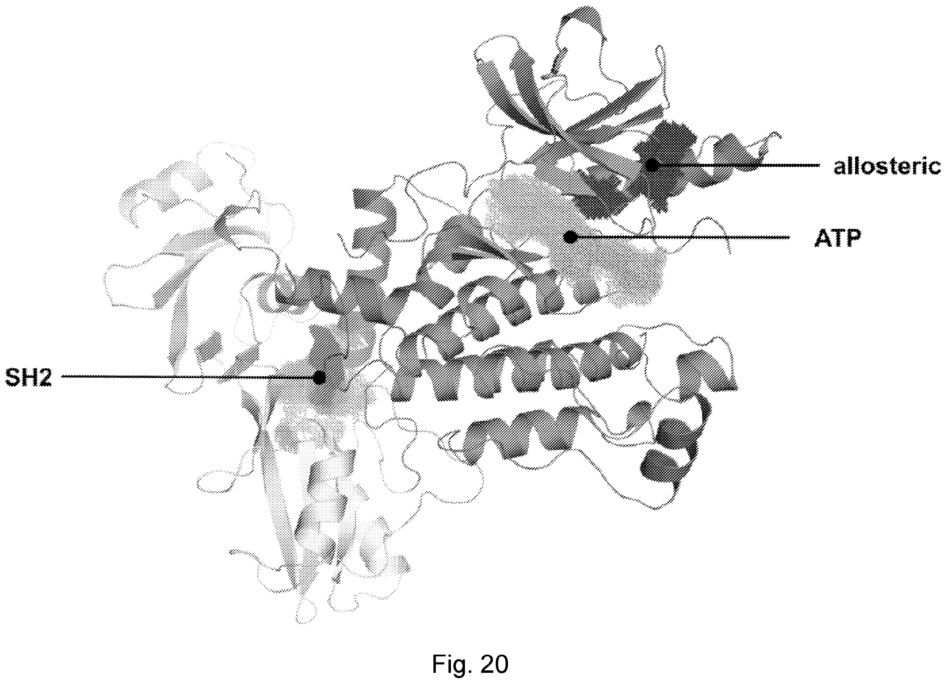

To study pose-sensitivity of our models, we scored each of the active target-compound pairs in D12 three times: i) with the top pose ii) the poor pose iii) a physically implausible pose obtained from the top pose by a random rotation of the ligand around ligand's center of mass (repeated 4 times). A good pose was the highest ranked pose by PoseRanker (poses were generated with CUina). A poor pose was the worst ranked pose by PoseRanker. An implausible pose was obtained from the good pose by a random rotation of a ligand around its center of mass. Every pose was scored and then scores for the poor and implausible poses were subtracted from the score of the good pose. The measure of pose-sensitivity is the median of the drop of the activity score between good and poor/implausible poses. Convolutional neural networks can detect features in the perceptive field of the input data. If that field is large and complex enough, the model can detect constellations of atoms that are characteristic to conserved binding sites, *e.g.* ATP binding sites in protein kinases. However, limiting the scope of the perceptive field, by *e.g.* pooling, drops the spatial information between detected features. As a result, the model can be biased by detecting chemically irrelevant features provided in the input data - the so called Picasso Problem. To monitor how the neighbor binding sites interfere with the model's inference, we selected a diverse set of known kinase inhibitors (about 300 diverse compounds labeled as actives) and mixed them with 10⁵ randomly selected compounds from the available screening library (MCULE, as of 2017/18/10; labeled as inactive). Each compound was docked to the ATP binding site, and an allosteric site that is 6-10 Å away from the ATP site. To monitor the potential bias of the model, we also docked all the compounds to the tentative binding site located on the distant SH2 domain (more than 50 Å away from the ATP binding site), Figure 20. The expectation was that the model with a good performance, can properly distinguish kinase inhibitors from the background random molecules when docked to the ATP binding site (expecting ROC AUC much higher than 0.5). On the other hand, a pose-sensitive model should not be biased by the neighbor ATP-site when compounds are docked to the allosteric site (ROC AUC close to 0.5), Figure 20. To account for any possible biases in the training set, ROC AUC for the spatially distant binding site, located at the SH2 domain was calculated, blue points in Figure 20.

### 3 Results

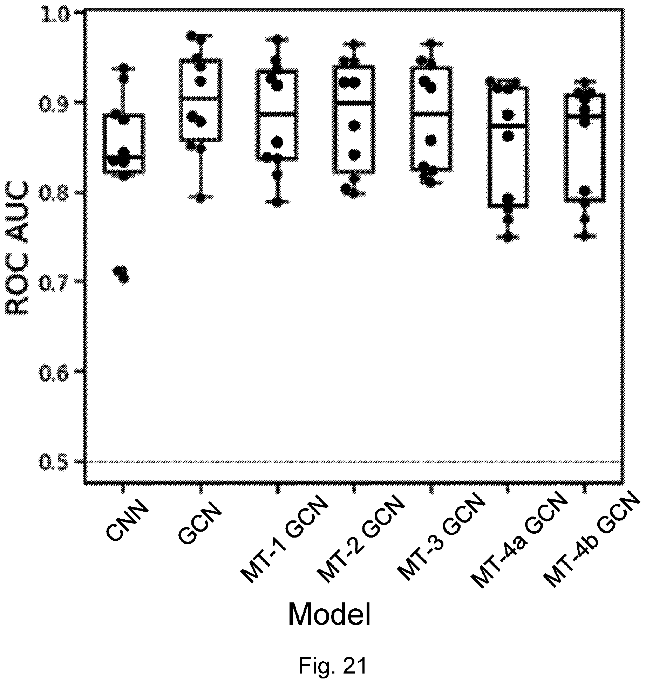

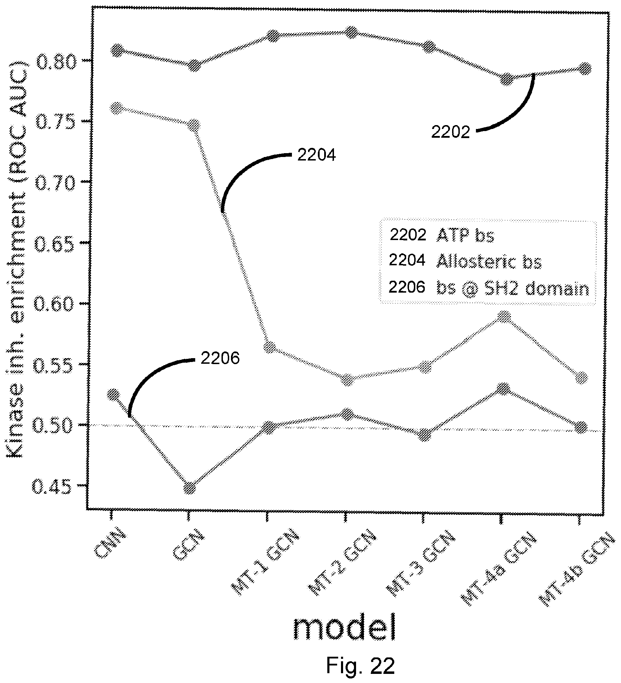

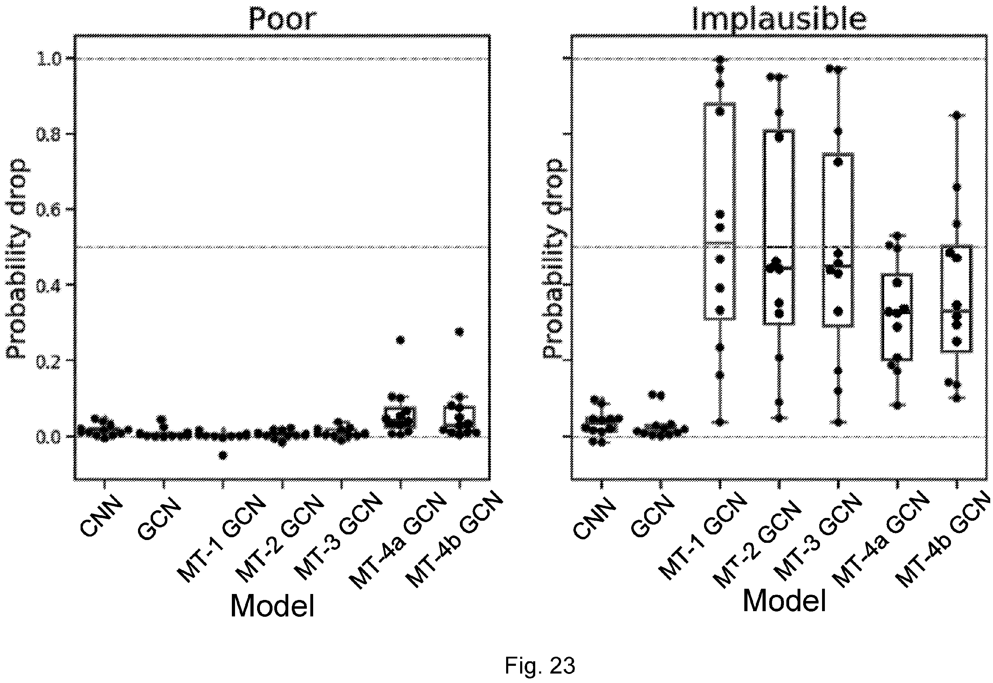

Results in Figure 21 show that the models studied in this example have a good performance on the holdout set, with GCN being slightly better than CNN. However, if both of the single task models were used in the virtual screen of the allosteric site of the human ZAP70 protein, both of them would enrich known ATP-site kinase inhibitors. This is because models do not learn the features of ligand-receptor interactions, and instead learn the independent representations of the ligand and the receptor. These learned representations/embeddings are then used for the models' inference. Because the ATP binding site is in the perceptive field of these two networks, GCN and CNN models can identify the features of the highly conserved ATP binding site (Figure 20, Figure 22), and make the predictions as if the models were asked about the ATP site instead of less common allosteric site, Figure 22. This result cannot be explained by a biased training set, as screening of a binding site that is spatially distant from the primary site (ATP site) gave no enrichment of the kinase inhibitors (SH2 site, Figure 20, Figure 22). This suggests that these two models, CNN and GCN, are of the proteochemometric nature (where ligand and receptor representations are used, but one is independent from the other). This is further corroborated by being insensitive not only to a ligand misplacement at the binding site (poor poses) Figure 23 left panel, but also to breaking the ligand-receptor interface, Figure 23, right panel. This peculiar behavior has been also observed in the previous work on the 3D-grid-based CNN [4, 13], but a generally applicable solution has not been proposed. The major drawback of the PCM model is its innate insensitivity to the pose used for the inference. Therefore, the solution to the Picasso problem is to ensure that the model is pose-sensitive. The minimum requirements for the model to be considered pose sensitive in this example are i) that the physically implausible poses (*e.g.* with multiple atom-atom overlaps) are penalized compared to poses without physically implausible features; ii) poses with ligands outside the binding pocket should be penalized over poses with ligands at the binding site; and iii) binding sites in the vicinity of the targeted site should not interfere with the prediction.

At first, it is non-intuitive that the single task (activity) model, trained on the structural data, does not use that structural information about ligand-receptor interactions. This however may be the case, since during the training, the main objective is to minimize the specified loss function, and it is only an assumption that usage of ligand-receptor interactions can give the model the edge in this task. In reality, *in silico* generated poses are subject to errors and uncertainties and, in turn, overreliance on them can hurt the performance of the model. Because the model has no incentive to learn the structural features of the ligand-receptor interactions, models often neglect them. Therefore training a multi-task model, where the additional tasks require structure sensitive embeddings should, in theory, alleviate the problem. This indeed is the case as can be seen for the MT models in the Figure 20 and Figure 22. It can be seen that adding a docking score regression as another task (model MT-1), already leads to a model that penalizes obviously incorrect poses (implausible poses) and decreases the amount of kinase inhibitors enriched in the top hits of the allosteric site of the hZAP70 protein (Figure 22). Because the poor poses are misplaced but do not have any atomic clashes, the MT-1 model is still not able to distinguish between good and poor poses used in the screen, Figure 23; left panel. Interestingly, this problem cannot be fixed just by adding a pose quality regression as the third task or even by conditioning the activity task on the pose quality, models MT-2 and MT-3 (Figure 23). This is because the models are shown only good poses, and they cannot learn the notion of what the bad poses look like.

To supplement that missing information, a data augmentation technique called pose-negatives is used. Pose-negatives are the examples, which originally were labeled as positive data points, and used with the best available poses. However we can choose the worst available pose (according to an arbitrary metric, which in our case is the PoseRanker score), and present them to the model with changed labels - as negative examples. With this approach, we observed that the models (MT-4a and MT-4b) were capable of penalizing both physically implausible poses as well as poor poses, Figure 23. Moreover, the same models also mitigated the Picasso problem. However, in this case, we observed that lack of the conditioning of the activity on the pose quality leads to the model that is more prone to the Picasso problem, Figure 22.

### 4 Conclusions

Multi-task architectures lead to the models that are both capable of predicting biological activity of the compounds, and also can make the full use of the structural data provided for the inference. Forcing the model to learn orthogonal tasks, regularizes the final model. The proposed solution is generally applicable (data not shown) for both 3D grid-based models and graph-based models. This approach opens up the fields of deep learning and structure-based drug discovery to novel binding sites and previously undruggable proteins.

The present work was developed in the context of our efforts to reduce the costs and development time associated with early-stage drug discovery. Success in this area could, in the long term, improve access to medication and reduce health-care costs. It should be acknowledged that our training dataset described here consists of publicly available data and thus necessarily reflects biases in allocation of research funding to various diseases and health conditions. We anticipate that our efforts to improve the generalizability of our model across protein binding sites help to mitigate this limitation in the training data.

### 4.1 Conditional Multi-task architectures

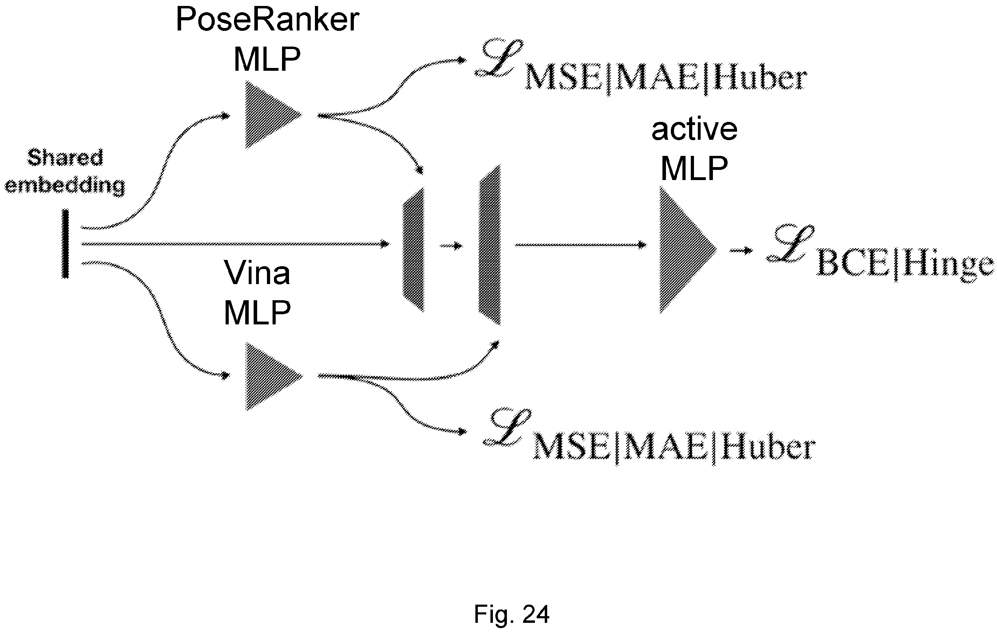

In practice there were no limitations on the loss functions that could be used for regression tasks (MSE, MAE, Huber, Log-Cosh *etc.)* and classification tasks (BCE, Hinge Loss, Squared Hinge, Focal loss, *etc*). Auxiliary tasks can condition the input to the active task i) by transformation of the shared embedding *xₑₘ* with the output si of the task *i:* ${x}_{em}^{′} = f \left({x}_{em}; si\right)$ ; ii) by concatenating the output score sj with the input embedding *xₑₘ:* ${x}_{em}^{′} = \left({x}_{em};sj\right)$ ; and iii) by a combination of i) and ii). Figure 24 shows the architecture, where the input embedding was first conditioned by the PoseRanker score (i), and next the Vina score was concatenated with the embedding (iii).

### 4.2 Data augmentation with pose-negatives

Using CUina docking, 64 poses were generated for every ligand-target pair. Next, PoseRanker was used to sort the poses according their quality[10], and the top 16 poses were selected. The highest ranked pose was used in training and scoring as a good pose, whereas the last (16^{th}) pose was used as a pose-negative and considered inactive (non-binder).

### 4.3 Training

Each model was trained for 10 epochs. For every neural-network architecture, six models were trained, each with 5/6^{th} of the data as the training set, and 1/6^{th} left out for cross-fold validation. Each data cross-fold contains clusters of proteins that share more than 70% of the sequence similarity. Models were trained using the ADAM optimizer with the learning rate lr = 0:001, and targets were sampled with replacement, proportionally to the number of active compounds associated with that target (targets without any measured active compounds were pruned from the training set).

### References

[1] Irwin and Shoichet, 2016, "Docking Screens for Novel Ligands Conferring New Biology: Miniperspective. Journal of Medicinal Chemistry," 59(9):4103-4120, May 2016. ISSN 167 0022-2623, 1520-4804. doi: 0.1021/acs.jmedchem.5b02008. URL https://pubs.acs.org/doi/10.1021/acs.jmedchem.5b02008.
[2] Wallach, Dzamba, and Heifets, 2015, "AtomNet: A Deep Convolutional Neural Network for Bioactivity Prediction in Structure-based Drug Discovery," arXiv:1510.02855 [cs,q-bio, stat].
[3] Ragoza et al, 2017, "Protein-Ligand Scoring with Convolutional Neural Networks, Journal of Chemical Information and Modeling 57(4), pp. 942-957.
[4] Stepniewska-Dziubinska et al., 2018, "Development and evaluation of a deep learning model for protein-ligand binding affinity prediction," Bioinformatics, 34(21), pp. 3666-3674.
[5] Boyles et al., 2019, "Learning from the ligand: using ligand-based features to improve binding affinity prediction," Bioinformatics, page btz665.
[6] Hsieh et al., 2019, "Miro1 Marks Parkinson's Disease Subset and Miro1 Reducer Rescues Neuron Loss in Parkinson's Models. Cell Metabolism," 30(6), pp. 1131-1140.
[7] Kipf and Welling, 2017, "Semi-Supervised Classification with Graph Convolutional Networks," arXiv:1609.02907 [cs, stat], February 2017. URL http://arxiv.org/abs/1609.02907. arXiv: 1609.02907.
[8] Feinberg et al., 2018, "PotentialNet for Molecular Property Prediction," ACS Central Science 4(11), pp. 1520-1530.
[9] Lim et al., 2019, "Predicting Drug-Target Interaction Using a Novel Graph Neural Network with 3D Structure-Embedded Graph Representation," Journal of Chemical Information and Modeling, 59(9), pp. 3981-3988.
[10] Stafford et al., 2021, "Enriching Ligand Pose Quality for Dynamic Proteins in Virtual High Throughput Screens," doi: 10.33774/chemrxiv-2021-t6xkj. URL https://chemrxiv.org/engage/chemrxiv/article-details/614b905e39ef6a1c36268003.
[11] Siege et al., 2019, "In Need of Bias Control: Evaluating Chemical Data for Machine Learning in Structure-Based Virtual Screening," Journal of Chemical Information and Modeling 59(3), pp. 947-961.
[12] Chen et al., 2019, "Hidden bias in the DUD-E dataset leads to misleading performance of deep learning in structure-based virtual screening," PLOS ONE 14(8):e0220113.
[13] Francoeur et al., 2020, "Three-Dimensional Convolutional Neural Networks and a Cross-Docked Data Set for Structure-Based Drug Design," Journal of Chemical Information and Modeling 60(9), pp. 4200-4215.
[14] Trott and Olson, 2010, "AutoDock Vina: Improving the speed and accuracy of docking with a new scoring function, efficient optimization, and multithreading," Journal of Computational Chemistry 31(2) pp. 455-461.
[15] Long et al., 2018, "Conditional Adversarial Domain Adaptation," arXiv:1705.10667 [cs], December 2018.
[16] Morrison et al., 2020, "CUina: An Efficient GPU Implementation of AutoDock Vina," August 2020. URL https://blog.atomwise.com/efficient-gpuimplementation-of-autodock-vina.

## Claims

1. A computer system (100) for characterizing an interaction between a test compound and a target polymer (38), the computer system comprising:
one or more processors (49); and
memory (92/90) addressable by the one or more processors, the memory storing at least one program (36) for execution by the one or more processors, the at least one program comprising instructions for:
(A) obtaining a plurality of atomic coordinates (40) for the target polymer, wherein the plurality of atomic coordinates comprises atomic coordinates for at least 400 atoms;
(B) obtaining a training dataset (44) comprising a respective electronic description of each training compound (46) in a plurality of training compounds, wherein the plurality of training compounds comprises at least 100 compounds, each respective electronic description comprising:
(i) a corresponding positive pose (48) of the corresponding training compound with respect to the plurality of atomic spatial coordinates coupled with a corresponding first positive interaction score (50) that represents a first binding coefficient or an *in silico* pose quality score of the corresponding training compound to the target polymer, and
(ii) a corresponding negative pose (60) of the corresponding training compound with respect to the plurality of atomic spatial coordinates coupled with a corresponding first negative interaction score (62) that represents a second binding coefficient or *in silico* pose quality score of the corresponding training compound to the target polymer; and
(C) training at least a first model (72), wherein the first model has a first plurality of parameters (73), and wherein the first plurality of parameters comprises more than 400 parameters, the training comprising, for each corresponding training compound in the plurality of training compounds:
(i) comparing an output from the first model, upon inputting a corresponding positive score for the corresponding positive pose of the corresponding training compound with respect to the target polymer into the first model, with the corresponding first positive interaction score of the corresponding training compound with respect to the target polymer, wherein the corresponding positive score is obtained by inputting the corresponding positive pose into a trained neural network (24), and
(ii) comparing an output from the first model, upon inputting a corresponding negative score for the corresponding negative pose of the corresponding training compound with respect to the target polymer into the first model, with the corresponding first negative interaction score of the corresponding training compound with respect to the target polymer, wherein the corresponding negative score is obtained by inputting the corresponding negative pose into the trained neural network,
thereby adjusting the first plurality of parameters, wherein at least an output of the first model provides a binding coefficient or an *in silico* pose quality score for the interaction between the test compound and the target polymer.

2. The computer system of claim 1, wherein the first model is a first fully connected neural network.

3. The computer system of claim 1, wherein
the training is a regression task in which the first plurality of parameters is adjusted by back-propagation through an associated loss function,
the corresponding first positive interaction score is related to the corresponding first negative interaction score by the expression: $B = N \times A ,$
wherein,
*A* is the corresponding positive interaction score,
*B* is the corresponding negative interaction score, and
*N* is a real number that is greater than zero and less than 1.

4. The computer system of claim 3, wherein the associated loss function is a mean squared error loss function, a mean absolute error loss function, a Huber loss function, a Log-Cosh loss function, or a quantile loss function.

5. The computer system of claim 3, wherein
the corresponding first positive interaction score and the corresponding first negative interaction score each represent a binding coefficient, and
the corresponding first positive interaction score is an *in vitro* measurement of the binding coefficient of the corresponding training compound to the target polymer.

6. The computer system of claim 5, wherein the first positive interaction score is an IC₅₀, EC₅₀, Kd, KI, or pKI for the respective training compound with respect to the target polymer.

7. The computer system of any one of claims 1-6, wherein
each respective electronic description in the training dataset further comprises a corresponding positive activity score (56) for the corresponding positive pose of the corresponding training compound and a corresponding negative activity score (68) for the corresponding negative pose of the corresponding training compound,
the training at least the first model (C) further comprises jointly training a second model (74) with the first model, wherein the second model has a second plurality of parameters (75), and the training further comprises, for each corresponding training compound in the plurality of training compounds:
(iii) comparing an output of the second model, upon inputting the corresponding positive score for the corresponding positive pose of the corresponding training compound with respect to the target polymer into the second model, with the corresponding positive activity score of the corresponding training compound, and
(iv) comparing an output of the second model, upon inputting the corresponding negative score for the corresponding negative pose of the corresponding training compound with respect to the target polymer into the second model, with the corresponding negative activity score of the corresponding training compound,
thereby adjusting the second plurality of parameters, wherein the second model provides an activity of the interaction between the test compound and the target polymer.

8. The computer system of any one of claims 1-6, wherein
each respective electronic description in the training dataset further comprises a corresponding positive activity score for the corresponding positive pose of the corresponding training compound and a corresponding negative activity score for the corresponding negative pose of the corresponding training compound,
the training at least the first model (C) further comprises jointly training a second model with the first model, wherein the second model has a second plurality of parameters, the training further comprises, for each corresponding training compound in the plurality of training compounds:
(iii) comparing an output of the second model, upon jointly inputting the corresponding positive score for the corresponding positive pose of the corresponding training compound with respect to the target polymer and the corresponding first positive interaction score into the second model, with the corresponding positive activity score of the corresponding training compound, and
(iv) comparing an output of the second model, upon jointly inputting the corresponding negative score for the corresponding negative pose of the corresponding training compound with respect to the target polymer and the corresponding first negative interaction score into the second model, with the corresponding negative activity score of the corresponding training compound,
thereby adjusting the second plurality of parameters, wherein the second model provides an activity score for the test compound with respect to the target polymer.

9. The computer system of claim 8, wherein
the training of the first model is a regression task in which the first plurality of parameters is adjusted by back-propagation through a first associated loss function, and
the training of the second model is a classification task in which the second plurality of parameters is adjusted by back-propagation through a second associated loss function.

10. The computer system of claim 1, wherein
each respective electronic description in the training dataset further comprises a corresponding second positive interaction score (58) for the corresponding positive pose of the corresponding training compound and a corresponding second negative interaction score (70) for the corresponding negative pose of the corresponding training compound,
each respective electronic description in the training dataset further comprises a corresponding positive activity score for the corresponding positive pose of the corresponding training compound and a corresponding negative activity score for the corresponding negative pose of the corresponding training compound,
the training at least the first model (C) further comprises jointly training a second model (74) and a third model (76) with the first model, wherein the second model has a second plurality of parameters (75) and the third model has a third plurality of parameters (77), the training further comprising, for each corresponding training compound in the plurality of training compounds:
(iii) comparing an output of the second model, upon inputting the corresponding positive score for the corresponding positive pose of the corresponding training compound with respect to the target polymer into the second model, with the corresponding second positive interaction score of the corresponding training compound with respect to the target polymer,
(iv) comparing an output of the second model, upon inputting the corresponding negative score for the corresponding negative pose of the corresponding training compound with respect to the target polymer as input to the second model, with the corresponding second negative interaction score of the corresponding training compound with respect to the target polymer,
thereby adjusting the second plurality of parameters,
(v) comparing an output of the third model, upon jointly inputting (a) the corresponding positive score for the corresponding positive pose of the corresponding training compound with respect to the target polymer, (b) the output of the first model upon input of the corresponding positive score for the corresponding positive pose of the corresponding training compound and (c) the output of the second model upon input of the corresponding positive score for the corresponding positive pose of the corresponding training compound into the third model, with the corresponding positive activity score of the corresponding training compound, and
(vi) comparing an output of the first model, upon jointly inputting (a) the corresponding negative score for the corresponding negative pose of the corresponding training compound with respect to the target polymer, (b) the output of the first model upon input of the corresponding negative score for the corresponding negative pose of the corresponding training compound, and (c) the output of the second model upon input of the corresponding negative score for the corresponding negative pose of the corresponding training compound into the third model, with the corresponding negative activity score of the corresponding training compound, thereby adjusting the third plurality of parameters of the third model, wherein an output of the third model provides an activity of the test compound with respective to the target polymer.

11. The computer system of claim 10, wherein
the training of the first model is a first regression task in which the first plurality of parameters is adjusted by back-propagation through a first associated loss function,
the training of the second model is a second regression task in which the second plurality of parameters is adjusted by back-propagation through a second associated loss function, and
the training of the third model is a classification task in which the third plurality of parameters is adjusted by back-propagation through a third associated loss function.

12. The computer system of claim 11, wherein
the corresponding first positive interaction score and the corresponding first negative interaction score each represent an *in silico* pose quality score of the corresponding training compound to the target polymer,
the corresponding second positive interaction score and the corresponding second negative interaction score each represent a binding coefficient of the corresponding training compound to the target polymer, and
the corresponding positive activity score is a first binary activity score and the corresponding negative activity score is a second binary activity score.

13. The computer system of claim 12, wherein
the first associated loss function is a mean squared error loss function, a mean absolute error loss function, a Huber loss function, a Log-Cosh loss function, or a quantile loss function,
the second associated loss function is a mean squared error loss function, a mean absolute error loss function, a Huber loss function, a Log-Cosh loss function, or a quantile loss function, and
the third associated loss function is a binary cross entropy loss function, a hinge loss function, or a squared hinged loss function.

14. A method for characterizing an interaction between a test compound and a target polymer (38), the method comprising:
at a computer system comprising a memory:
(A) obtaining a plurality of atomic coordinates (40) for the target polymer, wherein the plurality of atomic coordinates comprises atomic coordinates for at least 400 atoms;
(B) obtaining a training dataset (44) comprising a respective electronic description of each training compound (46) in a plurality of training compounds, wherein the plurality of training compounds comprises at least 100 compounds, each respective electronic description comprising:
(i) a corresponding positive pose (48) of the corresponding training compound with respect to the plurality of atomic spatial coordinates coupled with a corresponding first positive interaction score (50) that represents a first binding coefficient or an *in silico* pose quality score of the corresponding training compound to the target polymer, and
(ii) a corresponding negative pose (60) of the corresponding training compound with respect to the plurality of atomic spatial coordinates coupled with a corresponding first negative interaction score (62) that represents a second binding coefficient or an *in silico* pose quality score of the corresponding training compound to the target polymer; and
(C) training at least a first model (72), wherein the first model has a first plurality of parameters (73), and wherein the first plurality of parameters comprises more than 400 parameters, the training comprising, for each corresponding training compound in the plurality of training compounds:
(i) comparing an output from the first model, upon inputting a corresponding positive score for the corresponding positive pose of the corresponding training compound with respect to the target polymer into the first model, with the corresponding first positive interaction score of the corresponding training compound with respect to the target polymer, wherein the corresponding positive score is obtained by inputting the corresponding positive pose into a trained neural network (24), and
(ii) comparing an output from the second model, upon inputting a corresponding negative score for the corresponding negative pose of the corresponding training compound with respect to the target polymer into the first model, with the corresponding first negative interaction score of the corresponding training compound with respect to the target polymer, wherein the corresponding negative score is obtained by inputting the corresponding negative pose into the trained neural network,
thereby adjusting the first plurality of parameters, wherein at least an output of the first model provides a binding coefficient or an *in silico* pose quality score for the interaction between the test compound and the target polymer.

15. A non-transitory computer readable storage medium, wherein the non-transitory computer readable storage medium stores instructions, which when executed by a computer system, cause the computer system to perform a method for characterizing an interaction between a test compound and a target polymer, the method comprising:
(A) obtaining a plurality of atomic coordinates (40) for the target polymer, wherein the plurality of atomic coordinates comprises atomic coordinates for at least 400 atoms;
(B) obtaining a training dataset (44) comprising a respective electronic description of each training compound (46) in a plurality of training compounds, wherein the plurality of training compounds comprises at least 100 compounds, each respective electronic description comprising:
(i) a corresponding positive pose (48) of the corresponding training compound with respect to the plurality of atomic spatial coordinates coupled with a corresponding first positive interaction score (50) that represents a first binding coefficient or an *in silico* pose quality score of the corresponding training compound to the target polymer, and
(ii) a corresponding negative pose (60) of the corresponding training compound with respect to the plurality of atomic spatial coordinates coupled with a corresponding first negative interaction score (62) that represents a second binding coefficient or an *in silico* pose quality score of the corresponding training compound to the target polymer; and
(C) training at least a first model (72), wherein the first model has a first plurality of parameters (73), and wherein the first plurality of parameters comprises more than 400 parameters, the training comprising, for each corresponding training compound in the plurality of training compounds:
(i) comparing an output from the first model, upon inputting a corresponding positive score for the corresponding positive pose of the corresponding training compound with respect to the target polymer into the first model, with the corresponding first positive interaction score of the corresponding training compound with respect to the target polymer, wherein the first corresponding positive score is obtained by inputting the corresponding positive pose into a trained neural network (24), and
(ii) comparing an output form the first model, upon inputting a corresponding negative score for the corresponding negative pose of the corresponding training compound with respect to the target polymer into the first model, with the corresponding first negative interaction score of the corresponding training compound with respect to the target polymer, wherein the corresponding negative score is obtained by inputting the corresponding negative pose into the trained neural network,
thereby adjusting the first plurality of parameters, wherein at least an output of the first model provides a binding coefficient or an *in silico* pose quality score for the interaction between the test compound and the target polymer.

## Patentansprüche

1. Computersystem (100) zur Charakterisierung einer Interaktion zwischen einer Testverbindung und einem Zielpolymer (38), das Computersystem umfassend:
einen oder mehrere Prozessoren (49); und
einen Speicher (92/90), der von dem einen oder den mehreren Prozessoren adressierbar ist, wobei der Speicher mindestens ein Programm (36) zur Ausführung durch den einen oder die mehreren Prozessoren speichert, wobei das mindestens eine Programm Befehle umfasst zum:
(A) Ermitteln einer Vielzahl von Atomkoordinaten (40) für das Zielpolymer, wobei die Vielzahl von Atomkoordinaten mindestens 400 Atome umfasst;
(B) Ermitteln eines Trainingsdatensatzes (44), der eine jeweilige elektronische Beschreibung jeder Trainingsverbindung (46) aus einer Vielzahl von Trainingsverbindungen umfasst, wobei die Vielzahl von Trainingsverbindungen mindestens 100 Verbindungen umfasst und jede jeweilige elektronische Beschreibung Folgendes umfasst:
(i) eine entsprechende positive Position (48) der entsprechenden Trainingsverbindung in Bezug auf die Vielzahl von atomaren Raumkoordinaten, gekoppelt mit einem entsprechenden ersten positiven Interaktionswert (50), der einen ersten Bindungskoeffizienten oder einen *in silico* Positionsqualitätswert der entsprechenden Trainingsverbindung zum Zielpolymer darstellt, und
(ii) eine entsprechende negative Position (60) der jeweiligen Trainingsverbindung in Bezug auf die Vielzahl von atomaren Raumkoordinaten, gekoppelt mit einem entsprechenden ersten negativen Interaktionswert (62), der einen zweiten Bindungskoeffizienten oder einen *in silico* Positionsqualitätswert der jeweiligen Trainingsverbindung zum Zielpolymer darstellt; und
(C) Trainieren mindestens eines ersten Modells (72), wobei das erste Modell eine erste Vielzahl von Parametern (73) aufweist und wobei die erste Vielzahl von Parametern mehr als 400 Parameter umfasst, das Training umfassend für jede entsprechende Trainingsverbindung aus der Vielzahl von Trainingsverbindungen:
(i) Vergleichen einer Ausgabe des ersten Modells, nach Eingabe eines entsprechenden positiven Werts für die entsprechende positive Position der entsprechenden Trainingsverbindung in Bezug auf das Zielpolymer in das erste Modell, mit dem entsprechenden ersten positiven Interaktionswert der entsprechenden Trainingsverbindung in Bezug auf das Zielpolymer, wobei der entsprechende positive Wert durch Eingabe der entsprechenden positiven Position in ein trainiertes neuronales Netzwerk (24) erhalten wird, und
(ii) Vergleichen einer Ausgabe des ersten Modells, nach Eingabe eines entsprechenden negativen Werts für die entsprechende negative Position der entsprechenden Trainingsverbindung in Bezug auf das Zielpolymer in das erste Modell, mit dem entsprechenden ersten negativen Interaktionswert der entsprechenden Trainingsverbindung in Bezug auf das Zielpolymer, wobei der entsprechende negative Wert durch Eingabe der entsprechenden negativen Position in das trainierte neuronale Netzwerk erhalten wird,
wodurch die erste Vielzahl von Parametern angepasst wird, wobei mindestens eine Ausgabe des ersten Modells einen Bindungskoeffizienten oder einen *in silico* Positionsqualitätswert für die Interaktion zwischen der Testverbindung und dem Zielpolymer liefert.

2. Computersystem nach Anspruch 1, wobei das erste Modell ein erstes vollverbundenes neuronales Netzwerk ist.

3. Computersystem nach Anspruch 1, wobei
das Training eine Regressionsaufgabe ist, bei der die erste Vielzahl von Parametern durch Rückpropagation über eine zugehörige Verlustfunktion angepasst wird,
der entsprechende erste positive Interaktionswert mit dem entsprechenden ersten negativen Interaktionswert durch den Ausdruck: $B = N \times A ,$
in Beziehung steht,
wobei,
*A* der er entsprechende positive Interaktionswert ist,
*B* der er entsprechende positive Interaktionswert ist,
*N* eine reelle Zahl ist, die größer als Null und kleiner als 1 ist.

4. Computersystem nach Anspruch 3, wobei die zugehörige Verlustfunktion eine mittlere quadratische Fehlerverlustfunktion, eine mittlere absolute Fehlerverlustfunktion, eine Huber-Verlustfunktion, eine Log-Cosh-Verlustfunktion oder eine Quantil-Verlustfunktion ist.

5. Computersystem nach Anspruch 3, wobei
der entsprechende erste positive Interaktionswert und der entsprechende erste negative Interaktionswert jeweils einen Bindungskoeffizienten darstellen und
der entsprechende erste positive Interaktionswert eine *in vitro* Messung des Bindungskoeffizienten der entsprechenden Trainingsverbindung an das Zielpolymer ist.

6. Computersystem nach Anspruch 5, wobei der erste positive Interaktionswert ein IC₅₀, EC₅₀, Kd, KI oder pKI für die jeweilige Trainingsverbindung in Bezug auf das Zielpolymer ist.

7. Computersystem nach einem der Ansprüche 1-6, wobei
jede jeweilige elektronische Beschreibung im Trainingsdatensatz ferner einen entsprechenden positiven Aktivitätswert (56) für die entsprechende positive Position der entsprechenden Trainingsverbindung und einen entsprechenden negativen Aktivitätswert (68) für die entsprechende negative Pose der entsprechenden Trainingsverbindung umfasst,
das Training zumindest des ersten Modells (C) umfasst ferner das gemeinsame Training eines zweiten Modells (74) mit dem ersten Modell, wobei das zweite Modell eine zweite Vielzahl von Parametern (75) aufweist, und das Training für jede entsprechende Trainingsverbindung aus der Vielzahl von Trainingsverbindungen ferner umfasst:
(iii) Vergleichen einer Ausgabe des zweiten Modells, nach Eingabe des entsprechenden positiven Werts für die entsprechende positive Position der entsprechenden Trainingsverbindung in Bezug auf das Zielpolymer in das zweite Modell, mit dem entsprechenden positiven Aktivitätswert der entsprechenden Trainingsverbindung, und
(iv) Vergleichen einer Ausgabe des zweiten Modells, nach Eingabe des entsprechenden negativen Werts für die entsprechende negative Position der entsprechenden Trainingsverbindung in Bezug auf das Zielpolymer in das zweite Modell, mit dem entsprechenden negativen Aktivitätswert der entsprechenden Trainingsverbindung,
wodurch die zweite Vielzahl von Parametern angepasst wird, wobei das zweite Modell eine Aktivität der Interaktion zwischen der Testverbindung und dem Zielpolymer liefert.

8. Computersystem nach einem der Ansprüche 1 bis 6, wobei
jede jeweilige elektronische Beschreibung im Trainingsdatensatz ferner einen entsprechenden positiven Aktivitätswert für die entsprechende positive Position der entsprechenden Trainingsverbindung und einen entsprechenden negativen Aktivitätswert für die entsprechende negative Position der entsprechenden Trainingsverbindung umfasst,
das Trainieren zumindest des ersten Modells (C) ferner das gemeinsame Trainieren eines zweiten Modells mit dem ersten Modell umfasst, wobei das zweite Modell eine zweite Vielzahl von Parametern aufweist, und das Trainieren ferner für jede entsprechende Trainingsverbindung in der Vielzahl von Trainingsverbindungen Folgendes umfasst:
(iii) Vergleichen einer Ausgabe des zweiten Modells, nach gemeinsamer Eingabe des entsprechenden positiven Werts für die entsprechende positive Position der entsprechenden Trainingsverbindung in Bezug auf das Zielpolymer und des entsprechenden ersten positiven Interaktionswerts in das zweite Modell, mit dem entsprechenden positiven Aktivitätswert der entsprechenden Trainingsverbindung, und
(iv) Vergleichen einer Ausgabe des zweiten Modells, nach gemeinsamer Eingabe des entsprechenden negativen Werts für die entsprechende negative Position der entsprechenden Trainingsverbindung in Bezug auf das Zielpolymer und des entsprechenden ersten negativen Interaktionswerts in das zweite Modell, mit dem entsprechenden negativen Aktivitätswert der entsprechenden Trainingsverbindung,
wodurch die zweite Vielzahl von Parametern angepasst wird, wobei das zweite Modell einen Aktivitätswert für die Testverbindung in Bezug auf das Zielpolymer liefert.

9. Computersystem nach Anspruch 8, wobei
das Training des ersten Modells eine Regressionsaufgabe ist, bei der die erste Vielzahl von Parametern durch Rückpropagation über eine erste zugehörige Verlustfunktion angepasst wird, und
das Training des zweiten Modells eine Klassifikationsaufgabe ist, bei der die zweite Vielzahl von Parametern durch Rückpropagation über eine zweite zugehörige Verlustfunktion angepasst wird.

10. Computersystem nach Anspruch 1, wobei
jede jeweilige elektronische Beschreibung im Trainingsdatensatz ferner einen entsprechenden zweiten positiven Interaktionswert (58) für die entsprechende positive Position der entsprechenden Trainingsverbindung und einen entsprechenden zweiten negativen Interaktionswert (70) für die entsprechende negative Position der entsprechenden Trainingsverbindung umfasst,
jede jeweilige elektronische Beschreibung im Trainingsdatensatz ferner einen entsprechenden positiven Aktivitätswert für die entsprechende positive Position der entsprechenden Trainingsverbindung und einen entsprechenden negativen Aktivitätswert für die entsprechende negative Position der entsprechenden Trainingsverbindung umfasst,
das Trainieren zumindest des ersten Modells (C) umfasst ferner das gemeinsame Trainieren eines zweiten Modells (74) und eines dritten Modells (76) mit dem ersten Modell, wobei das zweite Modell eine zweite Vielzahl von Parametern (75) und das dritte Modell eine dritte Vielzahl von Parametern (77) aufweist, das Trainieren ferner umfassend für jede entsprechende Trainingsverbindung in der Vielzahl von Trainingsverbindungen:
(iii) Vergleichen einer Ausgabe des zweiten Modells, nach Eingabe des entsprechenden positiven Werts für die entsprechende positive Pose der entsprechenden Trainingsverbindung in Bezug auf das Zielpolymer in das zweite Modell, mit dem entsprechenden zweiten positiven Interaktionswert der entsprechenden Trainingsverbindung in Bezug auf das Zielpolymer,
(iv) Vergleichen einer Ausgabe des zweiten Modells, nach Eingabe des entsprechenden negativen Werts für die entsprechende negative Position der entsprechenden Trainingsverbindung in Bezug auf das Zielpolymer in das zweite Modell, mit dem entsprechenden zweiten negativen Interaktionswert der entsprechenden Trainingsverbindung in Bezug auf das Zielpolymer,
wodurch die zweite Vielzahl von Parametern angepasst wird,
(v) Vergleichen einer Ausgabe des dritten Modells, nach gemeinsamer Eingabe von (a) der entsprechende positive Wert für die entsprechende positive Position der entsprechenden Trainingsverbindung in Bezug auf das Zielpolymer, (b) die Ausgabe des ersten Modells nach Eingabe des entsprechenden positiven Werts für die entsprechende positive Position der entsprechenden Trainingsverbindung und (c) die Ausgabe des zweiten Modells nach Eingabe des entsprechenden positiven Werts für die entsprechende positive Position der entsprechenden Trainingsverbindung in das dritte Modell, mit dem entsprechenden positiven Aktivitätswert der entsprechenden Trainingsverbindung, und
(vi) Vergleichen einer Ausgabe des ersten Modells nach gemeinsamer Eingabe von (a) der entsprechenden negativen Bewertung für die entsprechende negative Position der entsprechenden Trainingsverbindung in Bezug auf das Zielpolymer, (b) der Ausgabe des ersten Modells nach Eingabe der entsprechenden negativen Bewertung für die entsprechende negative Position der entsprechenden Trainingsverbindung, sowie (c) der Ausgabe des zweiten Modells nach Eingabe der entsprechenden negativen Bewertung für die entsprechende negative Position der entsprechenden Trainingsverbindung in das dritte Modell, mit der entsprechenden negativen Aktivitätsbewertung der entsprechenden Trainingsverbindung, wodurch die dritte Vielzahl von Parametern des dritten Modells angepasst wird, wobei eine Ausgabe des dritten Modells eine Aktivität der Testverbindung in Bezug auf das Zielpolymer liefert.

11. Computersystem nach Anspruch 10, wobei
das Training des ersten Modells eine erste Regressionsaufgabe ist, bei der die erste Vielzahl von Parametern durch Rückpropagation über eine erste zugehörige Verlustfunktion angepasst wird,
das Training des zweiten Modells eine zweite Regressionsaufgabe ist, bei der die zweite Vielzahl von Parametern durch Rückpropagation über eine zweite zugehörige Verlustfunktion angepasst wird, und
das Training des dritten Modells eine Klassifizierungsaufgabe ist, bei der die dritte Vielzahl von Parametern durch Rückpropagation über eine dritte zugehörige Verlustfunktion angepasst wird.

12. Computersystem nach Anspruch 11, wobei
der entsprechende erste positive Interaktionswert und der entsprechende erste negative Interaktionswert jeweils einen *in silico* Positionsqualitätswert der entsprechenden Trainingsverbindung gegenüber dem Zielpolymer darstellen,
der entsprechende zweite positive Interaktionswert und der entsprechende zweite negative Interaktionswert jeweils einen Bindungskoeffizienten der entsprechenden Trainingsverbindung gegenüber dem Zielpolymer darstellen und
der entsprechende positive Aktivitätswert ein erster binärer Aktivitätswert ist und der entsprechende negative Aktivitätswert ein zweiter binärer Aktivitätswert ist.

13. Computersystem nach Anspruch 12, wobei
die erste zugehörige Verlustfunktion eine mittlere quadratische Fehlerverlustfunktion, eine mittlere absolute Fehlerverlustfunktion, eine Huber-Verlustfunktion, eine Log-Cosh-Verlustfunktion oder eine Quantil-Verlustfunktion ist,
die zweite zugehörige Verlustfunktion eine mittlere quadratische Fehlerverlustfunktion, eine mittlere absolute Fehlerverlustfunktion, eine Huber-Verlustfunktion, eine Log-Cosh-Verlustfunktion oder eine Quantil-Verlustfunktion ist, und
die dritte zugehörige Verlustfunktion eine binäre Kreuzentropie-Verlustfunktion, eine Hinge-Verlustfunktion oder eine quadrierte Hinge-Verlustfunktion ist.

14. Verfahren zur Charakterisierung einer Interaktion zwischen einer Testverbindung und einem Zielpolymer (38), das Verfahren umfassend:
auf einem Computersystem, das einen Speicher umfasst:
(A) Ermitteln einer Vielzahl von Atomkoordinaten (40) für das Zielpolymer, wobei die Vielzahl von Atomkoordinaten mindestens 400 Atome umfasst;
(B) das Ermitteln eines Trainingsdatensatzes (44), der eine jeweilige elektronische Beschreibung jeder Trainingsverbindung (46) aus einer Vielzahl von Trainingsverbindungen umfasst, wobei die Vielzahl von Trainingsverbindungen mindestens 100 Verbindungen umfasst und jede jeweilige elektronische Beschreibung Folgendes umfasst:
(i) eine entsprechende positive Position (48) der entsprechenden Trainingsverbindung in Bezug auf die Vielzahl von atomaren Raumkoordinaten, gekoppelt mit einem entsprechenden ersten positiven Interaktionswert (50), der einen ersten Bindungskoeffizienten oder einen in silico Positionsqualitätswert der entsprechenden Trainingsverbindung zum Zielpolymer darstellt, und
(ii) eine entsprechende negative Position (60) der jeweiligen Trainingsverbindung in Bezug auf die Vielzahl von atomaren Raumkoordinaten, gekoppelt mit einem entsprechenden ersten negativen Interaktionswert (62), der einen zweiten Bindungskoeffizienten oder einen in silico Positionsqualitätswert der jeweiligen Trainingsverbindung zum Zielpolymer darstellt; und
(C) Trainieren mindestens eines ersten Modells (72), wobei das erste Modell eine erste Vielzahl von Parametern (73) aufweist und wobei die erste Vielzahl von Parametern mehr als 400 Parameter umfasst, das Training umfassend für jede entsprechende Trainingsverbindung aus der Vielzahl von Trainingsverbindungen:
(i) Vergleichen einer Ausgabe des ersten Modells, nach Eingabe eines entsprechenden positiven Werts für die entsprechende positive Position der entsprechenden Trainingsverbindung in Bezug auf das Zielpolymer in das erste Modell, mit dem entsprechenden ersten positiven Interaktionswert der entsprechenden Trainingsverbindung in Bezug auf das Zielpolymer, wobei der entsprechende positive Wert durch Eingabe der entsprechenden positiven Position in ein trainiertes neuronales Netzwerk (24) erhalten wird, und
(ii) Vergleichen einer Ausgabe des ersten Modells, nach Eingabe eines entsprechenden negativen Werts für die entsprechende negative Position der entsprechenden Trainingsverbindung in Bezug auf das Zielpolymer in das erste Modell, mit dem entsprechenden ersten negativen Interaktionswert der entsprechenden Trainingsverbindung in Bezug auf das Zielpolymer, wobei der entsprechende negative Wert durch Eingabe der entsprechenden negativen Position in das trainierte neuronale Netzwerk erhalten wird,
wodurch die erste Vielzahl von Parametern angepasst wird, wobei mindestens eine Ausgabe des ersten Modells einen Bindungskoeffizienten oder einen in silico Positionsqualitätswert für die Interaktion zwischen der Testverbindung und dem Zielpolymer liefert.

15. Ein nichtflüchtiges, computerlesbares Speichermedium, wobei das nichtflüchtige, computerlesbare Speichermedium Anweisungen speichert, die, wenn sie von einem Computersystem ausgeführt werden, das Computersystem veranlassen, ein Verfahren zur Charakterisierung einer Interaktion zwischen einer Testverbindung und einem Zielpolymer durchzuführen, das Verfahren umfassend:
(A) Ermitteln einer Vielzahl von Atomkoordinaten (40) für das Zielpolymer, wobei die Vielzahl von Atomkoordinaten mindestens 400 Atome umfasst;
(B) das Ermitteln eines Trainingsdatensatzes (44), der eine jeweilige elektronische Beschreibung jeder Trainingsverbindung (46) aus einer Vielzahl von Trainingsverbindungen umfasst, wobei die Vielzahl von Trainingsverbindungen mindestens 100 Verbindungen umfasst und jede jeweilige elektronische Beschreibung Folgendes umfasst:
(i) eine entsprechende positive Position (48) der entsprechenden Trainingsverbindung in Bezug auf die Vielzahl von atomaren Raumkoordinaten, gekoppelt mit einem entsprechenden ersten positiven Interaktionswert (50), der einen ersten Bindungskoeffizienten oder einen in silico Positionsqualitätswert der entsprechenden Trainingsverbindung zum Zielpolymer darstellt, und
(ii) eine entsprechende negative Position (60) der jeweiligen Trainingsverbindung in Bezug auf die Vielzahl von atomaren Raumkoordinaten, gekoppelt mit einem entsprechenden ersten negativen Interaktionswert (62), der einen zweiten Bindungskoeffizienten oder einen in silico Positionsqualitätswert der jeweiligen Trainingsverbindung zum Zielpolymer darstellt; und
(C) Trainieren mindestens eines ersten Modells (72), wobei das erste Modell eine erste Vielzahl von Parametern (73) aufweist und wobei die erste Vielzahl von Parametern mehr als 400 Parameter umfasst, das Training umfassend für jede entsprechende Trainingsverbindung aus der Vielzahl von Trainingsverbindungen:
(i) Vergleichen einer Ausgabe des ersten Modells, nach Eingabe eines entsprechenden positiven Werts für die entsprechende positive Position der entsprechenden Trainingsverbindung in Bezug auf das Zielpolymer in das erste Modell, mit dem entsprechenden ersten positiven Interaktionswert der entsprechenden Trainingsverbindung in Bezug auf das Zielpolymer, wobei der entsprechende positive Wert durch Eingabe der entsprechenden positiven Position in ein trainiertes neuronales Netzwerk (24) erhalten wird, und
(ii) Vergleichen einer Ausgabe des ersten Modells, nach Eingabe eines entsprechenden negativen Werts für die entsprechende negative Position der entsprechenden Trainingsverbindung in Bezug auf das Zielpolymer in das erste Modell, mit dem entsprechenden ersten negativen Interaktionswert der entsprechenden Trainingsverbindung in Bezug auf das Zielpolymer, wobei der entsprechende negative Wert durch Eingabe der entsprechenden negativen Position in das trainierte neuronale Netzwerk erhalten wird,
wodurch die erste Vielzahl von Parametern angepasst wird, wobei mindestens eine Ausgabe des ersten Modells einen Bindungskoeffizienten oder einen in silico Positionsqualitätswert für die Interaktion zwischen der Testverbindung und dem Zielpolymer liefert.

## Revendications

1. Système informatique (100) de caractérisation d'une interaction entre un composé d'essai et un polymère cible (38), le système informatique comprenant :
un ou plusieurs processeurs (49) ; et
une mémoire (92/90) adressable par les un ou plusieurs processeurs, la mémoire stockant au moins un programme (36) destiné à être exécuté par les un ou plusieurs processeurs, l'au moins un programme comprenant des instructions pour :
(A) l'obtention d'une pluralité de coordonnées atomiques (40) pour le polymère cible, dans lequel la pluralité de coordonnées atomiques comprend des coordonnées atomiques pour au moins 400 atomes ;
(B) l'obtention d'un ensemble de données d'entraînement (44) comprenant une description électronique respective de chaque composé d'entraînement (46) dans une pluralité de composés d'entraînement, dans lequel la pluralité de composés d'entraînement comprend au moins 100 composés, chaque description électronique respective comprenant :
(i) une pose positive (48) correspondante du composé d'entraînement correspondant par rapport à la pluralité de coordonnées spatiales atomiques couplées à un premier score d'interaction positif (50) correspondant qui représente un premier coefficient de liaison ou un score de qualité de pose *in silico* du composé d'entraînement correspondant au polymère cible, et
(ii) une pose négative (60) correspondante du composé d'entraînement correspondant par rapport à la pluralité de coordonnées spatiales atomiques couplées à un premier score d'interaction négatif (62) correspondant qui représente un deuxième coefficient de liaison ou un score de qualité de pose *in silico* du composé d'entraînement correspondant au polymère cible ; et
(C) l'entraînement d'au moins un premier modèle (72), dans lequel le premier modèle présente une première pluralité de paramètres (73), et dans lequel la première pluralité de paramètres comprend plus de 400 paramètres, l'entraînement comprenant, pour chaque composé d'entraînement correspondant dans la pluralité de composés d'entraînement :
(i) la comparaison d'une sortie du premier modèle, lors de l'entrée d'un score positif correspondant pour la pose positive correspondante du composé d'entraînement correspondant par rapport au polymère cible dans le premier modèle, au premier score d'interaction positif correspondant du composé d'entraînement correspondant par rapport au polymère cible, dans lequel le score positif correspondant est obtenu en entrant la pose positive correspondante dans un réseau neuronal entraîné (24), et
(ii) la comparaison d'une sortie du premier modèle, lors de l'entrée d'un score négatif correspondant pour la pose négative correspondante du composé d'entraînement correspondant par rapport au polymère cible dans le premier modèle, avec le premier score d'interaction négatif correspondant du composé d'entraînement correspondant par rapport au polymère cible, dans lequel le score négatif correspondant est obtenu en entrant la pose négative correspondante dans le réseau neuronal entraîné,
pour ainsi ajuster la première pluralité de paramètres, dans lequel au moins une sortie du premier modèle fournit un coefficient de liaison ou un score de qualité de pose *in silico* pour l'interaction entre le composé d'essai et le polymère cible.

2. Système informatique selon la revendication 1, dans lequel le premier modèle est un premier réseau neuronal entièrement connecté.

3. Système informatique selon la revendication 1, dans lequel
l'entraînement est une tâche de régression dans laquelle la première pluralité de paramètres est ajustée par rétropropagation au moyen d'une fonction de perte associée,
le premier score d'interaction positif correspondant est lié au premier score d'interaction négatif correspondant par l'expression : $B = N \times A ,$
dans laquelle,
A est le score d'interaction positif correspondant,
B est le score d'interaction négatif correspondant, et
N est un nombre réel supérieur à zéro et inférieur à 1.

4. Système informatique selon la revendication 3, dans lequel la fonction de perte associée est une fonction de perte d'erreur quadratique moyenne, une fonction de perte d'erreur absolue moyenne, une fonction de perte de Huber, une fonction de perte Log-Cosh ou une fonction de perte quantile.

5. Système informatique selon la revendication 3, dans lequel
le premier score d'interaction positif correspondant et le premier score d'interaction négatif correspondant représentent chacun un coefficient de liaison, et
le premier score d'interaction positif correspondant est une mesure *in vitro* du coefficient de liaison du composé d'entraînement correspondant au polymère cible.

6. Système informatique selon la revendication 5, dans lequel le premier score d'interaction positif est un IC₅₀, EC₅₀, Kd, KI ou pKI pour le composé d'entraînement respectif par rapport au polymère cible.

7. Système informatique selon l'une quelconque des revendications 1 à 6, dans lequel
chaque description électronique respective dans l'ensemble de données d'entraînement comprend en outre un score d'activité positif (56) correspondant pour la pose positive correspondante du composé d'entraînement correspondant et un score d'activité négatif (68) correspondant pour la pose négative correspondante du composé d'entraînement correspondant,
l'entraînement d'au moins le premier modèle (C) comprend en outre l'entraînement conjoint d'un deuxième modèle (74) avec le premier modèle, dans lequel le deuxième modèle présente une deuxième pluralité de paramètres (75), et l'entraînement comprend en outre, pour chaque composé d'entraînement correspondant dans la pluralité de composés d'entraînement :
(iii) la comparaison d'une sortie du deuxième modèle, lors de l'entrée du score positif correspondant pour la pose positive correspondante du composé d'entraînement correspondant par rapport au polymère cible dans le deuxième modèle, au score d'activité positif correspondant du composé d'entraînement correspondant, et
(iv) la comparaison d'une sortie du deuxième modèle, lors de l'entrée du score négatif correspondant pour la pose négative correspondante du composé d'entraînement correspondant par rapport au polymère cible dans le deuxième modèle, au score d'activité négatif correspondant du composé d'entraînement correspondant,
pour ainsi ajuster la deuxième pluralité de paramètres, dans lequel le deuxième modèle fournit une activité de l'interaction entre le composé d'essai et le polymère cible.

8. Système informatique selon l'une quelconque des revendications 1 à 6, dans lequel
chaque description électronique respective dans l'ensemble de données d'entraînement comprend en outre un score d'activité positif correspondant pour la pose positive correspondante du composé d'entraînement correspondant et un score d'activité négatif correspondant pour la pose négative correspondante du composé d'entraînement correspondant,
l'entraînement d'au moins le premier modèle (C) comprend en outre l'entraînement conjoint d'un deuxième modèle avec le premier modèle, dans lequel le deuxième modèle présente une deuxième pluralité de paramètres, l'entraînement comprend en outre, pour chaque composé d'entraînement correspondant dans la pluralité de composés d'entraînement :
(iii) la comparaison d'une sortie du deuxième modèle, lors de l'entrée conjointe du score positif correspondant pour la pose positive correspondante du composé d'entraînement correspondant par rapport au polymère cible et du premier score d'interaction positif correspondant dans le deuxième modèle, au score d'activité positif correspondant du composé d'entraînement correspondant, et
(iv) la comparaison d'une sortie du deuxième modèle, lors de l'entrée conjointe du score négatif correspondant pour la pose négative correspondante du composé d'entraînement correspondant par rapport au polymère cible et du premier score d'interaction négatif correspondant dans le deuxième modèle, au score d'activité négatif correspondant du composé d'entraînement correspondant,
pour ainsi ajuster la deuxième pluralité de paramètres, dans lequel le deuxième modèle fournit un score d'activité pour le composé d'essai par rapport au polymère cible.

9. Système informatique selon la revendication 8, dans lequel
l'entraînement du premier modèle est une tâche de régression dans laquelle la première pluralité de paramètres est ajustée par rétropropagation au moyen d'une première fonction de perte associée, et
l'entraînement du deuxième modèle est une tâche de classification dans laquelle la deuxième pluralité de paramètres est ajustée par rétropropagation au moyen d'une deuxième fonction de perte associée.

10. Système informatique selon la revendication 1, dans lequel
chaque description électronique respective dans l'ensemble de données d'entraînement comprend en outre un deuxième score d'interaction positif (58) correspondant pour la pose positive correspondante du composé d'entraînement correspondant et un deuxième score d'interaction négatif (70) correspondant pour la pose négative correspondante du composé d'entraînement correspondant,
chaque description électronique respective dans l'ensemble de données d'entraînement comprend en outre un score d'activité positif correspondant pour la pose positive correspondante du composé d'entraînement correspondant et un score d'activité négatif correspondant pour la pose négative correspondante du composé d'entraînement correspondant,
l'entraînement d'au moins le premier modèle (C) comprend en outre l'entraînement conjoint d'un deuxième modèle (74) et d'un troisième modèle (76) avec le premier modèle, dans lequel le deuxième modèle présente une deuxième pluralité de paramètres (75) et le troisième modèle présente une troisième pluralité de paramètres (77), l'entraînement comprenant en outre, pour chaque composé d'entraînement correspondant dans la pluralité de composés d'entraînement :
(iii) la comparaison d'une sortie du deuxième modèle, lors de l'entrée du score positif correspondant pour la pose positive correspondante du composé d'entraînement correspondant par rapport au polymère cible dans le deuxième modèle, au deuxième score d'interaction positif correspondant du composé d'entraînement correspondant par rapport au polymère cible,
(iv) la comparaison d'une sortie du deuxième modèle, lors de l'entrée du score négatif correspondant pour la pose négative correspondante du composé d'entraînement correspondant par rapport au polymère cible en tant qu'entrée dans le deuxième modèle, au deuxième score d'interaction négatif correspondant du composé d'entraînement correspondant par rapport au polymère cible,
pour ainsi ajuster la deuxième pluralité de paramètres,
(v) la comparaison d'une sortie du troisième modèle, lors de l'entrée conjointe (a) du score positif correspondant pour la pose positive correspondante du composé d'entraînement correspondant par rapport au polymère cible, (b) de la sortie du premier modèle lors de l'entrée du score positif correspondant pour la pose positive correspondante du composé d'entraînement correspondantet (c) de la sortie du deuxième modèle lors de l'entrée du score positif correspondant pour la pose positive correspondante du composé d'entraînement correspondant dans le troisième modèle, au score d'activité positif correspondant du composé d'entraînement correspondant, et
(vi) la comparaison d'une sortie du premier modèle, lors de l'entrée conjointe (a) du score négatif correspondant pour la pose négative correspondante du composé d'entraînement correspondant par rapport au polymère cible, (b) de la sortie du premier modèle lors de l'entrée du score négatif correspondant pour la pose négative correspondante du composé d'entraînement correspondant, et (c) de la sortie du deuxième modèle lors de l'entrée du score négatif correspondant pour la pose négative correspondante du composé d'entraînement correspondant dans le troisième modèle, au score d'activité négatif correspondant du composé d'entraînement correspondant, pour ainsi ajuster la troisième pluralité de paramètres du troisième modèle, dans lequel une sortie du troisième modèle fournit une activité du composé d'essai par rapport au polymère cible.

11. Système informatique selon la revendication 10, dans lequel
l'entraînement du premier modèle est une première tâche de régression dans laquelle la première pluralité de paramètres est ajustée par rétropropagation au moyen d'une première fonction de perte associée,
l'entraînement du deuxième modèle est une deuxième tâche de régression dans laquelle la deuxième pluralité de paramètres est ajustée par rétropropagation au moyen d'une deuxième fonction de perte associée, et
l'entraînement du troisième modèle est une tâche de classification dans laquelle la troisième pluralité de paramètres est ajustée par rétropropagation au moyen d'une troisième fonction de perte associée.

12. Système informatique selon la revendication 11, dans lequel
le premier score d'interaction positif correspondant et le premier score d'interaction négatif correspondant représentent chacun un score de qualité de pose *in silico* du composé d'entraînement correspondant au polymère cible,
le deuxième score d'interaction positif correspondant et le deuxième score d'interaction négatif correspondant représentent chacun un coefficient de liaison du composé d'entraînement correspondant au polymère cible, et
le score d'activité positif correspondant est un premier score d'activité binaire et le score d'activité négatif correspondant est un deuxième score d'activité binaire.

13. Système informatique selon la revendication 12, dans lequel
la première fonction de perte associée est une fonction de perte d'erreur quadratique moyenne, une fonction de perte d'erreur absolue moyenne, une fonction de perte de Huber, une fonction de perte Log-Cosh ou une fonction de perte quantile,
la deuxième fonction de perte associée est une fonction de perte d'erreur quadratique moyenne, une fonction de perte d'erreur absolue moyenne, une fonction de perte de Huber, une fonction de perte Log-Cosh ou une fonction de perte quantile, et
la troisième fonction de perte associée est une fonction de perte d'entropie croisée binaire, une fonction de perte charnière ou une fonction de perte charnière carrée.

14. Procédé de caractérisation d'une interaction entre un composé d'essai et un polymère cible (38), le procédé comprenant :
au niveau d'un système informatique comprenant une mémoire :
(A) l'obtention d'une pluralité de coordonnées atomiques (40) pour le polymère cible, dans lequel la pluralité de coordonnées atomiques comprend des coordonnées atomiques pour au moins 400 atomes ;
(B) l'obtention d'un ensemble de données d'entraînement (44) comprenant une description électronique respective de chaque composé d'entraînement (46) dans une pluralité de composés d'entraînement, dans lequel la pluralité de composés d'entraînement comprend au moins 100 composés, chaque description électronique respective comprenant :
(i) une pose positive (48) correspondante du composé d'entraînement correspondant par rapport à la pluralité de coordonnées spatiales atomiques couplées à un premier score d'interaction positif (50) correspondant qui représente un premier coefficient de liaison ou un score de qualité de pose *in silico* du composé d'entraînement correspondant au polymère cible, et
(ii) une pose négative (60) correspondante du composé d'entraînement correspondant par rapport à la pluralité de coordonnées spatiales atomiques couplées à un premier score d'interaction négatif (62) correspondant qui représente un deuxième coefficient de liaison ou un score de qualité de pose *in silico* du composé d'entraînement correspondant au polymère cible ; et
(C) l'entraînement d'au moins un premier modèle (72), dans lequel le premier modèle présente une première pluralité de paramètres (73), et dans lequel la première pluralité de paramètres comprend plus de 400 paramètres, l'entraînement comprenant, pour chaque composé d'entraînement correspondant dans la pluralité de composés d'entraînement :
(i) la comparaison d'une sortie du premier modèle, lors de l'entrée d'un score positif correspondant pour la pose positive correspondante du composé d'entraînement correspondant par rapport au polymère cible dans le premier modèle, au premier score d'interaction positif correspondant du composé d'entraînement correspondant par rapport au polymère cible, dans lequel le score positif correspondant est obtenu en entrant la pose positive correspondante dans un réseau neuronal entraîné (24), et
(ii) la comparaison d'une sortie du deuxième modèle, lors de l'entrée d'un score négatif correspondant pour la pose négative correspondante du composé d'entraînement correspondant par rapport au polymère cible dans le premier modèle, avec le premier score d'interaction négatif correspondant du composé d'entraînement correspondant par rapport au polymère cible, dans lequel le score négatif correspondant est obtenu en entrant la pose négative correspondante dans le réseau neuronal entraîné,
pour ainsi ajuster la première pluralité de paramètres, dans lequel au moins une sortie du premier modèle fournit un coefficient de liaison ou un score de qualité de pose *in silico* pour l'interaction entre le composé d'essai et le polymère cible.

15. Support de stockage non transitoire lisible par ordinateur, dans lequel le support de stockage non transitoire lisible par ordinateur stocke des instructions qui, lorsqu'elles sont exécutées par un système informatique, amènent le système informatique à mettre en œuvre un procédé de caractérisation d'une interaction entre un composé d'essai et un polymère cible, le procédé comprenant :
(A) l'obtention d'une pluralité de coordonnées atomiques (40) pour le polymère cible, dans lequel la pluralité de coordonnées atomiques comprend des coordonnées atomiques pour au moins 400 atomes ;
(B) l'obtention d'un ensemble de données d'entraînement (44) comprenant une description électronique respective de chaque composé d'entraînement (46) dans une pluralité de composés d'entraînement, dans lequel la pluralité de composés d'entraînement comprend au moins 100 composés, chaque description électronique respective comprenant :
(i) une pose positive (48) correspondante du composé d'entraînement correspondant par rapport à la pluralité de coordonnées spatiales atomiques couplées à un premier score d'interaction positif (50) correspondant qui représente un premier coefficient de liaison ou un score de qualité de pose *in silico* du composé d'entraînement correspondant au polymère cible, et
(ii) une pose négative (60) correspondante du composé d'entraînement correspondant par rapport à la pluralité de coordonnées spatiales atomiques couplées à un premier score d'interaction négatif (62) correspondant qui représente un deuxième coefficient de liaison ou un score de qualité de pose *in silico* du composé d'entraînement correspondant au polymère cible ; et
(C) l'entraînement d'au moins un premier modèle (72), dans lequel le premier modèle présente une première pluralité de paramètres (73), et dans lequel la première pluralité de paramètres comprend plus de 400 paramètres, l'entraînement comprenant, pour chaque composé d'entraînement correspondant dans la pluralité de composés d'entraînement :
(i) la comparaison d'une sortie du premier modèle, lors de l'entrée d'un score positif correspondant pour la pose positive correspondante du composé d'entraînement correspondant par rapport au polymère cible dans le premier modèle, au premier score d'interaction positif correspondant du composé d'entraînement correspondant par rapport au polymère cible, dans lequel le premier score positif correspondant est obtenu en entrant la pose positive correspondante dans un réseau neuronal entraîné (24), et
(ii) la comparaison d'une sortie du premier modèle, lors de l'entrée d'un score négatif correspondant pour la pose négative correspondante du composé d'entraînement correspondant par rapport au polymère cible dans le premier modèle, avec le premier score d'interaction négatif correspondant du composé d'entraînement correspondant par rapport au polymère cible, dans lequel le score négatif correspondant est obtenu en entrant la pose négative correspondante dans le réseau neuronal entraîné,
pour ainsi ajuster la première pluralité de paramètres, dans lequel au moins une sortie du premier modèle fournit un coefficient de liaison ou un score de qualité de pose *in silico* pour l'interaction entre le composé d'essai et le polymère cible.
